# EUROPEAN PATENT APPLICATION

(11) **EP 4 060 010 A2**
(43) Date of publication of application: **21.09.2022**
(21) Application number: 22161344.1
(22) Date of filing: 10.03.2022
(51) Int. Cl.: C11D 3/386, C12N 9/22, C12N 15/52, C11D 11/00

(54) **CLEANING COMPOSITIONS CONTAINING POLYPEPTIDE VARIANTS**

(30) Priority: 15.03.2021 EP 21162672
(71) Applicant: The Procter & Gamble Company, Cincinnati, OH 45202 (US)
(72) Inventor: CHRISTENSEN, Lars Lehmann Hylling, 2880 Bagsvaerd (DK); JOHANSEN, Annette Helle, 2880 Bagsvaerd (DK); LANT, Neil Joseph, Newcastle upon Tyne, NE12 9BZ (GB); MALTEN, Marco, 2880 Bagsvaerd (DK); OEHLENSCHLAEGER, Christian Berg, 2880 Bagsvaerd (DK); OESTERGAARD, Lars Henrik, 2880 Bagsvaerd (DK)
(74) Representative: P&G Patent Belgium UK

(57) **Abstract**

Detergent compositions comprising DNase variants and cleaning adjunct. Methods of treating surfaces such as fabrics by contacting the surface with an aqueous wash liquor having the detergent composition therein. The compositions and methods are particularly for improving whiteness of a fabric, improved soil removal from a fabric, for malodour removal from a fabric, for anti-wrinkle benefits, anti-redeposition benefits and/or for improved drying of a fabric.

## Description

### REFERENCE TO A SEQUENCE LISTING

This application contains a Sequence Listing in computer readable form. The computer readable form is incorporated herein by reference.

### FIELD OF THE INVENTION

The present invention relates to detergent compositions and methods of cleaning comprising certain DNase enzymes having improved properties such as improved stability for example, in detergent and/or on storage. The compositions are preferably laundry detergent compositions and/or hard surface cleaning compositions, for example, dish-washing compositions, and are suitable for use in hand-washing or automatic washing. The invention also relates to methods of making such detergent compositions and methods of cleaning surfaces by hand and/or in automatic washing machines and methods of using such compositions.

### BACKGROUND OF THE INVENTION

Hard surfaces and fabrics are exposed to soils and microorganisms from the environment and from body soils. Microorganisms contained in deposited soil may promote the formation of soils of microbial origin, for example biofilm soils. For example, on laundry, examples of biofilm-producing bacteria include *Acinetobacter* sp., *Aeromicrobium* sp., *Brevundimonas* sp., *Microbacterium* sp., *Micrococcus luteus, Pseudomonas* sp., *Staphylococcus epidermidis* and *Stenotrophomonas* sp. Biofilm comprises a matrix of extracellular polymeric substance (EPS): a polymeric mixture generally comprising extracellular DNA, proteins, and polysaccharides. These soils are sticky, difficult to remove and tend to cause further adhesion of other soils. Thus, household care-related soils are complex and may comprise particulates, protein, starch, grease and also EPS. Consequently, stain removal is also complex and a variety of enzymes may be useful, including deoxyribonuclease (DNase) enzymes.

Detergent compositions comprising DNases are already known. However, to be useful in household cleaning processes an enzyme such as a DNase needs to be stable in detergent compositions, for example in the presence of detergent components such as surfactants, builders, and/or bleaches etc. The present invention provides detergent compositions comprising DNase enzymes having good stability therein.

### SUMMARY OF THE INVENTION

The present invention provides a detergent composition comprising a polypeptide having DNase activity and which has good stability in a detergent composition, and a cleaning adjunct. The polypeptide is a DNase variant which compared to the polypeptide of SEQ ID NO: 1 comprises at least one and preferably two or more substitutions selected from the group consisting of N61D, T65I, T65V, S82R, K107Q, T127S, T127V, G149N, S164D and L181S, wherein the variant has at least 60% sequence identity to SEQ ID NO: 1 and has DNase activity.

The invention also provides a method of treating a surface, preferably a fabric, the method comprising contacting a surface with an aqueous wash liquor comprising a polypeptide having DNase activity and wherein the polypeptide is a DNase variant which compared to the polypeptide of SEQ ID NO: 1 comprises at least one substitution, and typically two or more substitutions selected from the group consisting of N61D, T65I, T65V, S82R, K107Q, T127S, T127V, G149N, S164D and L181S, wherein the variant has at least 60% sequence identity to SEQ ID NO: 1, and a cleaning adjunct.

Preferably the surface is contacted with the aqueous wash liquor at a temperature of 60°C or less, or more preferably at a temperature of 40°C or less or 35°C or less, most preferably at a temperature of 30°C or less than 30°C; and (iii) rinsing the surface. The compositions and methods herein are particularly useful for treating any surface, particularly fabrics and/or hard surfaces such as dishware, for example made from synthetic or natural materials, including cotton, wool, silk, polyester, nylon, elastane or mixed fabrics, such as polycotton.

The invention also relates to the use of a composition or method as described above for: improved cleaning, such as deep cleaning; maintaining or improving whiteness of a fabric; improved soil removal from a surface, such as a fabric; malodour reduction or removal from a surface, such as a fabric; anti-wrinkle benefits on a fabric; improved drying of a fabric; soil anti-redeposition benefits, prevention and/or reduction of stickiness of a surface, pretreatment of stains, prevention and /or reduction of adherence of soil to a surface.

The invention also relates to a method of making a detergent composition comprising preparing a polypeptide having DNase activity as described herein and mixing with a cleaning adjunct.

### DETAILED DESCRIPTION OF THE INVENTION

### Sequences

SEQ ID NO: 1 variant of the polypeptide of SEQ ID NO: 27 from *Bacillus cibi*
SEQ ID NO: 2 mature polypeptide obtained from *Bacillus horikoshii*
SEQ ID NO: 3 mature polypeptide obtained from *Bacillus sp-62520*
SEQ ID NO: 4 mature polypeptide obtained from *Bacillus horikoshii*
SEQ ID NO: 5 mature polypeptide obtained from *Bacillus horikoshii*
SEQ ID NO: 6 mature polypeptide obtained from *Bacillus sp-16840*
SEQ ID NO: 7 mature polypeptide obtained from *Bacillus sp-16840*
SEQ ID NO: 8 mature polypeptide obtained from *Bacillus sp-62668*
SEQ ID NO: 9 mature polypeptide obtained from *Bacillus sp-13395*
SEQ ID NO: 10 mature polypeptide obtained from *Bacillus horneckiae*
SEQ ID NO: 11 mature polypeptide obtained from *Bacillus sp-11238*
SEQ ID NO: 12 mature polypeptide obtained from *Bacillus sp-62451*
SEQ ID NO: 13 mature polypeptide obtained from *Bacillus sp-18318*
SEQ ID NO: 14 mature polypeptide obtained from *Bacillus idriensis*
SEQ ID NO: 15 is the mature polypeptide obtained from *Bacillus algicola*
SEQ ID NO: 16 mature polypeptide obtained from *environmental sample J*
SEQ ID NO: 17 mature polypeptide obtained from *Bacillus vietnamensis*
SEQ ID NO: 18 mature polypeptide obtained from *Bacillus hwajinpoensis*
SEQ ID NO: 19 mature polypeptide obtained from *Paenibacillus mucilaginosus*
SEQ ID NO: 20 mature polypeptide obtained from *Bacillus indicus*
SEQ ID NO: 21 mature polypeptide obtained from *Bacillus marisflavi*
SEQ ID NO: 22 mature polypeptide obtained from *Bacillus luciferensis*
SEQ ID NO: 23 mature polypeptide obtained from *Bacillus marisflavi*
SEQ ID NO: 24 mature polypeptide obtained from *Bacillus sp. SA2-6*
SEQ ID NO: 25 motif [D/M/L][S/T]GYSR[D/N]
SEQ ID NO: 26 motif ASXNRSKG
SEQ ID NO: 27 mature polypeptide obtained from *Bacillus cibi*
SEQ ID NO: 28 protease variant

### Definitions

The term "clade" means a group of polypeptides clustered together based on homologous features traced to a common ancestor. Polypeptide clades can be visualized as phylogenetic trees, and a clade is a group of polypeptides that consists of a common ancestor and all of its lineal descendants. Polypeptides forming a group, e.g. a clade, as shown in a phylogenetic tree often share common properties and are more closely related than other polypeptides not belonging to the clade.

The term "deep cleaning" refers to disruption or removal of components of organic matter, e.g. biofilm, such as polysaccharides, proteins, DNA, soil or other components present in the organic matter.

The terms "DNase", "DNase variant" and "DNase parent" refer to a polypeptide with DNase activity (i.e. deoxyribonuclease activity) that catalyzes the hydrolytic cleavage of phosphodiester linkages in DNA, thus degrading DNA. DNases belong to the esterases (EC-number 3.1), a subgroup of the hydrolases. The DNases are classified e.g. in E.C. 3.1.11, E.C. 3.1.12, E.C. 3.1.15, E.C. 3.1.16, E.C. 3.1.21, E.C 3.1.22, E.C 3.1.23, E.C 3.1.24 and E.C.3.1.25 as well as EC 3.1.21.X, where X=1, 2, 3, 4, 5, 6, 7, 8 or 9. The term "DNase" and the expression "a polypeptide with DNase activity" may be used interchangeably throughout the application. For purposes of the present invention, DNase activity may be determined according to the procedure described in Assay I below. The DNase variants of the present invention preferably have at least one improved property compared to the parent DNase. In one embodiment, the DNase variants have improved stability, e.g. improved storage stability in a detergent composition, compared to the parent DNase. Preferably, DNase variants have a half-life improvement factor (T½ IF, or HIF) of at least 1.1, more preferably at least 1.2, preferably at least 1.5, more preferably at least 2, such as at least 3, after storage in a liquid detergent, determined e.g. as described in Example 3 herein, relative to the half-life of a reference DNase, for example the DNase of SEQ ID NO: 1 and/or the parent DNase. The half-life improvement factor of a DNase variant is calculated as the half-life of a DNase variant relative to the half-life of the reference DNase.

The term "effective amount" in relation to an enzyme refers to the quantity of enzyme necessary to achieve the enzymatic activity required in the specific application, e.g., in a defined detergent composition. Such effective amounts are readily ascertained by one of ordinary skill in the art and are based on many factors, such as the particular enzyme used, the cleaning application, the specific composition of the detergent composition, and whether a liquid or dry (e.g., granular, bar) composition is required, and the like. The term "effective amount" of a DNase variant refers to the quantity of DNase variant described hereinbefore that achieves a desired level of enzymatic activity, e.g., in a defined detergent composition.

The term "expression" includes any step involved in the production of the variant including, but not limited to, transcription, post-transcriptional modification, translation, post-translational modification, and secretion.

The term "expression vector" means a linear or circular DNA molecule that comprises a polynucleotide encoding a variant and is operably linked to additional nucleotides that provide for its expression.

The term "fabric" encompasses any textile material. Thus, it is intended that the term encompass garments, as well as fabrics, yarns, fibers, non-woven materials, natural materials, synthetic materials, and any other textile material.

The term "host cell" means any cell type that is susceptible to transformation, transfection, transduction, and the like with a nucleic acid construct or expression vector comprising a polynucleotide of the present invention. The term "host cell" encompasses any progeny of a parent cell that is not identical to the parent cell due to mutations that occur during replication.

The term "improved property" means a characteristic associated with a variant that is improved compared to the parent and/or compared to the DNase polypeptide having SEQ ID NO: 1, or compared to a DNase having the identical amino acid sequence of said variant but not having the alterations defined herein at one or more of said specified positions. Such improved properties include, but are not limited to, stability, such as detergent stability, e.g. detergent storage stability, and wash performance, e.g. deep cleaning effect, where the deep-cleaning effect may include a degluing effect. The DNase variants preferably have improved stability, in particular improved detergent storage stability, compared to the polypeptide of SEQ ID NO: 1.

The term "improved DNase activity" is defined herein as an altered DNase activity e.g. by increased hydrolytic cleavage of phosphodiester linkages in the DNA by a DNase variant relative to the activity of the parent DNase, such as compared to a DNase with SEQ ID NO: 1.

The term "improved wash performance" includes but is not limited to the term "deep cleaning effect". Improved performance e.g. deep cleaning performance of a DNase variant according to the invention is measured compared to the DNase parent, e.g. the DNase shown in SEQ ID NO: 1 or SEQ ID NO: 27. The improved performance e.g. deep cleaning performance may be expressed as a Remission value of the stained swatches. After washing and rinsing the swatches are spread out flat and allowed to air dry at room temperature overnight. All washed swatches are evaluated the day after washing. Light reflectance evaluations of the swatches are performed using a Macbeth Color Eye 7000 reflectance spectrophotometer with a very small aperture. The measurements are made without UV in the incident light and remission at 460 nm is extracted. A positive response indicates that soil has been removed; this may include soiled that sticks to the fabric due to e.g. a sticky biofilm layer.

The term "mature polypeptide" means a polypeptide in its final form following translation and any post-translational modifications, such as Nterminal processing, Cterminal truncation, glycosylation, phosphorylation, etc. The N-terminals of a mature polypeptide may be experimentally determined based on EDMAN N-terminal sequencing data and Intact MS data. It is known in the art that a host cell may produce a mixture of two of more different mature polypeptides (*i.e.*, with a different C-terminal and/or N-terminal amino acid) expressed by the same polynucleotide.

The term "mature polypeptide coding sequence" means a polynucleotide that encodes a mature polypeptide having DNase activity.

The term "nucleic acid construct" means a nucleic acid molecule, either single- or doublestranded, which is isolated from a naturally occurring gene or is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence of the present invention.

The term "operably linked" means a configuration in which a control sequence is placed at an appropriate position relative to the coding sequence of a polynucleotide such that the control sequence directs the expression of the coding sequence.

The terms "parent DNase", "DNase parent" or "precursor DNase" may be used interchangeably. In the context of the present invention, a "parent DNase" is to be understood as a DNase into which at least one alteration is made in the amino acid sequence to produce a DNase variant having an amino acid sequence which is less than 100% identical to the DNase sequence into which the alteration was made. Thus, the parent is a DNase having identical amino acid sequence compared to the variant but not having the alterations at one or more of the specified positions. The DNase parent may be a DNase having at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 80%, at least 81%, at least 82%, at least 83%, at least 84%, at least 85%, at least 90%, at least 91%, at least 92%, at least 93%, at least 94%, at least 95%, at least 96%, at least 97%, at least 98%, at least 99% or 100% identity to a polypeptide shown in SEQ ID NO: 1.

The term "relevant washing conditions" is used herein to indicate the conditions, particularly washing temperature, time, washing mechanics, detergent concentration, type of detergent and water hardness, used in households in a detergent market segment.

The term "stability" includes storage stability and stability during use, e.g. during a wash process and reflects the stability of the DNase variant according to the invention as a function of time, e.g. how much activity is retained when the DNase variant is kept a detergent or in a detergent solution. The stability is influenced by many factors such as pH, temperature, detergent composition, e.g. amount of builder, surfactants etc. The DNase stability may be measured as described in Example 3. The term "improved stability" or "increased stability" is defined herein as a variant DNase displaying an increased stability in solution, e.g. in a detergent solution, relative to the stability of the parent DNase and/or relative to SEQ ID NO: 1. "Improved stability" and "increased stability" may be detergent stability during storage, or in-use ("in-wash stability").

The term "variant" means a polypeptide having DNase activity and which comprises a substitution at one or more positions as defined elsewhere herein. A substitution means replacement of the amino acid occupying a position with a different amino acid, a deletion means removal of an amino acid occupying a position, and an insertion means adding amino acids e.g. 1 to 10 amino acids, preferably 1-3 amino acids, adjacent to an amino acid occupying a position. The variants of the present invention have at least 20%, *e.g.*, at least 40%, at least 50%, at least 60%, at least 70%, at least 80%, at least 90%, at least 95%, or at least 100% of the DNase activity of the polypeptide of SEQ ID NO: 1.

The term "wash liquor" refers to an aqueous solution comprising a DNase variant as described herein and a cleaning adjunct.

The term "water hardness" or "degree of hardness" or "dH" or "°dH" as used herein refers to German degrees of hardness. One degree is defined as 10 milligrams of calcium oxide per liter of water.

### Conventions for Designation of Variants

Unless indicated otherwise, the polypeptide disclosed in SEQ ID NO: 1 is used for purposes of the present invention to determine the corresponding amino acid residue in another DNase. The amino acid sequence of another DNase is aligned with the polypeptide disclosed in SEQ ID NO: 1, and based on the alignment, the amino acid position number corresponding to any amino acid residue in the polypeptide disclosed in SEQ ID NO: 1 is determined using e.g. the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix.

Identification of the corresponding amino acid residue in another DNase can be determined by an alignment of multiple polypeptide sequences using several computer programs including, but not limited to, MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680), using their respective default parameters.

When another enzyme has diverged from the polypeptide of SEQ ID NO: 1 such that traditional sequence-based comparison fails to detect their relationship (Lindahl and Elofsson, 2000, J. Mol. Biol. 295: 613-615), other pairwise sequence comparison algorithms can be used. Greater sensitivity in sequence-based searching can be attained using search programs that utilize probabilistic representations of polypeptide families (profiles) to search databases. For example, the PSI-BLAST program generates profiles through an iterative database search process and is capable of detecting remote homologs (Atschul et al., 1997, Nucleic Acids Res. 25: 3389-3402). Even greater sensitivity can be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources (PSI-BLAST, secondary structure prediction, structural alignment profiles, and solvation potentials) as input to a neural network that predicts the structural fold for a query sequence. Similarly, the method of Gough et al., 2000, J. Mol. Biol. 313: 903-919, can be used to align a sequence of unknown structure with the superfamily models present in the SCOP database. These alignments can in turn be used to generate homology models for the polypeptide, and such models can be assessed for accuracy using a variety of tools developed for that purpose.

For proteins of known structure, several tools and resources are available for retrieving and generating structural alignments. For example, the SCOP super families of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (Shindyalov and Bourne, 1998, Protein Engineering 11: 739-747), and implementation of these algorithms can additionally be utilized to query structure databases with a structure of interest in order to discover possible structural homologs (*e.g.*, Holm and Park, 2000, Bioinformatics 16: 566-567).

As different amino acids may be present at a given position depending on the selected parent for the variants the amino acid positions are indicated with #₁, #₂, etc. in the definitions below. In describing the variants of the present invention, the nomenclature described below is adapted for ease of reference. The accepted IUPAC single letter or three letters amino acid abbreviations are employed.

Substitutions: For an amino acid substitution, the following nomenclature is used: Original amino acid, position, substituted amino acid. Accordingly, the substitution of valine at position #₁ with alanine is designated as "Val#₁Ala" or "V#₁A". Multiple mutations are separated by addition marks ("+") or by commas (,), *e.g*., "Val#₁Ala + "Pro#₂Gly" or V#₁A, P#₂G, representing substitutions at positions #₁ and #₂ of valine (V) and proline (P) with alanine (A) and glycine (G), respectively. If more than one amino acid may be substituted in a given position these are listed in brackets, such as [X] or {X}. Thus, if both Trp and Lys may be substituted instead of the amino acid occupying at position #₁ this is indicated as X#₁ {W, K}, X#₁ [W, K] or X#₁ [W/K], where the X indicate the amino acid residue present at the position of the parent DNase e.g. such as a DNase shown in SEQ ID NO: 1 or a DNase having at least 60% identity hereto. In some cases, the variants may be represented as #₁ {W, K} or X#₂P indicating that the amino acids to be substituted vary depending on the parent.

For convenience, as SEQ ID NO: 1 is used for numbering the substitutions and other mutations disclosed herein, the amino acid in the corresponding position in SEQ ID NO: 1 is indicated, for example, T65V. However, it will be clear to the skilled artisan that a DNase variant comprising T65V is not limited to parent DNases having threonine at a position corresponding to position 65 of SEQ ID NO: 1. In a parent DNase having e.g. asparagine in position 65, the skilled person would translate the mutation specified herein as T65V to N65V. In the event a parent DNase has valine in position 65, the skilled person would recognize that the parent DNase is not changed at this position. The same applies for deletions and insertions described below. This may also be indicated using an "X", which is defined to be any of the 20 natural amino acids, as the original amino acid, e.g. X65V means that any amino acid residue other than V in position 65 is altered to V.

Deletions: For an amino acid deletion, the following nomenclature is used: Original amino acid, position, ^{∗}. Accordingly, the deletion of valine at position #₁ is designated as "Val#₁^{∗}" or "V#₁^{∗}". Multiple deletions are separated by addition marks ("+") or commas, *e.g*., "Val#₁^{∗} + Pro#₂^{∗}" or "V#₁^{∗}, P#₂^{∗}".

Insertions: The insertion of an additional amino acid residue such as *e.g.* a lysine after Val#₁ may be indicated by: Val#₁ValLys or V#₁VK. Alternatively, insertion of an additional amino acid residue such as lysine after V#₁ may be indicated by: ^{∗}#₁aK. When more than one amino acid residue is inserted, such as *e.g.* a Lys, and Gly after #₁ this may be indicated as: Val#₁ValLysGly or V#₁VKG. In such cases, the inserted amino acid residue(s) may also be numbered by the addition of lower case letters to the position number of the amino acid residue preceding the inserted amino acid residue(s), in this example: ^{∗}#₁aK ^{∗}#₁bG.

Multiple alterations: Variants comprising multiple alterations are separated by addition marks ("+") or by commas (,), *e.g*., "Val#₁Trp+Pro#₂Gly", "V#₁W, P#₂G" or "V#₁W + P#₂G" representing a substitution of valine and proline at positions #₁ and #₂ with tryptophan and glycine, respectively as described above.

Different alterations: Where different alterations can be introduced at a position, the different alterations may be separated by a comma, *e.g*., "Val#₁Trp, Lys" or V#₁W, K representing a substitution of valine at position #₁ with tryptophan or lysine. Thus, "Val#₁Trp, Lys + Pro#₂Asp" designates the following variants: "Val#₁Trp+Pro#₂Asp", "Val#₁Lys+Pro#₂Asp" or V#₁W, K + P#₂D.

Different alterations may also be indicated as using the nomenclature [IV] or [I/V], which means that the amino acid at this position may be isoleucine (Ile, I) or valine (Val, V). Likewise, the nomenclature [LVI] and [L/V/I] means that the amino acid at this position may be a leucine (Leu, L), valine (Val, V) or isoleucine (Ile, I), and so forth for other combinations as described herein. For example, T65I/V refers to a substitution of T in position 65 with either I or V.

### DETAILED DESCRIPTION OF THE INVENTION

The present invention provides a detergent composition comprising a DNase variant and a cleaning adjunct. The DNase variant when compared to the polypeptide of SEQ ID NO: 1 comprises at least one substitution selected from the group consisting of N61D, T65I, T65V, S82R, K107Q, T127S, T127V, G149N, S164D and L181S, wherein the variant has at least 60% sequence identity to SEQ ID NO: 1, such as at least 65%, at least 70%, at least 75% or at least 80% or at least 81%, or at least 82%, or at least 83%, or at least 84%, or at least 85%, or at least 86%, or at least 87%, or at least 88%, or at least 89%, or at least 90% sequence identity to SEQ ID NO: 1. In a preferred embodiment, the DNase variant comprises at least one, preferably at least two or more substitutions selected from the group consisting of N61D, T65I, T65V, S82R, K107Q, T127S, T127V, G149N, S164D and L181S.

The detergent composition of the present invention is for laundry cleaning and/or treatment and/or hard surface cleaning or treatment, particularly dishwashing. The composition of the invention may be a solid (for example, a bar, tablet, powder/granules), liquid, gel and/or paste or may be in the form of a sheet. A preferred form is a unit dose form which may be a tablet, sheet, or preferably a water-soluble pouch comprising liquid and/or solid compositions. Preferred are multi-compartment pouches. Preferred unit dose compositions comprise solid (preferably in the form of a powder), or liquid or a combination thereof, preferably in a multi-compartment unit dose. Especially preferred is a unit dose form comprising liquid detergent encapsulated in water-soluble material in the form of a pouch, optionally in the form of a multi-component pouch.

### DNASE VARIANTS

In one aspect, the DNase variant when compared to the polypeptide of SEQ ID NO: 1 comprises one or preferably two or more substitutions selected from the group consisting of N61D, T65I, T65V, S82R, K107Q, T127S, T127V, G149N, S164D and L181S, wherein the variant has at least 60% sequence identity to SEQ ID NO: 1, e.g. at least 70%, at least 75% or at least 80% or at least 81%, or at least 82%, or at least 83%, or at least 84%, or at least 85%, or at least 86%, or at least 87%, or at least 88%, or at least 89%, or at least 90% or at least 95% sequence identity to SEQ ID NO:1, and has DNase activity.

The DNase variant may comprise two, three, four, five or more of said substitutions, typically two, three, four or five, and optionally with one or more additional substitutions described herein.

In one embodiment, the DNase variant comprises the substitutions N61D+T65I, and optionally one or more additional substitutions described herein.

In one embodiment, the DNase variant comprises the substitutions N61D+T65V, and optionally one or more additional substitutions described herein.

In one embodiment, the DNase variant comprises the substitutions N61D+S82R, and optionally one or more additional substitutions described herein.

In one embodiment, the DNase variant comprises the substitutions N61D+K107Q, and optionally one or more additional substitutions described herein.

In one embodiment, the DNase variant comprises the substitutions N61D+T127S, and optionally one or more additional substitutions described herein.

In one embodiment, the DNase variant comprises the substitutions N61D+T127V, and optionally one or more additional substitutions described herein.

In one embodiment, the DNase variant comprises the substitutions N61D+G149N, and optionally one or more additional substitutions described herein.

In one embodiment, the DNase variant comprises the substitutions N61D+S164D, and optionally one or more additional substitutions described herein.

In one embodiment, the DNase variant comprises the substitutions N61D+L181S, and optionally one or more additional substitutions described herein.

In one embodiment, the DNase variant comprises the substitutions T65I+S82R, and optionally one or more additional substitutions described herein.

In one embodiment, the DNase variant comprises the substitutions T65I+K107Q, and optionally one or more additional substitutions described herein.

In one embodiment, the DNase variant comprises the substitutions T65I+T127S, and optionally one or more additional substitutions described herein.

In one embodiment, the DNase variant comprises the substitutions T65I+T127V, and optionally one or more additional substitutions described herein.

In one embodiment, the DNase variant comprises the substitutions T65I+G149N, and optionally one or more additional substitutions described herein.

In one embodiment, the DNase variant comprises the substitutions T65I+S164D, and optionally one or more additional substitutions described herein.

In one embodiment, the DNase variant comprises the substitutions T65I+L181S, and optionally one or more additional substitutions described herein.

In one embodiment, the DNase variant comprises the substitutions T65V+S82R, and optionally one or more additional substitutions described herein.

In one embodiment, the DNase variant comprises the substitutions T65V+K107Q, and optionally one or more additional substitutions described herein.

In one embodiment, the DNase variant comprises the substitutions T65V+T127S, and optionally one or more additional substitutions described herein.

In one embodiment, the DNase variant comprises the substitutions T65V+T127V, and optionally one or more additional substitutions described herein.

In one embodiment, the DNase variant comprises the substitutions T65V+G149N, and optionally one or more additional substitutions described herein.

In one embodiment, the DNase variant comprises the substitutions T65V+S164D, and optionally one or more additional substitutions described herein.

In one embodiment, the DNase variant comprises the substitutions T65V+L181S, and optionally one or more additional substitutions described herein.

In one embodiment, the DNase variant comprises the substitutions S82R+K107Q, and optionally one or more additional substitutions described herein.

In one embodiment, the DNase variant comprises the substitutions S82R+T127S, and optionally one or more additional substitutions described herein.

In one embodiment, the DNase variant comprises the substitutions S82R+T127V, and optionally one or more additional substitutions described herein.

In one embodiment, the DNase variant comprises the substitutions S82R+G149N, and optionally one or more additional substitutions described herein.

In one embodiment, the DNase variant comprises the substitutions S82R+S164D, and optionally one or more additional substitutions described herein.

In one embodiment, the DNase variant comprises the substitutions S82R+L181S, and optionally one or more additional substitutions described herein.

In one embodiment, the DNase variant comprises the substitutions K107Q+T127S, and optionally one or more additional substitutions described herein.

In one embodiment, the DNase variant comprises the substitutions K107Q+T127V, and optionally one or more additional substitutions described herein.

In one embodiment, the DNase variant comprises the substitutions K107Q+G149N, and optionally one or more additional substitutions described herein.

In one embodiment, the DNase variant comprises the substitutions K107Q+S164D, and optionally one or more additional substitutions described herein.

In one embodiment, the DNase variant comprises the substitutions K107Q+L181S, and optionally one or more additional substitutions described herein.

In one embodiment, the DNase variant comprises the substitutions T127S+G149N, and optionally one or more additional substitutions described herein.

In one embodiment, the DNase variant comprises the substitutions T127S+S164D, and optionally one or more additional substitutions described herein.

In one embodiment, the DNase variant comprises the substitutions T127S+L181S, and optionally one or more additional substitutions described herein.

In one embodiment, the DNase variant comprises the substitutions T127V+G149N, and optionally one or more additional substitutions described herein.

In one embodiment, the DNase variant comprises the substitutions T127V+S164D, and optionally one or more additional substitutions described herein.

In one embodiment, the DNase variant comprises the substitutions T127V+L181S, and optionally one or more additional substitutions described herein.

In one embodiment, the DNase variant comprises the substitutions G149N+S164D, and optionally one or more additional substitutions described herein.

In one embodiment, the DNase variant comprises the substitutions G149N+L181S, and optionally one or more additional substitutions described herein.

In one embodiment, the DNase variant comprises the substitutions S164D+L181S, and optionally one or more additional substitutions described herein.

In a preferred embodiment, the DNase variant comprises the substitutions T65I or T65V together with at least two of the substitutions N61D, S82R, K107Q, T127S, T127V, G149N, S164D and L181S.

In another preferred embodiment, the DNase variant comprises the substitution N61D together with at least two of the substitutions T65I/V, S82R, K107Q, T127S/V, G149N, S164D and L181S, for example two, three or four of said substitutions. In a preferred embodiment, the variant comprises N61D together with at least two of the substitutions T65I/V, S82R, K107Q, T127S and S164D, for example two, three or four of said substitutions.

In another preferred embodiment, the DNase variant comprises the substitution S82R together with at least two of the substitutions N61D, T65I, T65V, K107Q, T127S, T127V, G149N, S164D and L181S, for example two, three or four of said substitutions.

In another preferred embodiment, the DNase variant comprises the substitution K107Q together with at least two of the substitutions N61D, T65I, T65V, S82R, T127S, T127V, G149N, S164D and L181S, for example two, three or four of said substitutions.

In another preferred embodiment, the DNase variant comprises the substitution T127S together with at least two of the substitutions N61D, T65I, T65V, S82R, K107Q, G149N, S164D and L181S, for example two, three or four of said substitutions.

In another preferred embodiment, the DNase variant comprises the substitution G149N together with at least one of the substitutions N61D, T65I, T65V, S82R, K107Q, T127S, T127V, S164D and L181S, for example two, three or four of said substitutions.

In another preferred embodiment, the DNase variant comprises the substitution S164D together with at least one of the substitutions N61D, T65I, T65V, S82R, K107Q, T127S, T127V, G149N and L181S, for example two, three or four of said substitutions.

In some embodiments, the variants may further comprise at least one substitution selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W, for example one, two or three or more of said substitutions, typically one, two or three.

Thus, the invention also provides a DNase variant which compared to the polypeptide of SEQ ID NO: 1 comprises at least one substitution, e.g. two or more substitutions, selected from the group consisting of N61D, T65I, T65V, S82R, K107Q, T127S, T127V, G149N, S164D and L181S; and at least one substitution, e.g. two or more substitutions, selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W; wherein the variant has at least 60% sequence identity to SEQ ID NO: 1, e.g. at least 65%, at least 70%, at least 75%, at least 80%, at least 85%, at least 90% or at least 95% sequence identity, and has DNase activity.

The DNase variant preferably comprises a set of substitutions selected from the group consisting of:
- K21L+Q48D+T65I+S82R+K107Q+T127S;
- Q14R+K21L+Q48D+T65I+T127S;
- Q48D+T65I+S82R+T127S+S164D;
- N61D+T65I+K107Q+T127S+S164D;
- Q48D+T65I+S82R+K107Q+T127S;
- Q14R+N61D+T65I+S82R+K107Q;
- N61D+S68L+G149N;
- Q14R+N61D+T65I+S82R+T127S+S164D;
- K21L+Q48D+T65I+S82R+K107Q;
- Q14R+T65I+K107Q+T127S;
- N61D+T65I+S82R+T127S+S164D;
- K21L+N61D+T65I+S82R+K107Q+T127S;
- T65V+T127V+G149N;
- T65I+K107Q+T127S+S164D;
- N61D+T65I+S82R+K107Q;
- Q14R+K21L+N61D+T65I+S82R;
- Q14R+K21L+N61D+T65I+S82R+K107Q;
- Q14R+K21L+N61D+T65I+T127S;
- N61D+T65I+S82R+K107Q+T127S+S164D;
- Q14R+K21L+T65I+K107Q+T127S;
- N61D+T65I+K107Q+T127S;
- T65I+S82R+K107Q+S164D;
- K21L+N61D+T65I+S82R;
- K21L+N61D+T65I+T127S;
- N61D+T65I+S82R+S164D;
- K21L+N61D+T65I+S82R+K107Q;
- S68L+S106A+G149N;
- N61D+T65I+T127S+S164D;
- Q14R+K21L+N61D+T65I;
- Q14R+K21L+T65I+T127S;
- T65V+G149N;
- T65V+R109T+T127V;
- Q14R+K21L+T65I+K107Q;
- K21L+T65I+S82R+K107Q;
- K21L+T65I+K107Q+T127S;
- N61D+S68L+S102Y+G149N+S164D+L181T;
- N61D+S68L+S106A+G149N+S164D;
- T65V+R109T+G149N;
- T65V+T127V+T171W;
- T65V+T127V+L181S;
- T65I+S164D+L181W;
- N61D+S66Y+S102Y+S164D;
- N61D+S66Y+S164D;
- N61D+T65V+S164D;
- Q14W+N61D+T65I;
- Q14W+N61D+T65I+S164D;
- Q14W+N61D+T65I+S164D+L181W;
- T65I+D116W+S164D+L181W;
- T65I+D116W+S164D;
- Q14W+T65I+S164D;
- R109T+G149N;
- G149N+T171W;
- G149N;
- S164D;
- P25S+L33K+D56I+T65V+Y77T+T127V+L181S;
- P25S+L33K+T65V+Y77T+T127V+L181S;
- P25S+L33K+D56I+T65V+Y77T+R109Q+T127V+L181S;
- P25S+L33K+D56I+T65V+Y77T+D116S+T127V+L181S;
- D56L+T65V+T127V;
- D56L+T65V+T127V+T171W;
- T65V+G149N+T171W;
- Q48D+T65I+K107Q+T127S+S164D;
- T65V+Y77T+T127V;
- T65V+R109T+T127V+G149N;
- T65V+R109T+T171W;
- T65V+R109T+G149N+T171W;
- P25S+D56I+T65V+Y77T+T127V+L181S;
- Q14R+K21L+T65I+K107Q+T127S;
- N61D+S68L+S102Y;
- S66Y+T127V+L181S;
- N61D+T65I+S82R+K107Q+L181D;
- T65V+G149N+L181E;
- T65V+T127V+G149N+Y182D;
- Q48D+N61D+T65I+S82R+K107Q;
- Q48D+T65V+G149N;
- N61D+T65I+S82R+T127S+S164D+Y182D;
- N61D+T65I+S82R+T127S+S164D+L181D;
- Q48D+N61D+T65I+K107Q+T127S+S164D;
- N61D+T65I+S82R+T127S+S164D+Y182N;
- T65I+K107Q+T127S+S164D+Y182D;
- N61D+T65I+K107Q+T127S+S164D+L181E;
- N61D+T65I+K107Q+T127S+S164D+L181D;
- T65I+K107Q+T127S+S164D+L181T;
- T65I+K107Q+T127S+S164D+L181E;
- N61D+T65I+K107Q+T127S+S164D+Y182D;
- T65I+K107Q+T127S+S164D+Y182N;
- N61D+T65I+K107Q+T127S+S164D+L181Q;
- N61D+T65V+S164D+Y182N;
- N61D+T65I+K107Q+T127S+S164D+L181T;
- T65I+K107Q+T127S+S164D+L181D;
- T65I+K107Q+T127S+S164D+L181Q;
- N61D+T65V+T127S+S164D;
- Q48D+N61D+T65V+S164D;
- K21L+N61D+T65I+K107Q+T127S;
- Q14W+N61D+T65I+R109T+G149N+S164D+L181W;
- K21L+N61D+T65I+K107Q;
- Q14W+N61D+T65I+R109T+G149N;
- Q14W+T65V+R109T+G149N+W154I+L181W;
- Q14W+T65V+R109T+G149N+W154I+S164D;
- Q14W+N61D+T65V+R109T+G149N+L181W;
- Q14W+T65I+R109T+D116W+G149N+S164D+L181W;
- T65V+R109T+T127V+T171W;
- R109T+T127V+T171W;
- N61D+T65I+S82R+T127S+S164D+T171E+D175G+L181S;
- T65V+Y77R+G149N;
- T65V+Y77H+G149N;
- T65V+L76K+G149N;
- T65V+L76R+G149N;
- T19E+P25S+L33K+D56I+T65V+Y77T+T127V+L181S;
- T65I+L181S;
- S66L+G149N;
- T65V+Y77T+G149N+L181S;
- T65V+Y77T+G149N;
- D56N+T65V+Y77T+G149N;
- D56N+T65V+L76H+G149N;
- D56Q+T65V+L76H+G149N;
- S68Q+G149N+L181S;
- G149N+L181S;
- S66L+K107E+G149N+Q166D+L181S;
- T65I+G149N+Q166D;
- T65I+G149N+Q166D+Y182G;
- T65I+G149N+Q166D+L181S;
- T65I+G149N+L181S;
- T65I+Q166D+Y182G;
- T65I+Y182G;
- S68Q+G149N+Q166D+L181S;
- T65V+G149N+L181T;
- T19I+S68Q+G149N+Y182G;
- T65I+K107E+G149N+Q166D+Y182G;
- G149N+Q166D+Y182G;
- T65I+K107E+G149N;
- S68Q+G149N+Q166D;
- G149N+Y182G;
- G149N+Q166D+L181S;
- S68Q+K107E+G149N+Y182G;
- K107E+G149N+Y182G;
- T19E+T65I+K107Q+T127S+S164D; and
- N61D+T65I+K107Q+T127S+S164D+Y182N..

Preferably the DNase variant may comprise or consist of SEQ ID NO: 1 with one of the sets of substitutions listed above.

Preferably the DNase variant comprises a set of substitutions selected from the group consisting of:
- T65V+T127V+L181S;
- N61D+T65I+S82R+K107Q;
- T65V+G149N;
- N61D+T65I+K107Q+T127S+S164D;
- N61D+T65I+T127S+S164D;
- N61D+T65V+S164D;
- T65V+T127V+G149N;
- N61D+T65I+S82R+T127S+S164D; and
- T65I+K107Q+T127S+S164D.

In a further preferred embodiment, the DNase variant may comprise or consist of SEQ ID NO: 1 with one of the preferred sets of substitutions listed above.

Preferably, the DNase variants comprise at least 5, such as at least 10, such as at least 15, of the indicated amino acid residues in the following positions: I in position 1, Y in position 13, P in position 22, P in position 25, L in position 27, P in position 39, G in position 42, W in position 57, V in position 59, L in position 76, Y in position 77, R in position 109, D in position 116, P in position 144, H in position 147, L in position 167, D in position 175 and L in position 181.

The DNase variants may e.g. comprise 10, 11, 12, 13, 14, 15, 16, 17 or 18 of the indicated amino acid residues in the following positions: I in position 1, Y in position 13, P in position 22, P in position 25, L in position 27, P in position 39, G in position 42, W in position 57, V in position 59, L in position 76, Y in position 77, R in position 109, D in position 116, P in position 144, H in position 147, L in position 167, D in position 175 and L in position 181.

The DNase variant preferably comprises, in addition to the substitutions disclosed elsewhere herein, one or more substitutions, such as two, three, four or more substitutions, selected from the group consisting of I1T, Y13S, P22T, P25S, L27S, P39S, G42S, W57S, V59S, L76V, Y77T, R109Q, D116S, P144S, H147A, L167S and D175G.

The DNase variant preferably comprises additional alterations, typically substitutions, in one or more positions of SEQ ID NO: 1 selected from the group consisting of positions 14, 21, 25, 33, 48, 56, 66, 68, 77, 102, 106, 109, 116, 171 and 181. Preferred substitutions in these positions include one or more substitutions selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W.

In one aspect, the invention provides a DNase variant which compared to the polypeptide of SEQ ID NO: 1 comprises a substitution selected from the group consisting of N61D, T65I, T65V, S82R, K107Q, T127S, T127V, G149N, S164D and L181S, and one or more substitutions selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W, wherein the variant has at least 60% sequence identity to SEQ ID NO: 1, e.g. at least 70%, at least 80%, at least 85%, at least 90% or at least 95% sequence identity, and has DNase activity.

In one aspect, the invention provides a DNase variant which compared to the polypeptide of SEQ ID NO: 1 comprises the substitution G149N and/or S164D, wherein the variant has at least 60% sequence identity to SEQ ID NO: 1, e.g. at least 70%, at least 80%, at least 85%, at least 90% or at least 95% sequence identity, and has DNase activity.

In one embodiment of this aspect, the DNase variant comprises the substitution G149N, wherein the variant has at least 60% sequence identity to SEQ ID NO: 1, e.g. at least 70%, at least 80%, at least 85%, at least 90% or at least 95% sequence identity, and has DNase activity. The variant may further comprise one or more additional substitutions, e.g. (i) one or more substitutions selected from the group consisting of N61D, T65I, T65V, S82R, K107Q, T127S, T127V, S164D and L181S, and/or (ii) one or more substitutions selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W. The DNase variant may further comprises one or more substitutions selected from the group consisting of I1T, Y13S, P22T, P25S, L27S, P39S, G42S, W57S, V59S, L76V, Y77T, R109Q, D116S, P144S, H147A, L167S, D175G and L181S.

In another embodiment of this aspect, the DNase variant comprises the substitution S164D, wherein the variant has at least 60% sequence identity to SEQ ID NO: 1, e.g. at least 70%, at least 80%, at least 85%, at least 90% or at least 95% sequence identity, and has DNase activity. The variant may further comprise one or more additional substitutions, e.g. (i) one or more substitutions selected from the group consisting of N61D, T65I, T65V, S82R, K107Q, T127S, T127V, G149N and L181S, and/or (ii) one or more substitutions selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W. The DNase variant may further comprises one or more substitutions selected from the group consisting of I1T, Y13S, P22T, P25S, L27S, P39S, G42S, W57S, V59S, L76V, Y77T, R109Q, D116S, P144S, H147A, L167S, D175G and L181S.

In a further aspect, the invention provides a polypeptide having DNase activity that comprises or consists of the amino acid sequence of SEQ ID NO: 1.

As indicated above, the DNase variant has at least 60% sequence identity to SEQ ID NO: 1, e.g. at least 70%, at least 75%, at least 80%, at least 85% or at least 90% sequence identity to SEQ ID NO: 1. The DNase variant may have at least 91%, at least 92%, at least 93%, at least 94%, at least 95%, at least 96%, at least 97%, at least 97%, at least 98% or at least 99% sequence identity to SEQ ID NO: 1.

The DNase variant may thus comprise more than two alterations, typically substitutions, compared to SEQ ID NO: 1, such as three, four, five, six, seven, eight, nine or ten alterations. In other embodiments, the DNase variant may comprise more than ten alterations, typically substitutions, compared to SEQ ID NO: 1, e.g. up to 15 or up to 20 alterations.

Preferably the DNase variant has an improved property which may be increased stability, e.g. improved detergent stability, improved in-wash stability and/or improved thermostability. Preferably, the DNase variants have improved stability detergent stability, in particular improved detergent storage stability. In addition, the variants preferably have substantially maintained or, more preferably, improved relative wash performance compared to a reference DNase, where the reference DNase can e.g. be the DNase of SEQ ID NO: 1 or SEQ ID NO: 27.

The variant may comprise additional mutations to those listed above. These additional modifications should preferably not significantly change the improved properties of the variant DNase and may e.g. be conservative substitutions.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for DNase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64.

### Parent DNase

Preferably the parent DNase is selected from any of the enzyme classes E.C. 3.1.11, E.C. 3.1.12, E.C. 3.1.15, E.C. 3.1.16, E.C. 3.1.21, E.C 3.1.22, E.C 3.1.23, E.C 3.1.24 and E.C.3.1.25.

Preferably, the DNase parent is obtained from a microorganism and the DNase is a microbial enzyme. The DNase is preferably of fungal or bacterial origin.

The DNase parent is preferably obtainable from *Bacillus, such as a Bacillus cibi, Bacillus sp-62451, Bacillus horikoshii, Bacillus sp-16840, Bacillus sp-62668, Bacillus sp-13395, Bacillus horneckiae, Bacillus sp-11238, Bacillus idriensis, Bacillus sp-62520, Bacillus algicola, Bacillus vietnamensis, Bacillus hwajinpoensis, Bacillus indicus, Bacillus marisflavi, Bacillus luciferensis, Bacillus sp. SA2-6.*

The parent DNase preferably belongs to the group of DNases comprised in the GYS-clade, which are DNases comprising the conservative motifs [D/M/L][S/T]GYSR[D/N] (SEQ ID NO: 25) or ASXNRSKG (SEQ ID NO: 26) and which share similar structural and functional properties, see e.g. WO 2017/060475. The DNases of the GYS-clade are preferably obtained from *Bacillus* genus.

In one embodiment the variant is a variant of a DNase parent of the GYS-clade having DNase activity, optionally wherein the parent comprises one or both motifs [D/M/L][S/T]GYSR[D/N] (SEQ ID NO: 25), ASXNRSKG (SEQ ID NO: 26), or wherein the parent is a variant of a DNase polypeptide comprising one or both of these motifs, e.g. a variant of a DNase polypeptide obtained from a naturally occurring microorganism, and wherein the polypeptide is selected from the group of polypeptides:
a) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 1,
b) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 2,
c) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 3,
d) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 4,
e) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 5,
f) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 6,
g) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 7,
h) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 8,
i) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 9,
j) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 10,
k) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 11,
l) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 12,
m) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 13,
n) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 14,
o) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 15,
p) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 16,
q) a polypeptide having at 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 17,
r) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 18,
s) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 19,
t) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 20,
u) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 21,
v) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 22,
w) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 23,
x) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 24, and
y) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 27.

Polypeptides having DNase activity and which comprise the GYS-clade motifs have shown particularly good deep cleaning properties, thus the compositions comprising such DNases are particularly effective in removing or reducing components of organic matter, such as biofilm components, from an item such as a textile or a hard surface.

In a preferred embodiment, the parent has a sequence identity to the polypeptide shown in SEQ ID NO: 1 of at least 60%, at least 65%, at least 70%, at least 75%, at least 80%, at least 85%, at least 90%, at least 95%, such as at least 96%, at least 97%, at least 98%, at least 99% or 100% sequence identity to the polypeptide shown in SEQ ID NO: 1, which has DNase activity.

In some aspects, the parent comprises or consists of the amino acid sequence of SEQ ID NO: 1.

### PREPARATION OF VARIANTS

The present invention also relates to a method for making a detergent composition comprising obtaining a DNase variant having at least one improved property compared to the parent DNase e.g. compared to the polypeptide shown in SEQ ID NO: 1; and mixing with a cleaning adjunct.

This aspect thus relates to a method for making a detergent composition comprising
(i) obtaining a DNase variant, comprising a) introducing into a parent DNase having at least 60% sequence identity to SEQ ID NO: 1 two or more substitutions selected from the group consisting of N61D, T65I, T65V, S82R, K107Q, T127S, T127V, G149N, S164D and L181S (wherein numbering is based on SEQ ID NO: 1), and
(ii) recovering the variant, wherein the variant has DNase activity; and
(iii) mixing with a cleaning adjunct.

It will be understood that the method of obtaining a DNase variant may further include introduction of any of the other substitutions or combinations of substitutions described above. For example, the method for obtaining a DNase variant may further comprise introduction of at least one substitution selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W. It may similarly comprise introduction of at least one substitution selected from the group consisting of I1T, Y13S, P22T, P25S, L27S, P39S, G42S, W57S, V59S, L76V, Y77T, R109Q, D116S, P144S, H147A, L167S and D175G.

In a preferred embodiment, the parent DNase has at least 80% sequence identity to the polypeptide of SEQ ID NO: 1, such as at least 85%, at least 90%, at least 95%, at least 96%, at least 97%, at least 98%, or at least 99% sequence identity to the polypeptide of SEQ ID NO: 1. In one embodiment, the parent DNase has the amino acid sequence of SEQ ID NO: 1.

In other embodiments, the variant may be based on a parent DNase selected from those set forth above, i.e. a polypeptide selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26 or SEQ ID NO: 27; or a polypeptide having at least 60%, at least 70%, at least 80%, at least 85%, at least 90%, at least 91%, at least 92%, at least 93%, at least 94%, at least 95%, at least 96%, at least 97%, at least 98% or at least 99% sequence identity to any of these.

The variant may be prepared by procedures that are well-known in the art such as those mentioned below.

Site-directed mutagenesis is a technique in which mutations are introduced at one or more defined sites in a polynucleotide encoding the parent. Site-directed mutagenesis can be accomplished *in vitro* by PCR involving the use of oligonucleotide primers containing the desired mutation. Site-directed mutagenesis can also be performed *in vitro* by cassette mutagenesis involving the cleavage by a restriction enzyme at a site in the plasmid comprising a polynucleotide encoding the parent and subsequent ligation of an oligonucleotide containing the mutation in the polynucleotide. Usually the restriction enzyme that digests the plasmid and the oligonucleotide is the same, permitting sticky ends of the plasmid and the insert to ligate to one another. See, *e.g.,* Scherer and Davis, 1979, Proc. Natl. Acad. Sci. USA 76: 4949-4955; and Barton et al., 1990, Nucleic Acids Res. 18: 7349-4966.

Site-directed mutagenesis can also be accomplished *in vivo* by methods known in the art. See, *e.g.,* U.S. Patent Application Publication No. 2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16.

Synthetic gene construction entails *in vitro* synthesis of a designed polynucleotide molecule to encode a polypeptide of interest. Gene synthesis can be performed utilizing several techniques, such as the multiplex microchip-based technology described by Tian et al. (2004, Nature 432: 1050-1054) and similar technologies wherein oligonucleotides are synthesized and assembled upon photo-programmable microfluidic chips.

Single or multiple amino acid substitutions, deletions, and/or insertions can be made and tested using known methods of mutagenesis, recombination and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al., 1991, Biochemistry 30: 10832-10837; US 5,223,409; WO 92/06204) and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized *de novo,* while other regions may be amplified using site-specific mutagenic primers, while yet other regions may be subjected to error-prone PCR or non-error prone PCR amplification. Polynucleotide subsequences may then be shuffled.

### NUCLEIC ACID CONSTRUCTS

Dislcosed herein are nucleic acid constructs comprising a polynucleotide encoding the DNase variants of the present invention operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the Bacillus amyloliquefaciens alpha-amylase gene (amyQ), Bacillus licheniformis alpha-amylase gene (amyL), Bacillus licheniformis penicillinase gene (penP), Bacillus stearothermophilus maltogenic amylase gene (amyM), Bacillus subtilis levansucrase gene (sacB), Bacillus subtilis xylA and xylB genes, Bacillus thuringiensis cryIIIA gene (Agaisse and Lereclus, 1994, Molecular Microbiology 13: 97-107), E. coli lac operon, E. coli trc promoter (Egon et al., 1988, Gene 69: 301-315), Streptomyces coelicolor agarase gene (dagA), and prokaryotic beta-lactamase gene (Villa-Kamaroff et al., 1978, Proc. Natl. Acad. Sci. USA 75: 3727-3731), as well as the tac promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook et al., 1989, supra. Examples of tandem promoters are disclosed in WO 99/43835.

Examples of suitable promoters for directing transcription of the nucleic acid constructs of the present invention in a filamentous fungal host cell are promoters obtained from the genes for *Bacillus nidulans* acetamidase, *Bacillus niger* neutral alpha-amylase, *Bacillus niger* acid stable alpha-amylase, *Bacillus niger* or *Bacillus awamori* glucoamylase (glaA), *Bacillus cibi* TAKA amylase, *Bacillus cibi* alkaline protease, *Bacillus cibi* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2 tpi promoter (a modified promoter from a *Bacillus* neutral alpha-amylase gene in which the untranslated leader has been replaced by an untranslated leader from a *Bacillus triose* phosphate isomerase gene; nonlimiting examples include modified promoters from a *Bacillus niger* neutral alpha-amylase gene in which the untranslated leader has been replaced by an untranslated leader from a *Bacillus nidulans* or *Bacillus cibi* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO 1), *Saccharomyces cerevisiae* galactokinase (GAL1), *Saccharomyces cerevisiae* alcohol dehydrogenase/glyceraldehyde-3 phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3 phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3' terminus of the polynucleotide encoding the polypeptide. Any terminator that is functional in the host cell may be used in the present invention.

Preferred terminators for bacterial host cells are obtained from the genes for *Bacillus clausii* alkaline protease (aprH), *Bacillus licheniformis* alpha-amylase (amyL), and *Escherichia coli* ribosomal RNA (rrnB). Other terminators may be obtained from the genes for *Bacillus nidulans* acetamidase, *Bacillus nidulans* anthranilate synthase, *Bacillus niger* glucoamylase, *Bacillus niger* alpha-glucosidase or *Bacillus cibi* TAKA amylase.

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I*, Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3 phosphate dehydrogenase. Other useful terminators for yeast host cells are described by Romanos et al., 1992, supra.

The control sequence may also be an mRNA stabilizer region downstream of a promoter and upstream of the coding sequence of a gene which increases expression of the gene. Examples of suitable mRNA stabilizer regions are obtained from a *Bacillus thuringiensis* cryIIIA gene (WO 94/25612) and a Bacillus subtilis SP82 gene (Hue et al., 1995, Journal of Bacteriology 177: 3465-3471).

The control sequence may also be a leader, a nontranslated region of an mRNA that is important for translation by the host cell. The leader is operably linked to the 5' terminus of the polynucleotide encoding the polypeptide. Any leader that is functional in the host cell may be used.

Preferred leaders for filamentous fungal host cells are obtained from the genes for *Bacillus cibi* TAKA amylase and *Bacillus nidulans* triose phosphate isomerase.

Suitable leaders for yeast host cells are obtained from the genes for *Saccharomyces cerevisiae* enolase (ENO 1), *Saccharomyces cerevisiae* 3 phosphoglycerate kinase, *Saccharomyces cerevisiae* alpha-factor, and *Saccharomyces cerevisiae* alcohol dehydrogenase/glyceraldehyde-3 phosphate dehydrogenase (ADH2/GAP).

The control sequence may also be a polyadenylation sequence, a sequence operably linked to the 3' terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Bacillus nidulans* anthranilate synthase, *Bacillus niger* glucoamylase, *Bacillus niger* alpha-glucosidase Bacillus cibi TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N terminus of a polypeptide and directs the polypeptide into the cell's secretory pathway. The 5' end of the coding sequence of the polynucleotide may inherently contain a signal peptide coding sequence naturally linked in translation reading frame with the segment of the coding sequence that encodes the polypeptide. Alternatively, the 5' end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (nprT, nprS, nprM), and *Bacillus subtilis* prsA. Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease *(aprE), Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N terminus of a polypeptide and the signal peptide sequence is positioned next to the N terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

### EXPRESSION VECTORS

Disclosed herein are recombinant expression vectors comprising a polynucleotide encoding the DNase variants of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression. In a further aspect, polynucleotide sequence codons have been modified by nucleotide substitutions to correspond to the codon usage of the host organism intended for production of the polypeptide of the present invention. The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosylaminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is a *hph-tk* dual selectable marker system.

The vector preferably contains an element(s) that permits integration of the vector into the host cell's genome or autonomous replication of the vector in the cell independent of the genome. For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo.

Examples of bacterial origins of replication are the origins of replication of plasmids pBR322, pUC19, pACYC177, and pACYC184 permitting replication in E. coli, and pUB110, pE194, pTA1060, and pAMβ1 permitting replication in Bacillus. Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

### HOST CELLS

Disclosed herein are recombinant host cells, comprising a polynucleotide of the present invention operably linked to one or more control sequences that direct the production of a polypeptide of the present invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, e.g., a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi subsp. Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a Bacillus cell may be effected by protoplast transformation (see, e.g., Chang and Cohen, 1979, Mol. Gen. Genet. 168: 111-115), competent cell transformation (see, e.g., Young and Spizizen, 1961, J. Bacteriol. 81: 823-829, or Dubnau and Davidoff-Abelson, 1971, J. Mol. Biol. 56: 209-221), electroporation (see, e.g., Shigekawa and Dower, 1988, Biotechniques 6: 742-751), or conjugation (see, e.g., Koehler and Thorne, 1987, J. Bacteriol. 169: 5271-5278). The introduction of DNA into an E. coli cell may be effected by protoplast transformation (see, e.g., Hanahan, 1983, J. Mol. Biol. 166: 557-580) or electroporation (see, e.g., Dower et al., 1988, Nucleic Acids Res. 16: 6127-6145). The introduction of DNA into a Streptomyces cell may be effected by protoplast transformation, electroporation (see, e.g., Gong et al., 2004, Folia Microbiol. (Praha) 49: 399-405), conjugation (see, e.g., Mazodier et al., 1989, J. Bacteriol. 171: 3583-3585), or transduction (see, e.g., Burke et al., 2001, Proc. Natl. Acad. Sci. USA 98: 6289-6294). The introduction of DNA into a Pseudomonas cell may be effected by electroporation (see, e.g., Choi et al., 2006, J. Microbiol. Methods 64: 391-397) or conjugation (see, e.g., Pinedo and Smets, 2005, Appl. Environ. Microbiol. 71: 51-57). The introduction of DNA into a Streptococcus cell may be effected by natural competence (see, e.g., Perry and Kuramitsu, 1981, Infect. Immun. 32: 1295-1297), protoplast transformation (see, e.g., Catt and Jollick, 1991, Microbios 68: 189-207), electroporation (see, e.g., Buckley et al., 1999, Appl. Environ. Microbiol. 65: 3800-3804), or conjugation (see, e.g., Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein cludes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (*Endomycetales*)*,* basidiosporogenous yeast, and yeast belonging to the Fungi Imperfecti (*Blastomycetes*)*.* Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth et al., 1995, supra). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Bacillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

### METHODS OF PRODUCTION

The present invention also relates to a method of producing a detergent composition comprising a DNase variant, comprising (a) cultivating a recombinant host cell as described above under conditions conducive for production of the DNase variant; and optionally, (b) recovering the DNase variant, and mixing the DNase variant with a cleaning adjunct.

### CLEANING ADJUNCT

The composition comprises a cleaning adjunct. Typically the cleaning adjunct will be present in the composition in an amount from 1 to 98.9 wt%, more typically from 5 to 80 wt% cleaning adjunct. Suitable cleaning adjuncts comprise: surfactants, builders, bleach ingredients, colorants, chelating agents, dye transfer agents, deposition aids, dispersants, additional enzymes, and enzyme stabilizers, catalytic materials, optical brighteners, photoactivators, fluorescers, fabric hueing agents (shading dyes), fabric conditioners, preformed peracids, polymeric dispersing agents, clay soil removal/anti-redeposition agents, filler salts, hydrotropes, brighteners, suds suppressors, structure elasticizing agents, fabric softeners, preservatives, anti-oxidants, anti-shrinkage agents, germicides, fungicides, anti-tarnish, anti-corrosion agents, alkalinity sources, solubilizing agents, carriers, processing aids, pigments, dyes, perfumes and pH control agents, encapsulates, polymers and mixtures thereof. For example, these may include: bleach ingredients such as bleach activators, bleach boosters such as imine bleach boosters, bleach catalysts, hydrogen peroxide, sources of hydrogen peroxide such as percarbonate and/or perborate, especially percarbonate coated with material such as carbonate and/or sulphate salt, silicate salt, borosilicate, and any mixture thereof, pre-formed peracid, including pre-formed peracid in encapsulated form, transition metal catalysts; suds suppressors or suppressor systems such as silicone based suds suppressors and/or fatty acid based suds suppressors; fabric-softeners such as clay, silicone and/or quaternary ammonium compounds; flocculants such as polyethylene oxide; dye transfer inhibitors such as polyvinylpyrrolidone, poly 4-vinylpyridine N-oxide and/or copolymer of vinylpyrrolidone and vinylimidazole; fabric integrity components such as oligomers produced by the condensation of imidazole and epichlorhydrin; soil dispersants and soil anti-redeposition aids such as alkoxylated polyamines and ethoxylated ethyleneimine polymers; anti-redeposition components such as polyesters; carboxylate polymers such as maleic acid polymers or co-polymers of maleic and acrylic acid; perfumes such as perfume microcapsules, starch encapsulated accords, perfume spray-on; soap rings; aesthetic particles; aesthetic dyes; fillers such as sodium sulphate and/or citrus fibres, although it may be preferred for the composition to be substantially free of fillers; silicate salt such as sodium silicate, including 1.6R and 2.0R sodium silicate, or sodium metasilicate; co-polyesters of di-carboxylic acids and diols; cellulosic polymers such as methyl cellulose, carboxymethyl cellulose, hydroxyethoxycellulose, or other alkyl or alkylalkoxy cellulose; solvents such as 1,2 propanediol, monoethanolamine; diethylene glycol, ethanol, and any mixture thereof; hydrotropes such as sodium cumene sulphonate, sodium xylene sulphonate, sodium toluene sulphonate, and any mixtures; organic acids and salts thereof, such as citric acid/citrate salts; and any combination thereof.

Preferably the composition comprises a surfactant. Preferably the composition comprises an anionic surfactant. Preferred anionic surfactants are sulfonate and sulfate surfactants, preferably alkylbenzene sulphonates and/or (optionally alkoxylated) alkyl sulfates. Particularly preferred anionic surfactant comprises linear alkylbenzene sulfonates (LAS). Preferred alkyl sulfates comprise alkyl ether sulfates, especially C-9-15 alcohol ether sulfates, especially those having an average degree of ethoxylation from 0.5 to 7, preferably from 1 to 5, C8-C16 ester sulfates and C10-C14 ester sulfates, such as mono dodecyl ester sulfates. In a preferred composition the anionic surfactant comprises alkyl benzene sulphonate and optionally in addition, optionally ethoxylated alkyl sulfate, preferably having a degree of ethoxylation from 0 to 7, more preferably from 0.5 to 3. Isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfosuccinic acid or salt of fatty acids (soap), and combinations thereof are also suitable anionic surfactants.

The anionic surfactant is preferably added to the detergent composition in the form of a salt. Preferred cations are alkali metal ions, such as sodium and potassium. However, the salt form of the anionic surfactant may be formed in situ by neutralization of the acid form of the surfactant with alkali such as sodium hydroxide or an amine, such as mono-, di-, or tri-ethanolamine. The composition preferably comprises from 1 to 60 weight % or from 1 to 50 wt% or 2 or 5 to 40 wt% of the composition, anionic surfactant. The surfactant preferably comprises a surfactant system comprising an anionic surfactant and in addition, one or more additional surfactants, which may be non-ionic including semi-polar and/or cationic and/or zwitterionic and/or ampholytic and/or amphoteric and/or semi-polar nonionic and/or mixtures thereof.

The composition of the invention preferably comprises a cleaning adjunct comprising a surfactant wherein the surfactant comprises an anionic and a nonionic surfactant, preferably having a weight ratio of anionic to nonionic of from 30:1 to 1:2, preferably from 20:1 to 2:3 or to 1:1.

Suitable nonionic surfactants include alcohol ethoxylates (AE), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Alcohol ethoxylates are particularly preferred, preferably having a C9-18 or preferably a C12-15 alkyl chain and preferably having an average degree of ethoxylation from 3 to 9, more preferably from 3 to 7. Commercially available nonionic surfactants cleaning include Plurafac ^{™}, Lutensol^{™} and PluroniC^{™} from BASF, Dehypon^{™} series from Cognis and Genapol^{™} series from Clariant.

The detergent composition preferably comprises from 0.5wt% to about 40wt% of a non-ionic surfactant, preferably 1 to 30 wt% of the composition non-ionic surfactant.

The composition may be such that the cleaning adjunct comprises one or more selected from the group consisting of (i) perfume microcapsule; (ii) fabric hueing agent; (iii) protease; (iv) amphiphilic cleaning polymer; (v) lipase, or (vi) mixtures thereof.

The detergent composition preferably comprises one or more additional enzymes, preferably selected from the group consisting of aminopeptidase, amylase, arabinase, alginate lyase, carbohydrase, carboxypeptidase, catalase, cellulase, chitinase, cutinase, cyclodextrin glycosyltransferase, deoxyribonuclease, esterase, galactanase, alpha-galactosidase, beta-galactosidase, glucoamylase, alpha-glucosidase, beta-glucosidase, haloperoxidase, hexosaminidase, invertase, laccase, lipase, mannanase, mannosidase, oxidase, pectinolytic enzyme, peptidoglutaminase, peroxidase, phytase, polyphenoloxidase, proteolytic enzyme, ribonuclease, transglutaminase, xylanase, xanthan lyase, xanthanase, endo -β-1,3-glucanase and mixtures thereof. Preferably the cleaning composition comprises additional enzyme selected from amylase, additional nuclease such as further DNase and/or RNase and mixtures thereof, hexosaminidase, mannanase, xanthan lyase, xanthanase, amylase and mixtures thereof.

Preferably the composition comprises additional enzymes selected from xanthan lyase, xanthanase, mannanase, hexosaminidase and mixtures thereof. Mannanase is particularly preferred.

The additional enzyme(s) may be produced, for example, by a microorganism belonging to the genus *Aspergillus, e.g., Aspergillus aculeatus, Aspergillus awamori, Aspergillus foetidus, Aspergillus fumigatus, Aspergillus japonicus, Aspergillus nidulans, Aspergillus niger,* or *Aspergillus oryzae; Fusarium, e.g., Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sulphureum, Fusarium toruloseum, Fusarium trichothecioides,* or *Fusarium venenatum; Humicola, e.g., Humicola insolens* or *Humicola lanuginosa;* or *Trichoderma, e.g., Trichoderma harzianum, Trichoderma koningii, Trichoderma longibrachiatum, Trichoderma reesei,* or *Trichoderma viride.*

Preferably the composition comprises a protease or mixture of more than one protease, a lipase or mixture of more than one lipase, a peroxidase or mixture of more than one peroxidase, one or more amylolytic enzymes, e.g., an alpha-amylase, glucoamylase, maltogenic amylase, and/or a cellulase or mixture thereof.

In general, the properties of the chosen enzyme(s) should be compatible with the selected detergent, (i.e., pH-optimum, compatibility with other enzymatic and non-enzymatic ingredients, etc.), and the enzyme(s) should be present in effective amounts. Preferably, the product of the invention comprises at least 0.01 mg, preferably from about 0.05 to about 10, more preferably from about 0.1 to about 6, especially from about 0.2 to about 5 mg of active further enzyme/ g of composition.

Proteases: The composition of the invention can comprise one or more proteases. A mixture of two or more proteases can contribute to an enhanced cleaning across a broader temperature, cycle duration, and/or substrate range, and/or provide superior shine benefits, especially when used in conjunction with an anti-redeposition agent and/or a sulfonated polymer.

Suitable proteases for use in combination with the variant proteases of the invention include metalloproteases and serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
subtilisins (EC 3.4.21.62), especially those derived from *Bacillus,* such as *Bacillus* sp., *B. lentus, B. alkalophilus, B. subtilis, B. amyloliquefaciens, B. gibsonii, B. akibaii, Bacillus Clausii* and *B. clarkii* described in WO2004067737, WO2015091989, WO2015091990, WO2015024739, WO2015143360, US 6,312,936 B1, US 5,679,630, US 4,760,025, DE102006022216A1, WO2015089447, WO2015089441, WO2016066756, WO2016066757, WO2016069557, WO2016069563, WO2016069569, WO2016174234, WO2017/089093, WO2020/156419. Specifically, mutations S9R, A15T, V66A, A188P, V199I, N212D, Q239R, N255D, X9E, X200L, X256E , X9R, X19L, X60D (Savinase numbering system);
subtilisins from *B. pumilus* such as the ones described in DE102006022224A1, WO2020/221578, WO2020/221579, WO2020/221580, including variants comprising amino acid substitutions in at least one or more of the positions selected from 9, 130, 133, 144, 252, 271 (BPN' numbering system);
trypsin-type or chymotrypsin-type proteases, such as trypsin (e.g., of porcine or bovine origin), including the *Fusarium* protease described in WO 89/06270 and the chymotrypsin proteases derived from *Cellumonas* described in WO 05/052161 and WO 05/052146;
metalloproteases, especially those derived from *Bacillus amyloliquefaciens* decribed in WO07/044993A2; from *Bacillus, Brevibacillus, Thermoactinomyces, Geobacillus, Paenibacillus, Lysinibacillus* or *Streptomyces* spp. Described in WO2014194032, WO2014194054 and WO2014194117; from Kribella alluminosa described in WO2015193488; and from *Streptomyces* and *Lysobacter* described in WO2016075078;
protease having at least 90% identity to the subtilase from *Bacillus* sp. TY145, NCIMB 40339, described in WO92/17577 (Novozymes A/S), including the variants of this *Bacillus* sp TY145 subtilase described in WO2015024739, and WO2016066757.

Especially preferred additional proteases for the composition are polypeptides demonstrating at least 90%, preferably at least 95%, more preferably at least 98%, even more preferably at least 99% and especially 100% identity with the wild-type enzyme from Bacillus lentus, comprising mutations in one or more, preferably two or more and more preferably three or more of the following positions, using the BPN' numbering system and amino acid abbreviations as illustrated in WO00/37627, which is incorporated herein by reference: S9R, A15T, V68A, N76D, N87S, S99D, S99SD, S99A, S101G, S101M, S103A, V104N/I, G118V, G118R, S128L, P129Q, S130A, Y167A, R170S, A194P, V205I, Q206L/D/E, Y209W, M222S, Q245R and/or M222S.

Most preferably the additional protease is selected from the group of proteases comprising the below mutations (BPN' numbering system) versus either the PB92 wild-type (SEQ ID NO:2 in WO 08/010925) or the subtilisin 309 wild-type (sequence as per PB92 backbone, except comprising a natural variation of N87S).
(i) G118V + S128L + P129Q + S130A
(ii) S101M + G118V + S128L + P129Q + S130A
(iii) N76D + N87R + G118R + S128L + P129Q + S130A + S188D + N248R
(iv) N76D + N87R + G118R + S128L + P129Q + S130A + S188D + V244R
(v) N76D + N87R + G118R + S128L + P129Q + S130A
(vi) V68A + N87S + S101G + V104N
(vii) S99AD
(viii) S9R+A15T+V68A+N218D+Q245R

Suitable commercially available additional protease enzymes include those sold under the trade names

Alcalase^{®}, Savinase^{®}, Primase^{®}, Durazym^{®}, Polarzyme^{®}, Kannase^{®}, Liquanase^{®}, Liquanase Ultra^{®}, Savinase Ultra^{®}, Liquanase^{®} Evity^{®}, Savinase^{®} Evity^{®}, Ovozyme^{®}, Neutrase^{®}, Everlase^{®}, Coronase^{®}, Blaze^{®}, Blaze Ultra^{®}, Blaze^{®} Evity^{®}, Blaze^{®} Exceed, Blaze^{®} Pro, Esperase^{®}, Progress^{®} Uno, Progress^{®} Excel, Progress^{®} Key, Ronozyme^{®}, Vinzon^{®} and Het Ultra^{®} by Novozymes A/S (Denmark);
those sold under the tradename Maxatase^{®}, Maxacal^{®}, Maxapem^{®}, Properase^{®}, Purafect^{®}, Purafect Prime^{®}, Purafect Ox^{®}, FN3^{®}, FN4^{®}, Excellase^{®}, Ultimase^{®} and Purafect OXP^{®} by Dupont;
those sold under the tradename Opticlean^{®} and Optimase^{®} by Solvay Enzymes;
those available from Henkel/Kemira, namely BLAP (sequence shown in Figure29 of US 5,352,604 with the following mutations S99D + S101R + S103A + V104I + G159S, hereinafter referred to as BLAP), BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D); and can optionally comprise a further mutation 101E or 101D;
KAP (*Bacillus alkalophilus* subtilisin with mutations A230V + S256G + S259N) from Kao; and Lavergy^{®}, Lavergy^{®} Pro, Lavergy^{®} C Bright from BASF.

Especially preferred for use herein in combination with the variant protease of the invention are commercial proteases selected from the group consisting of Properase^{®}, Blaze^{®}, Ultimase^{®}, Everlase^{®}, Savinase^{®}, Excellase^{®}, Blaze Ultra^{®}, BLAP and BLAP variants.

Preferred levels of protease in the product of the invention include from about 0.05 to about 10, more preferably from about 0.5 to about 7 and especially from about 1 to about 6 mg of active protease/g of composition.

Lipases: The composition preferably comprises a lipase. The presence of oils and/or grease can further increase the resiliency of stains comprising mannans and other polysaccharides. As such, the presence of lipase in the enzyme package can further improve the removal of such stains. Suitable lipases include those of bacterial or fungal or synthetic origin. Chemically modified or protein engineered mutants are included. Examples of useful lipases include lipases from *Humicola* (synonym *Thermomyces)*, e.g., from *H. lanuginosa* (*T. lanuginosus)* or from *H. insolens,* a *Pseudomonas lipase,* e.g., from *P. alcaligenes* or *P. pseudoalcaligenes, P. cepacia P. stutzeri, P. fluorescens, Pseudomonas* sp. strain SD 705, *P. wisconsinensis,* a *Bacillus* lipase, e.g., from *B. subtilis* (Dartois et al. (1993), Biochemica et Biophysica Acta, 1131, 253-360), *B. stearothermophilus* or *B. pumilus .*

The lipase may be a "first cycle lipase" such as those described in U.S. Patent 6,939,702 B1 and US PA 2009/0217464. In one aspect, the lipase is a first-wash lipase, preferably a variant of the wild-type lipase from *Thermomyces lanuginosus* comprising T231R and N233R mutations. The wild-type sequence is the 269 amino acids (amino acids 23 - 291) of the Swissprot accession number Swiss-Prot O59952 (derived from *Thermomyces lanuginosus (Humicola lanuginosa)).* Preferred lipases include those sold under the tradenames Lipex^{®}, Lipolex^{®} and Lipoclean^{®}.

Other suitable lipases include: Liprl 139, e.g. as described in WO2013/171241; TfuLip2, e.g. as described in WO2011/084412 and WO2013/033318; *Pseudomonas stutzeri* lipase, e.g. as described in WO2018228880; *Microbulbifer thermotolerans* lipase, e.g. as described in WO2018228881; *Sulfobacillus acidocaldarius* lipase, e.g. as described in EP3299457; LIP062 lipase e.g. as described in WO2018209026; PinLip lipase e.g. as described in WO2017036901 and *Absidia* sp. lipase e.g. as described in WO2017005798.

A suitable lipase is a variant of SEQ ID NO:5 comprising:
(a) substitution T231R
   and
(b) substitution N233R or N233C
   and
(c) at least three further substitutions selected from E1C, D27R, N33Q, G38A, F51V, G91Q, D96E, K98L, K98I, D111A, G163K, H198S, E210Q, Y220F, D254S, I255A, and P256T;
where the positions correspond to the positions of SEQ ID NO:5 and wherein the lipase variant has at least 90% but less than 100% sequence identity to the polypeptide having the amino acid sequence of SEQ ID NO: 5 and wherein the variant has lipase activity.

One preferred lipase is a variant of SEQ ID NO: 5 comprising the following substitutions: T231R, N233R, D27R, G38A, D96E, D111A, G163K, D254S and P256T

One preferred lipase is a variant of SEQ ID NO: 5 comprising the following substitutions: T231R, N233R, N33Q, G91Q, E210Q, I255A.

Suitable lipases are commercially available from Novozymes, for example as Lipex Evity 100L, Lipex Evity 200L (both liquid raw materials) and Lipex Evity 105T (a granulate). These lipases have different structures to the products Lipex 100L, Lipex 100T and Lipex Evity 100T which are outside the scope of the invention.

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum.* disclosed in US 4,435,307 , US 5,648,263 , US 5,691,178, US 5,776,757 and US 5,691,178 .

In one aspect, preferred enzymes include microbial-derived endoglucanases exhibiting endo-beta-1,4-glucanase activity (E.C. 3.2.1.4), prefrebaly selected from the group comprising:
(a) a bacterial polypeptide endogenous to a member of the genus Bacillus which has a sequence of at least 90%, 94%, 97% and even 99% identity to the amino acid sequence SEQ ID NO:2 in US 7,141,403B2, preferred substitutions compriseone or more positions corresponding to positions 292, 274, 266, 265, 255, 246, 237, 224 and 221 of the mature polypeptide of SEQ ID NO: 2, and t he variant has cellulase activity.;
(b) a glycosyl hydrolase having enzymatic activity towards both xyloglucan and amorphous cellulose substrates, wherein the glycosyl hydrolase is selected from GH families 5, 7, 12, 16, 44 or 74;
(c) a glycosyl hydrolase having a sequence of at least 90%, 94%, 97% and even 99% identity to the amino acid sequence SEQ ID NO:3 in WO09/148983;
(d) Variants exhibiting at least 70% identity with SEQ ID NO: 5 in WO2017106676. Preferred substitutions comprise one or more positions corresponding to positions 4, 20, 23, 29, 32, 36, 44, 51, 77, 80, 87, 90, 97, 98, 99, 102, 112, 116, 135, 136, 142, 153, 154, 157, 161, 163, 192, 194, 204, 208, 210, 212, 216, 217, 221, 222, 225, 227, and 232;
(e) and mixtures thereof.

Suitable endoglucanases are sold under the tradenames Celluclean^{®} and Whitezyme^{®} (Novozymes A/S, Bagsvaerd, Denmark). Examples include Celluclean^{®} 5000L, Celluclean^{®} Classic 400L, Celluclean^{®} Classic 700T, Celluclean^{®} 4500T, Whitezyme^{®} 1.5T, Whitezyme^{®} 2.0L.

Other commercially available cellulases include Celluzyme^{®}, Carezyme^{®}, Carezyme^{®} Premium (Novozymes A/S), Clazinase^{®}, Puradax HA^{®}, Revitalenz^{®} 1000, Revitalenz^{®} 2000 (Genencor International Inc.), KAC-500(B)^{®} (Kao Corporation), Biotouch^{®} FCL, Biotouch^{®} DCL, Biotouch^{®} DCC, Biotouch^{®} NCD, Biotouch^{®} FCC, Biotouch^{®} FLX1 (AB Enzymes)

Suitable glucanases include endo-β-1,3-glucanases, preferably from E.C. class 3.2.1.39, preferably obtained from *Paenibacillus sp, Zobellia galactanivorans, Thermotoga petrophila* or *Trichoderma sp* micro-organism, preferably *Paenibacillus sp* or *Zobellia galactanivorans,* most preferably *Paenibacillus sp.*

Amylases: Preferably the composition of the invention comprises an amylase. Suitable alpha-amylases include those of bacterial or fungal origin. Chemically or genetically modified mutants (variants) are included. A preferred alkaline alpha-amylase is derived from a strain of Bacillus, such as *Bacillus licheniformis, Bacillus amyloliquefaciens, Bacillus stearothermophilus, Bacillus subtilis,* or other *Bacillus sp.,* such as Bacillus sp. NCBI 12289, NCBI 12512, NCBI 12513, DSM 9375 (USP 7,153,818) DSM 12368, DSMZ no. 12649, KSM AP1378 (WO 97/00324), KSM K36 or KSM K38 (EP 1,022,334). Preferred amylases include:
(a) variants described in USP 5,856,164 and WO99/23211, WO 96/23873, WO00/60060, WO06/002643 and WO2017/192657, especially the variants with one or more substitutions in the following positions versus the AA560 enzyme listed as SEQ ID No. 12 in WO 06/002643: 26, 30, 33, 82, 37, 106, 118, 128, 133, 149, 150, 160, 178, 182, 186, 193, 202, 214, 231, 246, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, preferably that also contain the deletions of D183^{∗} and G184^{∗}.
(b) variants exhibiting at least 85%, preferably 90% identity with SEQ ID No. 4 in WO06/002643, the wild-type enzyme from Bacillus SP722, especially variants with deletions in the 183 and 184 positions and variants described in WO 00/60060, WO2011/100410 and WO2013/003659, particularly those with one or more substitutions at the following positions versus SEQ ID No. 4 in WO06/002643 which are incorporated herein by reference: 51, 52, 54, 109, 304, 140, 189, 134, 195, 206, 243, 260, 262, 284, 347, 439, 469, 476 and 477.
(c) variants exhibiting at least 90% identity with the wild-type enzyme from Bacillus sp.707 (SEQ ID NO:7 in US 6,093,562), especially those comprising one or more of the following mutations M202, M208, S255, R172, and/or M261. Preferably said amylase comprises one or more of M202L, M202V, M202S, M202T, M202I, M202Q, M202W, S255N and/or R172Q. Particularly preferred are those comprising the M202L or M202T mutations. Additional relevant mutations/deletions based on SP707 backbone include W48, A51, V103, V104, A113, R118, N125, V131, T132, E134, T136, E138, R142, S154, V165, R182, G182, H183, E190, D192, T193, 1206, M208, D209, E212, V213, V214, N214, L217, R218, N219, V222, T225, T227, G229, 1235, K242, Y243, S244, F245, T246, 1250, S255, A256, H286, V291, T316, V317, V318, N417, T418, A419, H420, P421, 1428, M429, F440, R443, N444, K445, Q448, S451, A465, N470, S472.
(d) variants described in WO 09/149130, preferably those exhibiting at least 90% identity with SEQ ID NO: 1 or SEQ ID NO:2 in WO 09/149130, the wild-type enzyme from *Geobacillus Stearophermophilus* or a truncated version thereof.
(e) variants described in WO10/115021, especially those exhibiting at least 75%, or at least 85% or at least 90% or at least 95% with SEQ ID NO:2 in WO10/115021, the alpha-amylase derived from *Bacillus sp.* TS-23.
(f) variants exhibiting at least 89% identity with SEQ ID NO:1 in WO2016091688, especially those comprising deletions at positions H183+G184 and additionally one or more mutations at positions 405, 421, 422 and/or 428.
(g) variants described in WO2014099523, especially those exhibiting at least 60% amino acid sequence identity with the "PcuAmyl α-amylase" from *Paenibacillus curdlanolyticus* YK9 (SEQ ID NO:3 in WO2014099523).
(h) variants described in WO2014099523, especially those exhibiting at least 60% amino acid sequence identity with the "CspAmy2 amylase" from *Cytophaga sp.* (SEQ ID NO:1 & 6 in WO2014164777. Especially those comprising one of more of the following deletions and/or mutations based on SEQ ID NO:1 in WO2014164777: R178^{∗}, G179^{∗}, T38N, N88H, N126Y, T129I, N134M, F153W, L171R, T180D, E187P, I203Y, G476K, G477E, Y303D.
(i) variants exhibiting at least 85% identity with AmyE from *Bacillus subtilis* (SEQ ID NO:1 in WO2009149271).
(j) variants exhibiting at least 90% identity with the wild-type amylase from Bacillus sp. KSM-K38 with accession number AB051102.
(k) variants described in WO2016180748, especially those exhibiting at least 80% identity with the mature amino acid sequence of AAI10 from *Bacillus sp* in SEQ ID NO: 7 in WO2016180748; those exhibiting at least 80% identity with the mature amino acid sequence of *Alicyclobacillus sp.* amylase in SEQ ID NO: 8 in WO2016180748, and those exhibiting at least 80% identity with the mature amino acid sequence of SEQ ID NO: 13 in WO2016180748, especially those comprising one or more of the following mutations H^{∗}, N54S, V56T, K72R, G109A, F113Q, R116Q, W167F, Q172G, A174S, G184T, N195F, V206L, K391A, P473R, G476K.
(l) variants described in WO2018060216, especially those exhibiting at least 70% identity with the mature amino acid sequence of SEQ ID NO: 4 in WO2018060216, the fusion molecule of *Bacillus amyloliquefaciens* and *Bacillus licheniformis.* Especially those comprising one or more substitutions at positions H1, N54, V56, K72, G109, F113, R116, T134, W140, W159, W167, Q169, Q172, L173, A174, R181, G182, D183, G184, W189, E194, N195, V206, G255, N260, F262, A265, W284, F289, S304, G305, W347, K391, Q395, W439, W469, R444, F473, G476, and G477.

Preferred amylases are engineered enzymes, wherein one or more of the amino acids prone to bleach oxidation have been substituted by an amino acid less prone to oxidation. In particular it is preferred that methionine residues are substituted with any other amino acid. In particular it is preferred that the methionine most prone to oxidation is substituted. Preferably the methionine in a position equivalent to 202 in SEQ ID NO:11 is substituted. Preferably, the methionine at this position is substituted with threonine or leucine, preferably leucine.

Suitable commercially available alpha-amylases include DURAMYL^{®}, LIQUEZYME^{®}, TERMAMYL^{®}, TERMAMYL ULTRA^{®}, NATALASE^{®}, SUPRAMYL^{®}, STAINZYME^{®}, STAINZYME PLUS^{®}, FUNGAMYL^{®}, ATLANTIC^{®}, ACHIEVE ALPHA^{®}, AMPLIFY^{®} PRIME, INTENTSA^{®} and BAN^{®} (Novozymes A/S, Bagsvaerd, Denmark), KEMZYM^{®} AT 9000 Biozym Biotech Trading GmbH Wehlistrasse 27b A-1200 Wien Austria, RAPIDASE^{®} , PURASTAR^{®}, ENZYSIZE^{®}, OPTISIZE HT PLUS^{®}, POWERASE^{®}, PREFERENZ S^{®} series (including PREFERENZ S1000^{®} and PREFERENZ S2000^{®} and PURASTAR OXAM^{®} (DuPont., Palo Alto, California) and KAM^{®} (Kao, 14-10 Nihonbashi Kayabacho, 1-chome, Chuoku Tokyo 103-8210, Japan).

Preferably, the composition comprises at least 0.01 mg, preferably from about 0.05 to about 10, more preferably from about 0.1 to about 6, especially from about 0.2 to about 5 mg of active amylase/ g of composition.

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g., from *C*. *cinereus,* and variants thereof as those described in WO 93/24618, WO 95/10602, and WO 98/15257.

Commercially available peroxidases include GUARDZYME^{®} (Novozymes A/S).

Pectate lyase: Suitable pectate lyases include those sold under the tradenames Pectawash^{®}, Pectaway^{®}, X-Pect^{®}, (all Novozymes A/S, Bagsvaerd, Denmark) Preferenz^{®} F1000 (DuPont Industrial Biosciences).

Mannanases. The composition preferably comprises one of more mannanase enzymes. As used herein, the term "mannanase" or "galactomannanase" denotes a mannanase enzyme defined according to that known in the art as mannan endo-1,4-beta-mannosidase and having the alternative names beta-mannanase and endo-1,4-mannanase and catalysing hydrolysis of 1,4-beta-D-mannosidic linkages in mannans, galactomannans, glucomannans, and galactoglucomannans. Mannanases are classified according to the Enzyme Nomenclature as EC 3.2.1.78 and belong in Glycosyl Hydrolase families 5, 26 and 113. Many suitable mannanases belong to Glycosyl Hydrolase family 5. Commercially available mannanases include all those sold under the tradenames Mannaway^{®} (Novozymes A/S) such as Mannaway^{®} 200L and Mannaway Evity 4.0T Other commercially available mannanases include Effectenz^{®} M1000, Mannastar^{®} 375, Preferenz M100 and Purabrite^{®} (all DuPont Industrial Biosciences) and Biotouch M7 (AB Enzymes). Other suitable mannanases belong to Glycosyl Hydrolase family 26 including those described in WO2018191135, WO2015040159, WO2017021515, WO2017021516, WO2017021517 and WO2019081515. Suitable mixtures of mannanases include the combinations of Glycosyl Hydrolase family 5 and Glycosyl Hydrolase family 26 mannanases described in WO2019081515.

Xanthan gum-degrading enzymes: The composition may comprise one of more xanthan gum-degrading enzymes. Suitable enzymes for degradation of xanthan gum-based soils include xanthan endoglucanase, optionally in conjunction with a xanthan lyase. As used herein, the term "xanthan endoglucanase" denotes an enzyme exhibiting endo-β-1,4-glucanase activity that is capable of catalysing hydrolysis of the 1,4-linked β-D-glucose polymeric backbone of xanthan gum, optionally in conjunction with a suitable xanthan lyase enzyme. Suitable xanthan endoglucanases are described in WO2013167581, WO2015181299, WO2015181292, WO2017046232, WO2017046260, WO201837062, WO201837065, WO2019038059 and WO2019162000. As used herein, the term "xanthan lyase" denotes an enzyme that cleaves the β-D-mannosyl-β-D-1,4-glucuronosyl bond of xanthan gum. Such enzymes belong to E.C. 4.2.2.12. Suitable xanthan lyases are described in WO2015001017, WO2018037061, WO201837064, WO2019038060, WO2019162000 and WO2019038057.

RNase: suitable RNases include wild-types and variants defined by SEQ ID NOS: 3, 6, 9, 12, 15, 57, 58, 59, 60, 61, 62, 63, 64, 65, 66, 67, 72 and 73 in WO2018178061 (Novozymes), incorporated herein by reference.

Hexosaminidases: The composition may comprise one or more hexosaminidases. The term hexosaminidase includes "dispersin" and the abbreviation "Dsp", which means a polypeptide having hexosaminidase activity, EC 3.2.1 .- that catalyzes the hydrolysis of β-1,6-glycosidic linkages of N-acetyl-glucosamine polymers found in soils of microbial origin. The term hexosaminidase includes polypeptides having N-acetylglucosaminidase activity and β-N-acetylglucosaminidase activity. Hexosaminidase activity may be determined according to Assay II described in WO2018184873. Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944. Variants of the *Terribacillus saccharophilus* hexosaminidase defined by SEQ ID NO: 1 of WO2020207944 may be preferred, especially the variants with improved thermostability disclosed in that publication.

Galactanase: The composition may comprise a galactanase, ie. an extracellular polymer-degrading enzyme that includes an endo-beta-1,6-galactanase enzyme. The term "endo-beta-1,6-galactanase" or "a polypeptide having endo-beta-1,6-galactanase activity" means a endo-beta-1,6-galactanase activity (EC 3.2.1.164) from the glycoside hydrolase family 30 that catalyzes the hydrolytic cleavage of 1,6-3-D-galactooligosaccharides with a degree of polymerization (DP) higher than 3, and their acidic derivatives with 4-O-methylglucosyluronate or glucosyluronate groups at the non-reducing terminals. For purposes of the present disclosure, endo-beta-1,6-galactanase activity is determined according to the procedure described in WO 2015185689 in Assay I. Suitable examples from class EC 3.2.1.164 are described in WO 2015185689, such as the mature polypeptide SEQ ID NO: 2.

The additional enzyme(s) may be included in the detergent composition by adding separate enzyme additives containing an additional enzyme, or a combined enzyme additive comprising two or several or all of the additional enzymes. Such an enzyme additive can be in the form of a granulate, a liquid or slurry, preferably additionally comprising an enzyme stabiliser.

Preferably the or each additional enzyme will be present in the composition in an amount of at least 0.0001 to about 0.1% weight percent of pure active enzyme protein, such as from about 0.0001% to about 0.01%, from about 0.001% to about 0.01% or from about 0.001% to about 0.01% based on the weight of the composition.

Fabric Hueing Agent. The composition may comprise a fabric hueing agent (sometimes referred to as shading, bluing or whitening agents/dyes). Typically the hueing agent provides a blue or violet shade to fabric. Hueing agents can be used either alone or in combination to create a specific shade of hueing and/or to shade different fabric types. This may be provided for example by mixing a red and green-blue dye to yield a blue or violet shade. Hueing agents may be selected from any known chemical class of dye, including but not limited to acridine, anthraquinone (including polycyclic quinones), azine, azo (e.g., monoazo, disazo, trisazo, tetrakisazo, polyazo), including premetallized azo, benzodifurane and benzodifuranone, carotenoid, coumarin, cyanine, diazahemicyanine, diphenylmethane, formazan, hemicyanine, indigoids, methane, naphthalimides, naphthoquinone, nitro and nitroso, oxazine, phthalocyanine, pyrazoles, stilbene, styryl, triarylmethane, triphenylmethane, xanthenes and mixtures thereof. Preferred are azo dyes, especially mono- or bis- azo dyes, triarylmethane dyes and anthraquinone dyes.

Suitable fabric hueing agents include dyes, dye-clay conjugates, and organic and inorganic pigments. Suitable dyes include small molecule dyes and polymeric dyes. Suitable small molecule dyes include small molecule dyes selected from the group consisting of dyes falling into the Colour Index (C.I.) classifications of Direct, Basic, Reactive or hydrolysed Reactive, Solvent or Disperse dyes. Examples of suitable small molecule dyes include for example small molecule dyes selected from the group consisting of Colour Index (Society of Dyers and Colourists, Bradford, UK) numbers Direct Violet dyes such as 9, 35, 48, 51, 66, and 99, Direct Blue dyes such as 1, 71, 80 and 279, Acid Red dyes such as 17, 73, 52, 88 and 150, Acid Violet dyes such as 15, 17, 24, 43, 49, 50 and 51, Acid Blue dyes such as 15, 17, 25, 29, 40, 45, 75, 80, 83, 90 and 113, Acid Black dyes such as 1, Basic Violet dyes such as 1, 3, 4, 10 and 35, Basic Blue dyes such as 3, 16, 22, 47, 66, 75 and 159, Disperse or Solvent dyes such as those described in EP1794275 orEP1794276, or dyes as disclosed in US 7,208,459 B2,and mixtures thereof.

Preferred are polymeric dyes include polymeric dyes selected from the group consisting of polymers containing covalently bound (sometimes referred to as conjugated) chromogens, (dye-polymer conjugates), for example polymers with chromogens co-polymerized into the backbone of the polymer and mixtures thereof. Polymeric dyes include those described in WO2011/98355, WO2011/47987, US2012/090102, WO2010/145887, WO2006/055787 and WO2010/142503.

Preferred polymeric dyes comprise alkoxylated, preferably ethoxylated azo, anthraquinone or triarylmethane dyes. Ethoxylated thiophene azo dyes are especially preferred, for example polymeric dyes selected from the group consisting of fabric-substantive colorants sold under the name of Liquitint^{®} (Milliken, Spartanburg, South Carolina, USA), dye-polymer conjugates formed from at least one reactive dye and a polymer selected from the group consisting of polymers comprising a moiety selected from the group consisting of a hydroxyl moiety, a primary amine moiety, a secondary amine moiety, a thiol moiety and mixtures thereof. Suitable polymeric dyes include polymeric dyes selected from the group consisting of Liquitint^{®} Violet CT, carboxymethyl cellulose (CMC) covalently bound to a reactive blue, reactive violet or reactive red dye such as CMC conjugated with C.I. Reactive Blue 19, sold by Megazyme, Wicklow, Ireland under the product name AZO-CM-CELLULOSE, product code S-ACMC, alkoxylated triphenylmethane polymeric colourants, alkoxylated thiophene polymeric colourants, and mixtures thereof.

Preferred hueing dyes include the alkoxylated thiophene azo whitening agents found in US2008/0177090 which may be optionally anionic, such as those selected from Examples 1-42 in Table 5 of WO2011/011799. Other preferred dyes are disclosed in US 8138222.

Suitable pigments include pigments selected from the group consisting of Ultramarine Blue (C.I. Pigment Blue 29), Ultramarine Violet (C.I. Pigment Violet 15) and mixtures thereof. Pigments and/or dyes may also be added to add colour for aesthetic reasons. Preferred are organic blue, violet and/or green pigments.

Builders: The detergent composition may further contain builders, such as builders based on carbonate, bicarbonate or silicates which may be Zeolites, such as Zeolite A, Zeolite MAP (Maximum Aluminium type P). Zeolites, useable in laundry preferably has the formula Na₁₂(AlO₂)₁₂(SiO₂)₁₂·27H₂O and the particle size is usually between 1-10 µm for zeolite A and 0.7-2 um for zeolite MAP. Other builders are Sodium metasilicate (Na₂SiO₃ · *n*H₂O or Na₂Si₂O₅ · *n* H₂O) strong alkaline and preferably used in dish wash. In preferred embodiments, the amount of a detergent builder may be above 5%, above 10%, above 20%, above 30%, above 40% or above 50%, and may be below 80%, 65%. In a dishwash detergent, the level of builder is typically 40-65%, particularly 50-65% or even 75-90%.

Encapsulates: The composition may comprise an encapsulated benefit agent, comprising a core and a shell having an inner and outer surface, said shell encapsulating said core. The core may comprise a material selected from the group consisting of perfumes; brighteners; dyes; insect repellants; silicones; waxes; flavors; vitamins; fabric softening agents; skin care agents in one aspect, paraffins; enzymes; anti-bacterial agents; bleaches; sensates; and mixtures thereof. The shell may comprise a material selected from the group consisting of polyethylenes; polyamides; polystyrenes; polyisoprenes; polycarbonates; polyesters; polyacrylates; aminoplasts, in one aspect said aminoplast may comprise a polyureas, polyurethane, and/or polyureaurethane, in one aspect said polyurea may comprise polyoxymethyleneurea and/or melamine formaldehyde; polyolefins; polysaccharides, in one aspect said polysaccharide may comprise alginate and/or chitosan; gelatin; shellac; epoxy resins; vinyl polymers; water insoluble inorganics; silicone; and mixtures thereof. Preferred encapsulates comprise a core comprising perfume. Such encapsulates are perfume microcapsules.

Enzyme stabilizer: The composition may comprise an enzyme stabilizer. Suitable enzyme stabilisers may be selected from the group consisting of (a) inorganic salts selected from the group consisting of calcium salts, magnesium salts and mixtures thereof; (b) carbohydrates selected from the group consisting of oligosaccharides, polysaccharides and mixtures thereof and sugar or sugar alcohol; (c) mass efficient reversible protease inhibitors selected from the group consisting of phenyl boronic acid and derivatives thereof, e.g., an aromatic borate ester, or a phenyl boronic acid derivative such as 4-formylphenyl boronic acid, or a peptide aldehyde such as di-, tri- or tetrapeptide aldehydes or aldehyde analogues (either of the form B1-B0-R wherein, R is H, CH3, CX3, CHX2, or CH2X (X=halogen), B0 is a single amino acid residue (preferably with an optionally substituted aliphatic or aromatic side chain); and B1 consists of one or more amino acid residues (preferably one, two or three), optionally comprising an N-terminal protection group, or as described in WO09118375, WO98/13459); and (d) reversible protease inhibitors such as a boron containing compound; (e) polyols such as propylene glycol or glycerol 1-2 propane diol; (f) calcium formate and/or sodium formate; (g) protease inhibitor of the protein type such as RASI, BASI, WASI (bifunctional alpha-amylase/subtilisin inhibitors of rice, barley and wheat) or CI2 or SSI and (h) any combination thereof.

Structurant: In one aspect, the composition may comprise a structurant selected from the group consisting of diglycerides and triglycerides, ethylene glycol distearate microcrystalline cellulose, cellulose-based materials, microfiber cellulose, biopolymers, xanthan gum, gellan gum, and mixtures thereof.

Polymers: The composition preferably comprises one or more polymers. Preferred examples are carboxymethylcellulose, poly(vinyl-pyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid co-polymers and amphiphilic polymers and mixtures thereof.

Amphiphilic cleaning polymers: Preferably, the amphiphilic cleanimg polymer is a compound having the following general structure: bis((C₂H₅O)(C₂H₄O)n)(CH₃)-N⁺-CₓH₂ₓ-N⁺-(CH₃)-bis((C₂H₅O)(C₂H₄O)n), wherein n = from 20 to 30, and x = from 3 to 8, or sulphated or sulphonated variants thereof.

Amphiphilic alkoxylated grease cleaning polymers of the present invention refer to any alkoxylated polymer having balanced hydrophilic and hydrophobic properties such that they remove grease particles from fabrics and surfaces. Specific embodiments of the amphiphilic alkoxylated grease cleaning polymers of the present invention comprise a core structure and a plurality of alkoxylate groups attached to that core structure. These may comprise alkoxylated polyalkylenimines, preferably having an inner polyethylene oxide block and an outer polypropylene oxide block.

The core structure may comprise a polyalkylenimine structure comprising, in condensed form, repeating units of formulae (I), (II), (III) and (IV): 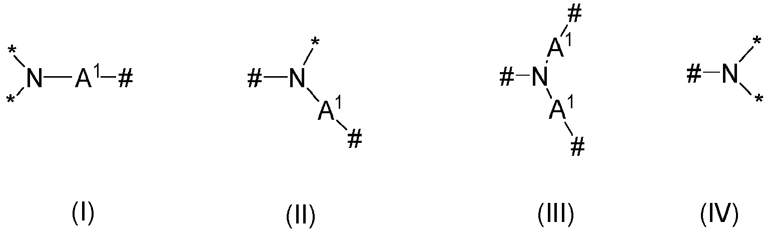 wherein # in each case denotes one-half of a bond between a nitrogen atom and the free binding position of a group A¹ of two adjacent repeating units of formulae (I), (II), (III) or (IV); ^{∗} in each case denotes one-half of a bond to one of the alkoxylate groups; and A¹ is independently selected from linear or branched C₂-C₆-alkylene; wherein the polyalkylenimine structure consists of 1 repeating unit of formula (I), x repeating units of formula (II), y repeating units of formula (III) and y+1 repeating units of formula (IV), wherein x and y in each case have a value in the range of from 0 to about 150; where the average weight average molecular weight, Mw, of the polyalkylenimine core structure is a value in the range of from about 60 to about 10,000 g/mol.

The core structure may alternatively comprise a polyalkanolamine structure of the condensation products of at least one compound selected from N-(hydroxyalkyl)amines of formulae (I.a) and/or (I.b), 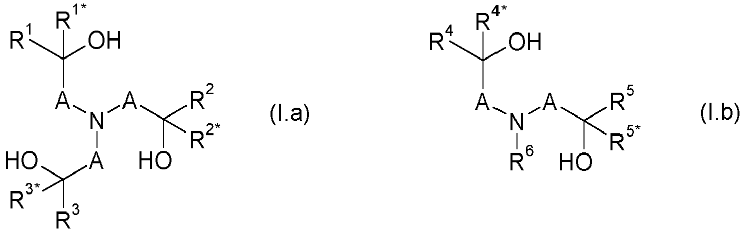 wherein A are independently selected from C₁-C₆-alkylene; R¹, R^{1∗}, R², R^{2∗}, R³, R^{3∗}, R⁴, R^{4∗}, R⁵ and R^{5∗} are independently selected from hydrogen, alkyl, cycloalkyl or aryl, wherein the last three mentioned radicals may be optionally substituted; and R⁶ is selected from hydrogen, alkyl, cycloalkyl or aryl, wherein the last three mentioned radicals may be optionally substituted.

The plurality of alkylenoxy groups attached to the core structure are independently selected from alkylenoxy units of the formula (V) 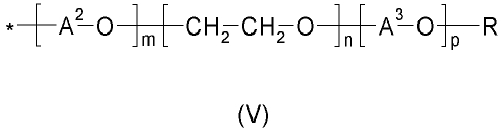 wherein ^{∗} in each case denotes one-half of a bond to the nitrogen atom of the repeating unit of formula (I), (II) or (IV); A² is in each case independently selected from 1,2-propylene, 1,2-butylene and 1,2-isobutylene; A³ is 1,2-propylene; R is in each case independently selected from hydrogen and C₁-C₄-alkyl; m has an average value in the range of from 0 to about 2; n has an average value in the range of from about 20 to about 50; and p has an average value in the range of from about 10 to about 50.

Carboxylate polymer: The composition preferably also includes one or more carboxylate polymers such as a maleate/acrylate random copolymer or polyacrylate homopolymer. In one aspect, the carboxylate polymer is a polyacrylate homopolymer having a molecular weight of from 4,000 Da to 9,000 Da, or from 6,000 Da to 9,000 Da.

Soil release polymer: The composition preferably also comprises one or more soil release polymers. Preferred are those having a structure as defined by one of the following structures (I), (II) or (III):
(I)

   -[(OCHR¹-CHR²)ₐ-O-OC-Ar-CO-]_{d}
(II)

   -[(OCHR³-CHR⁴)_{b}-O-OC-sAr-CO-]ₑ
(III)

   -[(OCHR⁵-CHR⁶)_{c}-OR⁷]_{f}

   wherein:
   a, b and c are from 1 to 200;
   d, e and f are from 1 to 50;
   Ar is a 1,4-substituted phenylene;
   sAr is 1,3-substituted phenylene substituted in position 5 with SO₃Me;
   Me is Li, K, Mg/2, Ca/2, Al/3, ammonium, mono-, di-, tri-, or tetraalkylammonium wherein the alkyl groups are C₁-C₁₈ alkyl or C₂-C₁₀ hydroxyalkyl, or mixtures thereof;
   R¹, R², R³, R⁴, R⁵ and R⁶ are independently selected from H or C₁-C₁₈ n- or iso-alkyl; and
   R⁷ is a linear or branched C₁-C₁₈ alkyl, or a linear or branched C₂-C₃₀ alkenyl, or a cycloalkyl group with 5 to 9 carbon atoms, or a C₈-C₃₀ aryl group, or a C₆-C₃₀ arylalkyl group.

Suitable soil release polymers are polyester soil release polymers such as Repel-o-tex polymers, including Repel-o-tex SF, SF-2 and SRP6 supplied by Rhodia. Other suitable soil release polymers include Texcare polymers, including Texcare SRA100, SRA300, SRN100, SRN170, SRN240, SRN300 and SRN325 supplied by Clariant. Other suitable soil release polymers are Marloquest polymers, such as Marloquest SL supplied by Sasol.

Cellulosic polymer: The composition preferably also comprises one or more cellulosic polymers including those selected from alkyl cellulose, alkyl alkoxyalkyl cellulose, carboxyalkyl cellulose, alkyl carboxyalkyl cellulose. In one aspect, the cellulosic polymers are selected from the group comprising carboxymethyl cellulose, methyl cellulose, methyl hydroxyethyl cellulose, methyl carboxymethyl cellulose, and mixures thereof. In one aspect, the carboxymethyl cellulose has a degree of carboxymethyl substitution from 0.5 to 0.9 and a molecular weight from 100,000 Da to 300,000 Da.

Bleaching system: The composition may contain a bleaching system, for example comprising a H₂O₂ source such as perborate or percarbonate which may be combined with a peracid-forming bleach activator such as tetraacetylethylenediamine or nonanoyloxybenzenesulfonate. Alternatively, the bleaching system may comprise peroxyacids of, e.g., the amide, imide, or sulfone type. In general, when a bleaching agent is used, the compositions of the present invention may comprise from about 0.1% to about 30% or even from about 0.1 % to about 25% bleaching agent by weight of the subject cleaning composition.

Chelating Agents: The composition preferably comprises a chelating agent, preferably in an amount from 0.005% to about 15% or even from about 3.0% to about 10% chelating agent by weight of the composition. Suitable chelating agents include copper, iron and/or manganese chelating agents and mixtures thereof. Preferred chelants (complexing agents) include DTPA (Diethylene triamine pentaacetic acid), HEDP (Hydroxyethane diphosphonic acid), DTPMP (Diethylene triamine penta(methylene phosphonic acid)), 1,2-Dihydroxybenzene-3,5-disulfonic acid disodium salt hydrate, ethylenediamine, diethylene triamine, ethylenediaminedisuccinic acid (EDDS), N-hydroxyethylethylenediaminetri-acetic acid (HEDTA), triethylenetetraaminehexaacetic acid (TTHA), N-hydroxyethyliminodiacetic acid (HEIDA), dihydroxyethylglycine (DHEG), ethylenediaminetetrapropionic acid (EDTP), methyl-glycine-diacetic acid (MGDA), glutamic-N,N- diacetic acid (GLDA), iminodisuccinic acid (IDS), carboxy methyl inulin; and salts derivatives thereof and mixtures thereof. Preferred chelants are selected from the group consisting of methyl-glycine-diacetic acid (MGDA), its salts and derivatives thereof, glutamic-N,N- diacetic acid (GLDA), its salts and derivatives thereof, iminodisuccinic acid (IDS), its salts and derivatives thereof, carboxy methyl inulin, its salts and derivatives thereof and mixtures thereof. MGDA and salts thereof are especially preferred, in particular comprising the three-sodium salt of MGDA.

The composition may also contain other conventional detergent ingredients such as e.g. fabric conditioners including clays, foam boosters, suds suppressors, anti-corrosion agents, soil-suspending agents, anti-soil re-deposition agents, dyes, bactericides, optical brighteners, hydrotropes, tarnish inhibitors, organic solvents such as ethanol or perfumes.

### Method of Use

The present invention also provides a method for treating a fabric, the method comprising in a contacting step, contacting a fabric with an aqueous wash liquor comprising a DNase variant as described herein, preferably in an amount from 0.01ppm to 10ppm, preferably from 0.1ppm to 1ppm; and a cleaning adjunct.

A preferred cleaning adjunct comprises an anionic surfactant preferably in an amount from 0.05 to 50g/l, more preferably from 0.2g/l to 5g/l or 0.5g/l to 3g/l.

The aqueous wash liquor may be formed by adding a detergent composition as described above to water, for example in a washing machine or hand washing process. The concentration of detergent composition is typically from 500ppm to 15000ppm, preferably from 1000 to 10000ppm, preferably from 1000 to 5000ppm.

Alternatively, the aqueous wash liquor may be formed by adding the DNase variant and cleaning adjunct as separate components, into water to form the wash liquor. The fabric may be optionally subsequently washed, and/or rinsed and/or dried.

In the contacting step, or in a subsequent step, it may be preferred to use mechanical agitation to promote cleaning and removal of the broken-down soil by-products from the fabric. The wash liquor preferably has a pH of from about 7 or 8 to about 10.5. The wash liquor preferably has a temperature from about 5 °C to about 40 °C, or preferably from 10 to 30 °C or less than 30 °C. The water to fabric ratio is typically from about 1:1 to about 30:1.

The pH of the wash liquor is typically in the range about 5.5 to about 10, more typically in the range of 7 to 9, such as in the range of about 7 to about 8.5 or about 7 to about 8.

The concentration of the DNase variant, and any additional enzyme, in the wash liquor is typically in the range of from 0.00001 ppm to 10 ppm enzyme protein, from 0.00002 ppm to 10 ppm, from 0.0001 ppm to 10 ppm, from 0.0002 ppm to 10 ppm, from 0.001 ppm to 10 ppm, from 0.002 ppm to 10 ppm, from 0.01 ppm to 10 ppm, from 0.02 ppm to 10 ppm, from 0.1 ppm to 10 ppm, from 0.2 ppm to 10 ppm, or from 0.5 ppm to 5 ppm.

### EXAMPLES

### Materials and methods

### Assay I: Determination of DNase activity

DNase activity may be determined by using the DNaseAlert^{™} Kit (IDT Intergrated DNA Technologies, Belgium) according to the supplier's manual. See Example 2 below for an example of this assay.

### Assay II: Storage stability assay for purified DNase variants

The storage stability of purified DNase variants in detergent compositions, expressed as a half-life improvement factor relative to a reference DNase, may e.g. be determined as described in Example 3 below.

### Example 1: Construction and screening of variants

Site-directed variants of the DNase of SEQ ID NO: 1 comprising specific substitutions were constructed. The variants were made by traditional cloning of DNA fragments (Sambrook et al., Molecular Cloning: A Laboratory Manual, 2nd Ed., Cold Spring Harbor, 1989) using PCR together with properly designed mutagenic oligonucleotides that introduced the desired mutations in the resulting sequence.

Mutagenic oligos were designed corresponding to the DNA sequence flanking the desired site(s) of mutation, separated by the DNA base pairs defining the insertions/deletions/- substitutions, and purchased from an oligo vendor such as Life Technologies. To test the DNase variants, mutated DNA comprising a variant was integrated into a competent *Bacillus subtilis* strain by homologous recombination. The transformations were grown for 1 hour in TB-Gly growth medium without antibiotic and then overnight in TB-Gly medium with 3 µg/ml chloramphenicol. After addition of glycerol to 25% v/v these were stored at -80°C. Replicates were grown for 3-4 days for DNase production in broth supplemented with chloramphenicol and trace elements: 50 µM FeCl3 , 20 µM CaCl2, 10 µM MnCl2, 10 µM ZnSO4 and 2 µM CuCl2.

### Example 2: Stability screening of DNase variants in supernatant

After growth, each supernatant sample was split into two identical samples by transferring 20 µl supernatant to two 96-well standard microtiter plates each containing 170 µl Tide Pods^{®} 3-in-1 detergent supplemented with 1% (v/v) of a liquid laundry protease, Progress^{®} Uno 101 L (Novozymes A/S), and 0.3% (w/v) sodium bisulfite. After shaking for 20 minutes at 7500 rpm in a microtiter plate shaker, one microtiter plate was incubated at 10°C (reference condition) for three days and the other microtiter plate was incubated in an incubator at 50°C for three days (stress condition). After three days, both sets of samples (the reference condition and the stressed condition) were diluted 20-fold in dilution buffer (50 mM Tris, HCl, 0.01% Tween20, pH 7.5) before assaying the activity using the DNAseAlert^{™} substrate solution (Integrated DNA Technologies, Belgium, part #11-04-02-04). 10 µl 20-fold diluted reference condition DNase sample and stress condition DNase sample were transferred to a new 384-well microtiter plate and 40 µl DNAseAlert^{™} assay solution was added (50 mM TrisHCl, pH 7.5, 5 mM MnCl₂, 0.01% Tween20, 10 µM DNAseAlert^{™} substrate). The fluorescence (excitation 536 nm, emission 556 nm) was read every 90 seconds for a total of 30 minutes. From the kinetic curves, the slope of the reference sample (activity under reference conditions) and the corresponding stress sample (activity under stress conditions) was determined by linear regression. The residual activity (RA) for each DNase variant and the reference DNase (SEQ ID NO: 1) was calculated as: slope (stress sample)/slope (reference sample). The RA was used for calculation of half-lives, the half-life improvement factor being calculated as: stress time (minutes) ^{∗} ln(0.5)/ln(RA). The calculated half-lives for the variants and the reference were used for calculation of the half-life improvement factor as the ratio between half-life of the variant and the half-life of the reference. The half-life improvement factor for the reference is by definition 1.0, and variants with half-life improvement factors larger than the reference have improved stability under the test conditions.

Single mutation variants showing improved stability in supernatant screening were used for construction and screening of combination variants comprising two or more individual mutations.

The transformations of these improved single mutation hits were streaked into single colonies, sequenced, grown overnight in TB-Gly broth with 3 µg/ml chloramphenicol. After growth in shakeflasks in 100 ml PS-1 growth medium with trace elements for 4 days at 30°C the enzyme variants were purified and their stability was tested in two different liquid detergents as described below.

Single mutations resulting in improved stability were combined in combination variants containing two or more mutations compared to SEQ ID NO: 1. These were streaked to single colonies directly after transformation and then screened as described above and sequenced. Improved combination variants were subsequently grown, purified and tested for storage stability in two different liquid detergents as described below.

For variants that were subsequently tested in purified form, stability data is shown in Tables 2 and 3 in Example 3. Table 1A shows stability data for a few additional variants tested as supernatants in concentrated liquid detergent (Tide Pods^{®} 3-in-1) stored at 55°C.

Other DNase variants were tested in a similar manner as described above, although with incubation in 10% Ariel Essential detergent at a temperature of 58°C and together with 1% of a protease (SEQ ID NO: 28). The results of this test are shown in Table 1B below.

**Table 1A: Half-life Improvement Factor of DNase variants tested as supernatants in concentrated liquid detergent (Tide Pods^{®} 3-in-1)**

| **Substitutions compared to SEQ ID NO: 1** | **Half-life improvement factor** |
|---|---|
| K21L,N61D,T65I,K107Q,T127S | 2.87 |
| Q14W,N61D,T65I,R109T,G149N,S164D,L181W | 2.18 |
| K21L,N61D,T65I,K107Q | 2.08 |
| Q14W,N61D,T65I,R109T,G149N | 1.87 |
| Q14W,T65V,R109T,G149N,W154I,L181W | 1.65 |
| Q14W,T65V,R109T,G149N,W154I,S164D | 1.62 |
| Q14W,N61D,T65V,R109T,G149N,L181W | 1.59 |
| Q14W,T65I,R109T,D116W,G149N,S164D,L181W | 1.44 |

**Table 1B: Half-life improvement factor of DNase variants tested as supernatants in concentrated liquid detergent (Ariel Essential)**

| **Substitutions compared to SEQ ID NO: 1** | **Half-life improvement factor** |
|---|---|
| T65V,R109T,T127V,T171W | 3.38 |
| R109T,T127V,T171W | 2.63 |
| T65V,R109T,T127V | 2.02 |

### Example 3: Storage stability assay for purified DNase variants

After purification (2-step procedure using a PPA HyperCel column and an SP-Sepharose^{®} FF column), purified DNase variants were diluted with 0.01% Triton X-100 to 0.2 and 0.1 mg/ml with the concentration calculated using absorbance at 280 nm. For each enzyme variant, two wells with the high concentration (0.2 mg/ml) and two wells with the low concentration (0.1 mg/ml) were tested. 15 µl diluted DNase sample was mixed with 285 µl concentrated detergent (either 1) Tide Pods^{®} 3-in-1, green compartment with 0.3% sodium bisulfite and 1% of a liquid laundry protease, Progress^{®} Uno 101 L (Novozymes A/S) added, or Tide Original HE heavy-duty liquid with 1% Progress^{®} Uno 101 L added, in the well of a microtiter plate ("detergent plate", Nunc U96 PP 0.5 ml) using a magnetic bar. After mixing the detergent plates were incubated at 50°C in a Biosan PST-100HL thermomixer.

After various incubation times (e.g. 0, 2, 24 and 72 hours), residual DNase activity was measured. 5 µl from the detergent plate was mixed with 195 µl DNA substrate solution (3.3 mg DNA (Sigma D1626) in 50 mM Tris pH 7, 5 mM MgCl₂, 5 mM CaCl₂, 1.3 mM EDTA). Once a minute for 30 minutes, viscosity was measured using pressure sensing during aspiration with a Hamilton Microlab STAR. From the measured reduction in viscosity, an activity was calculated.

The decrease in activity during incubation with detergent is assumed to be exponential. Half-lives (T½) are found from linear regression of Log(Activity) versus incubation time, and half-life improvement factors (HIF) were calculated as half-life of DNase variants relative to the half-life of a reference DNase.

The half-life improvement factor of DNase variants is shown in Tables 2 and 3 below, where Table 2 shows the results for variants tested in the concentrated liquid detergent Tide Pods^{®} 3-in-1, while Table 3 shows the results for variants tested in the heavy-duty liquid detergent Tide Original HE.

**Table 2: Half-life Improvement Factor of purified DNase variants tested in concentrated liquid detergent (Tide Pods^{®} 3-in-1)**

| **Substitutions compared to SEQ ID NO: 1** | **Half-life improvement factor** |
|---|---|
| Q14R,Q48D,T65I,K107Q,T127S,S164D | 9.05 |
| K21L,Q48D,T65I,K107Q,T127S | 8.64 |
| K21L,Q48D,T65I,S82R,K107Q,T127S | 7.08 |
| Q14R,K21L,Q48D,T65I,T127S | 6.95 |
| Q48D,T65I,S82R,T127S,S164D | 6.92 |
| K21L,Q48D,T65I,T127S | 6.68 |
| N61D,T65I,K107Q,T127S,S164D | 6.64 |
| Q48D,T65I,S82R,K107Q,T127S | 6.54 |
| Q14R,N61D,T65I,S82R,K107Q | 6.18 |
| N61D,S68L,G149N | 6.15 |
| Q14R,N61D,T65I,S82R,T127S,S164D | 6.11 |
| K21L,Q48D,T65I,S82R,K107Q | 5.88 |
| Q14R,K21L,N61D,T65I,K107Q,T127S | 5.69 |
| Q14R,T65I,K107Q,T127S | 5.62 |
| N61D,T65I,S82R,T127S,S164D | 5.59 |
| K21L,N61D,T65I,S82R,K107Q,T127S | 5.49 |
| T65V,T127V,G149N | 5.43 |
| Q14R,K21L,Q48D,T65I,K107Q,T127S | 5.40 |
| T65I,K107Q,T127S,S164D | 5.36 |
| N61D,T65I,S82R,K107Q | 5.33 |
| Q14R,K21L,N61D,T65I,S82R | 5.28 |
| Q14R,K21L,N61D,T65I,S82R,K107Q | 5.25 |
| Q14R,K21L,N61D,T65I,T127S | 5.23 |
| N61D,T65I,S82R,K107Q,T127S,S164D | 5.21 |
| Q14R,K21L,T65I,K107Q,T127S | 5.19 |
| N61D,T65I,K107Q,T127S | 5.13 |
| T65I,S82R,K107Q,S164D | 5.00 |
| K21L,N61D,T65I,S82R | 4.83 |
| K21L,N61D,T65I,T127S | 4.81 |
| N61D,T65I,S82R,S164D | 4.79 |
| K21L,N61D,T65I,S82R,K107Q | 4.76 |
| S68L,S106A,G149N | 4.73 |
| N61D,T65I,T127S,S164D | 4.67 |
| Q14R,K21L,N61D,T65I | 4.63 |
| Q14R,K21L,T65I,T127S | 4.55 |
| N61D,T65I,S82R,T127S,S164D,T171E,D175G,L181S | 4.54 |
| T65V,G149N | 4.36 |
| T65V,Y77R,G149N | 4.23 |
| T65V,R109T,T127V | 4.21 |
| T65V,Y77H,G149N | 4.20 |
| Q14R,K21L,T65I,K107Q | 4.18 |
| K21L,T65I,S82R,K107Q | 4.18 |
| K21L,T65I,K107Q,T127S | 4.15 |
| T65V,L76K,G149N | 4.00 |
| N61D,S68L,S102Y,G149N,S164D,L181T | 3.92 |
| N61D,S68L,S106A,G149N,S164D | 3.88 |
| T65V,R109T,G149N | 3.75 |
| T65V,L76R,G149N | 3.71 |
| T65V,T127V,T171W | 3.68 |
| T19E,P25S,L33K,D56I,T65V,Y77T,T127V,L181S | 3.59 |
| T65V,T127V,L181S | 3.55 |
| T65I,S164D,L181W | 3.46 |
| T65I,L181S | 3.15 |
| S66L,G149N | 3.09 |
| N61D,S66Y,S102Y,S164D | 3.09 |
| N61D,S66Y,S164D | 3.05 |
| N61D,T65V,S164D | 2.98 |
| T65V,Y77T,G149N,L181S | 2.97 |
| T65V,Y77T,G149N | 2.96 |
| D56N,T65V,Y77T,G149N | 2.79 |
| D56N,T65V,L76H,G149N | 2.76 |
| D56Q,T65V,L76H,G149N | 2.42 |
| Q14W,N61D,T65I | 2.23 |
| S68Q,G149N,L181S | 2.13 |
| Q14W,N61D,T65I,S164D | 2.10 |
| Q14W,N61D,T65I,S164D,L181W | 2.06 |
| T65I,D116W,S164D,L181W | 1.88 |
| T65I,D116W,S164D | 1.78 |
| Q14W,T65I,S164D | 1.65 |
| R109T,G149N | 1.57 |
| G149N,T171W | 1.55 |
| G149N,L181S | 1.55 |
| G149N | 1.53 |
| S164D | 1.10 |

**Table 3: Half-life Improvement Factor of purified DNase variants tested in heavy-duty liquid detergent (Tide Original HE)**

| **Substitutions compared to SEQ ID NO: 1** | **Half-life improvemen t factor** |
|---|---|
| P25S,L33K,D56I,T65V,Y77T,T127V,L181S | 3.57 |
| S66L,K107E,G149N,Q166D,L181S | 3.40 |
| Q48D,T65I,S82R,K107Q,T127S | 3.32 |
| N61D,T65I,S82R,K107Q,L181D | 3.30 |
| T65I,G149N,Q166D | 3.30 |
| T65I,G149N,Q166D,Y182G | 3.21 |
| P25S,L33K,T65V,Y77T,T127V,L181S | 3.05 |
| T65I,G149N,Q166D,L181S | 3.01 |
| P25S,L33K,D56I,T65V,Y77T,R109Q,T127V,L181S | 3.00 |
| T65V,G149N,L181E | 2.93 |
| T65I,G149N,L181S | 2.92 |
| T19E,P25S,L33K,D56I,T65V,Y77T,T127V,L181S | 2.76 |
| T65V,T127V,G149N,Y182D | 2.59 |
| T65V,T127V,L181S | 2.54 |
| Q48D,N61D,T65I,S82R,K107Q | 2.53 |
| T65I,L181S | 2.50 |
| P25S,L33K,D56I,T65V,Y77T,D116S,T127V,L181S | 2.49 |
| T65I,Q166D,Y182G | 2.48 |
| D56L,T65V,T127V | 2.44 |
| Q48D,T65I,S82R,T127S,S164D | 2.43 |
| T65I,Y182G | 2.43 |
| S68Q,G149N,Q166D,L181S | 2.38 |
| N61D,S68L,G149N | 2.38 |
| T65V,T127V,G149N | 2.35 |
| D56L,T65V,T127V,T171W | 2.34 |
| Q48D,T65V,G149N | 2.31 |
| T65V,G149N,L181T | 2.29 |
| T19I,S68Q,G149N,Y182G | 2.22 |
| N61D,T65I,S82R,K107Q | 2.15 |
| T65I,K107E,G149N,Q166D,Y182G | 2.14 |
| G149N,Q166D,Y182G | 2.14 |
| T65V,T127V,T171W | 2.12 |
| S66L,G149N | 2.12 |
| G149N,L181S | 2.09 |
| T65I,K107E,G149N | 2.06 |
| S68Q,G149N,Q166D | 2.04 |
| N61D,T65I,S82R,T127S,S164D,Y182D | 2.04 |
| N61D,T65I,S82R,T127S,S164D,L181D | 2.01 |
| G149N,Y182G | 1.95 |
| S68L,S106A,G149N | 1.94 |
| Q48D,N6 1D,T65I,K 107Q,T 127S, S164D | 1.94 |
| T65V,G149N,T171W | 1.93 |
| G149N,Q166D,L181S | 1.89 |
| S68Q,G149N,L181S | 1.86 |
| Q48D,T65I,K107Q,T127S,S164D | 1.83 |
| N61D,T65I,S82R,T127S,S164D,Y182N | 1.79 |
| S68Q,K107E,G149N,Y182G | 1.77 |
| T65I,K107Q,T127S,S164D,Y182D | 1.76 |
| T65V,Y77T,G149N,L181S | 1.73 |
| T65V,Y77T,T127V | 1.71 |
| K107E,G149N,Y182G | 1.70 |
| T65V,G149N | 1.68 |
| N61D,T65I,K107Q,T127S,S164D,L181E | 1.68 |
| N61D,T65I,S82R,T127S,S164D | 1.65 |
| N61D, S68L,S106A,G149N,S164D | 1.61 |
| N61D,S66Y,S102Y,S164D | 1.60 |
| N61D,T65I,K107Q,T127S,S164D,L181D | 1.59 |
| T65I,K107Q,T127S,S164D,L181T | 1.53 |
| T65I,K107Q,T127S,S164D,L181E | 1.48 |
| T65V,Y77R,G149N | 1.48 |
| T65V,Y77T,G149N | 1.47 |
| N61D,T65I,K107Q,T127S,S164D,Y182D | 1.46 |
| T65I,K107Q,T127S,S164D,Y182N | 1.45 |
| N61D,T65I,S82R,T127S,S164D,T171E,D175G,L181S | 1.45 |
| T65V,Y77H,G149N | 1.43 |
| N61D,T65I,K107Q,T127S,S164D,L 181Q | 1.43 |
| N61D,T65V,S164D,Y182N | 1.43 |
| N61D,T65I,K107Q,T127S,S164D,L181T | 1.42 |
| N61D,T65I,S82R,S164D | 1.41 |
| T65I,K107Q,T127S,S164D,L181D | 1.41 |
| N61D,S66Y,S164D | 1.40 |
| N61D,T65I,K107Q,T127S,S164D | 1.39 |
| T65I,S82R,K107Q,S164D | 1.39 |
| T65V,R109T,T127V,G149N | 1.38 |
| G149N | 1.36 |
| N61D,T65I,T127S,S164D | 1.34 |
| T65I,K107Q,T127S,S164D,L181Q | 1.34 |
| T65V,R109T,T127V | 1.31 |
| N61D,T65V,T127S,S164D | 1.30 |
| T19E,T65I,K107Q,T127S,S164D | 1.29 |
| Q48D,N61D,T65V,S164D | 1.28 |
| D56N,T65V,Y77T,G149N | 1.27 |
| N61D,T65I,K107Q,T127S,S164D,Y182N | 1.26 |
| T65V,R109T,G149N | 1.24 |
| T65I,K107Q,T127S,S164D | 1.20 |
| G149N,T171W | 1.19 |
| T65V,R109T,T171W | 1.18 |
| T65V,R109T,G149N,T171W | 1.16 |
| N61D,T65V,S164D | 1.15 |
| T65V,L76K,G149N | 1.15 |
| D56N,T65V,L76H,G149N | 1.12 |
| T65I,S164D,L181W | 1.11 |
| P25S,D56I,T65V,Y77T,T127V,L181S | 1.10 |

### DETERGENT EXAMPLES

Examples 1-6. Granular laundry detergent compositions designed for hand washing or top-loading washing machines.

| | 1 (wt %) | 2 (wt %) | 3 (wt %) | 4 (wt %) | 5 (wt %) | 6 (wt %) |
|---|---|---|---|---|---|---|
| Linear alkylbenzenesulfonate | 20 | 22 | 20 | 15 | 20 | 20 |
| C12-14 Dimethylhydroxyethyl ammonium chloride | 0.7 | 0.2 | 1 | 0.6 | 0.0 | 0.0 |
| AE3S | 0.9 | 1 | 0.9 | 0.0 | 0.5 | 0.9 |
| AE7 | 0.0 | 0.0 | 0.0 | 1 | 0.0 | 3 |
| Sodium tripolyphosphate | 5 | 0.0 | 4 | 9 | 2 | 0.0 |
| Zeolite A | 0.0 | 1 | 0.0 | 1 | 4 | 1 |
| 1.6R Silicate (SiO2:Na2O at ratio 1.6:1) | 7 | 5 | 2 | 3 | 3 | 5 |
| Sodium carbonate | 25 | 20 | 25 | 17 | 18 | 19 |
| Polyacrylate MW 4500 | 1 | 0.6 | 1 | 1 | 1.5 | 1 |
| Random graft copolymer¹ | 0.1 | 0.2 | 0.0 | 0.0 | 0.0 | 0.0 |
| Carboxymethyl cellulose | 1 | 0.3 | 1 | 1 | 1 | 1 |
| Protease (Savinase^{®}, 32.89 mg active/g) | 0.1 | 0.1 | 0.1 | 0.1 | | 0.1 |
| ⁵DNase as defined herein (mg active per 100g composition) | 4.0 | 6.0 | 10.0 | 2.2 | 4.4 | 1.5 |
| Lipase - Lipex^{®} (18 mg active /g) | 0.03 | 0.07 | 0.3 | 0.1 | 0.07 | 0.4 |
| ⁴Amylase Stainzyme^{®} Plus (mg active) | 3.0 | 5.0 | 3.0 | 2.2 | 6.0 | 6.0 |
| Fluorescent Brightener 1 | 0.06 | 0.0 | 0.06 | 0.18 | 0.06 | 0.06 |
| Fluorescent Brightener 2 | 0.1 | 0.06 | 0.1 | 0.0 | 0.1 | 0.1 |
| DTPA | 0.6 | 0.8 | 0.6 | 0.25 | 0.6 | 0.6 |
| MgSO4 | 1 | 1 | 1 | 0.5 | 1 | 1 |
| Sodium Percarbonate | 0.0 | 5.2 | 0.1 | 0.0 | 0.0 | 0.0 |
| Sodium Perborate Monohydrate | 4.4 | 0.0 | 3.85 | 2.09 | 0.78 | 3.63 |
| NOBS | 1.9 | 0.0 | 1.66 | 0.0 | 0.33 | 0.75 |
| TAED | 0.58 | 1.2 | 0.51 | 0.0 | 0.015 | 0.28 |
| Sulphonated zinc phthalocyanine | 0.0030 | 0.0 | 0.0012 | 0.0030 | 0.0021 | 0.0 |
| S-ACMC | 0.1 | 0.0 | 0.0 | 0.0 | 0.06 | 0.0 |
| Direct Violet 9 | 0.0 | 0.0 | 0.0003 | 0.0005 | 0.0003 | 0.0 |
| Acid Blue 29 | 0.0 | 0.0 | 0.0 | 0.0 | 0.0 | 0.0003 |
| Sulfate/Moisture | Balance | | | | | |

Examples 7-13. Granular laundry detergent compositions designed for front-loading automatic washing machines.

| | 7 (wt%) | 8 (wt%) | 9 (wt%) | 10 (wt%) | 11 (wt%) | 12 (wt%) | 13 (wt%) |
|---|---|---|---|---|---|---|---|
| Linear alkylbenzenesulfonate | 8 | 7.1 | 7 | 6.5 | 7.5 | 7.5 | 11 |
| AE3S | 0 | 4.8 | 0 | 5.2 | 4 | 4 | 0 |
| C12-14 Alkylsulfate | 1 | 0 | 1 | 0 | 0 | 0 | 1 |
| AE7 | 2.2 | 0 | 3.2 | 0 | 0 | 0 | 1 |
| C10-12 Dimethyl hydroxyethylammonium chloride | 0.75 | 0.94 | 0.98 | 0.98 | 0 | 0 | 0 |
| Crystalline layered silicate (δ-Na2Si2O5) | 4.1 | 0 | 4.8 | 0 | 0 | 0 | 7 |
| Zeolite A | 5 | 0 | 5 | 0 | 2 | 2 | 4 |
| Citric Acid | 3 | 5 | 3 | 4 | 2.5 | 3 | 0.5 |
| Sodium Carbonate | 15 | 20 | 14 | 20 | 23 | 23 | 14 |
| Silicate 2R (SiO2:Na2O at ratio 2:1) | 0.08 | 0 | 0.11 | 0 | 0 | 0 | 0.01 |
| Soil release agent | 0.75 | 0.72 | 0.71 | 0.72 | 0 | 0 | 0.1 |
| Acrylic Acid/Maleic Acid Copolymer | 1.1 | 3.7 | 1.0 | 3.7 | 2.6 | 3.8 | 2 |
| Carboxymethylcellulose | 0.15 | 1.4 | 0.2 | 1.4 | 1 | 0.5 | 0.2 |
| Protease - Purafect^{®} (84 mg active/g) | 0.2 | 0.2 | 0.3 | 0.15 | 0.12 | 0.13 | 0.18 |
| Lipase - Lipex^{®} (18.00 mg active/g) | 0.05 | 0.15 | 0.1 | 0 | 0 | 0 | 0.1 |
| Cellulase - Celluclean^{™} (15.6 mg active/g) | 0 | 0 | 0 | 0 | 0.1 | 0.1 | 0 |
| ⁴Amylase Stainzyme^{®} Plus (mg active) | 4.0 | 5.0 | 10 | 2.2 | 4.4 | 1.5 | 1.5 |
| Mannanase - Mannaway^{®} (4mg active/g) | 0.05 | 0.1 | 0 | 0.05 | 0.1 | 0 | 0.1 |
| ⁵DNase as defined herein (mg active per 100g detergent) | 4.0 | 5.0 | 10.0 | 2.2 | 8.0 | 1.5 | 0.0 |
| Hexosaminidase (mg active per 100g of detergent) | 3.3 | 9.2 | 12.0 | 4.7 | 3.7 | 13.2 | 3.3 |
| TAED | 3.6 | 4.0 | 3.6 | 4.0 | 2.2 | 1.4 | 1 |
| Percarbonate | 13 | 13.2 | 13 | 13.2 | 16 | 14 | 10 |
| Na salt of Ethylenediamine-N,N'-disuccinic acid, (S,S) isomer (EDDS) | 0.2 | 0.2 | 0.001 | 0.2 | 0.2 | 0.2 | 0.001 |
| Hydroxyethane di phosphonate (HEDP) | 0.2 | 0.2 | 0.5 | 0.2 | 0.2 | 0.2 | 0.5 |
| MgSO4 | 0.42 | 0.42 | 0.42 | 0.42 | 0.4 | 0.4 | 0 |
| Perfume | 0.5 | 0.6 | 0.5 | 0.6 | 0.6 | 0.6 | 0.8 |
| Suds suppressor agglomerate | 0.05 | 0.1 | 0.05 | 0.1 | 0.06 | 0.05 | 0.05 |
| Soap | 0.45 | 0.45 | 0.45 | 0.45 | 0 | 0 | 0 |
| Sulphonated zinc phthalocyanine (active) | 0.000 7 | 0.001 2 | 0.000 7 | 0 | 0 | 0 | 0 |
| S-ACMC | 0.01 | 0.01 | 0 | 0.01 | 0 | 0 | 0 |
| Direct Violet 9 (active) | 0 | 0 | 0.000 1 | 0.000 1 | 0 | 0 | 0.001 |
| Sulfate/ Water & Miscellaneous | Balance | | | | | | |

| | | | | | | | |
|---|---|---|---|---|---|---|---|
| ^{∗}DNase is shown as mgs of active enzyme per 100g of detergent. | | | | | | | |

Examples 14-23. Heavy Duty Liquid laundry detergent compositions

| | 14 (wt%) | 15 (wt%) | 16 (wt%) | 17 (wt%) | 18 (wt%) | 19 (wt%) | 20 (wt%) | 21 (wt%) |
|---|---|---|---|---|---|---|---|---|
| C12-15 Alkylethoxy(1.8)sulfate | 14.7 | 11.6 | 0.0 | 16.3 | 0.0 | 17.3 | 20 | 12 |
| C11.8 Alkylbenzene sulfonate | 4.3 | 11.6 | 8.3 | 7.8 | 11.7 | 7.8 | 7 | 0 |
| C16-17 Branched alkyl sulfate | 1.7 | 1.29 | 0.0 | 3.09 | 0.0 | 3.3 | 0 | 0 |
| C12-14 Alkyl -9-ethoxylate | 0.9 | 1.07 | 0.0 | 1.31 | 0.0 | 1.31 | 5 | 0 |
| C12 dimethylamine oxide | 0.6 | 0.64 | 0.0 | 1.03 | 0.0 | 1.03 | 2 | 3 |
| Citric acid | 3.5 | 0.65 | 3 | 0.66 | 2.27 | 0.67 | 1 | 0 |
| C12-18 fatty acid | 1.5 | 2.32 | 3.6 | 1.52 | 0.82 | 1.52 | 1 | 0 |
| Sodium Borate (Borax) | 2.5 | 2.46 | 1.2 | 2.53 | 0.0 | 2.53 | 0 | 1 |
| Sodium C12-14 alkyl ethoxy 3 sulfate | 0.0 | 0.0 | 2.9 | 0.0 | 3.9 | 0.0 | 0 | 14 |
| C14-15 alkyl 7-ethoxylate | 0.0 | 0.0 | 4.2 | 0.0 | 1.9 | 0.0 | 0 | 4.2 |
| C12-14 Alkyl -7-ethoxylate | 0.0 | 0.0 | 1.7 | 0.0 | 0.5 | 0.0 | 0 | 1.7 |
| Ca chloride dihydrate | 0.0 | 0.0 | 0.0 | 0.0 | 0.045 | 0.0 | 0 | 0 |
| Ca formate | 0.09 | 0.09 | 0.0 | 0.09 | 0.0 | 0.09 | 0.09 | 0 |
| A compound: bis((C2H5O)(C2H4O)n)(C H3)-N+-CxH2x-N+-(CH3)-bis((C2H5O)(C2H4O)n); | 0.0 | 0.0 | 1.2 | 0.0 | 0.66 | 0.0 | 0.0 | 1.2 |
| n is 20 to 30; x is 3 to 8, optionally sulphated or sulphonated | | | | | | | | |
| Random graft co-polymer¹ | 0.0 | 1.46 | 0.5 | 0.0 | 0.83 | 0.0 | 0.0 | 0.5 |
| Ethoxylated Polyethylenimine² | 1.5 | 1.29 | 0.0 | 1.44 | 0.0 | 1.44 | 1.44 | 0.0 |
| Diethylene triamine pentaacetic acid | 0.34 | 0.64 | 0.0 | 0.34 | 0.0 | 0.34 | 0.34 | 0.0 |
| Diethylene triamine penta (methylene phosphonic acid) | 0.0 | 0.0 | 0.3 | 0.0 | 0.3 | 0.0 | 0.0 | 0.3 |
| 1-hydroxyethyidene-1,1-diphosphonic acid | 0.0 | 0.0 | 0.0 | 0.0 | 0.18 | 0.0 | 0.0 | 0.0 |
| Dihydroxybenzene-3,5-disulfonic acid disodium salt hydrate | 0.0 | 0.0 | 0.0 | 0.0 | 0.0 | 0.19 | 0.19 | 0.0 |
| Tinopal AMS-GX | 0.0 | 0.06 | 0.0 | 0.0 | 0.0 | 0.29 | 0.29 | 0.0 |
| Tinopal CBS-X | 0.2 | 0.17 | 0.0 | 0.29 | 0.0 | 0.0 | 0.0 | 0.0 |
| Tinopal TAS-X B36 | 0.0 | 0.0 | 0.0 | 0.0 | 0.091 | 0.0 | 0.0 | 0.0 |
| Amphiphilic alkoxylated grease cleaning polymer³ | 1.28 | 1 | 0.4 | 1.93 | 0.0 | 1.93 | 1.93 | 0.4 |
| CHEC | 0.0 | 0.0 | 0.2 | 0.0 | 0.0 | 0.0 | 0.0 | 0.2 |
| Ethanol | 2 | 1.58 | 1.6 | 5.4 | 1.2 | 3.57 | 0 | 1.6 |
| Propylene Glycol | 3.9 | 3.59 | 1.3 | 4.3 | 0.0 | 3.8 | 3.8 | 1.3 |
| Diethylene glycol | 1.05 | 1.54 | 0.0 | 1.15 | 0.0 | 1.15 | 1.15 | 0.0 |
| Polyethylene glycol | 0.06 | 0.04 | 0.0 | 0.1 | 0.0 | 0.1 | 0.1 | 0.0 |
| ⁴Amylase Amplify^{®} (mg active) | 8.0 | 7.0 | 2.5 | 4.0 | 3.0 | 1.7 | 3 | 2.5 |
| ⁵DNase (mg active per 100g detergent) | 7.0 | 3.0 | 2.5 | 4.0 | 1.25 | 10.0 | 3 | 2.5 |
| Hexosaminidase (mg active per 100g of detergent) | 3.2 | 4.1 | 7.9 | 12.4 | 3.7 | 5.0 | 17.3 | 2.1 |
| Monoethanolamine | 3.05 | 2.41 | 0.4 | 1.26 | 0.31 | 1.13 | 1.13 | 0.4 |
| NaOH | 2.44 | 1.8 | 0.0 | 3.01 | 3.84 | 0.24 | 0.24 | 0.0 |
| Sodium Cumene Sulphonate | 0.0 | 0.0 | 1 | 0.0 | 0.95 | 0.0 | 0.0 | 1 |
| Sodium Formate | 0.0 | 0.11 | 0.0 | 0.09 | 0.2 | 0.12 | 0.12 | 0.0 |
| Polyethoxylated azo thiophene dye | 0.001 | 0.001 | 0.001 | 0.05 | 0.000 1 | 0.000 1 | 0.000 1 | 0.001 |
| Water, Aesthetics (Dyes, perfumes) and Minors (Enzymes including lipase, protease, additional amylase each at 0.2% active protein, solvents, structurants) | balance | | | | | | | |

| | 22 (wt%) | | 23 (wt%) | |
|---|---|---|---|---|
| C13 Branched Alkyl Sulfate | 4.0 | | 1.0 | |
| C11.8 Alkylbenzene sulfonate | 8.5 | | 1.5 | |
| C15 Branched Alkyl Sulfate | 6.0 | | 3.0 | |
| Sodium Lauryl Sulfate | 3.70 | | 0.0 | |
| C12-14 Alkyl -9-ethoxylate | 8.0 | | 13.0 | |
| C14-15 Alkyl-7-ethoxylate | 4.0 | | --- | |
| C12 dimethylamine oxide | 1.0 | | 0.5 | |
| Citric acid | 6.8 | | 0.7 | |
| C12-18 fatty acid | 1.0 | | 3.0 | |
| Sodium Borate (Borax) | 2.0 | | 0.1 | |
| Ca formate | 0.2 | | 0.3 | |
| Ethoxylated Polyethylenimine² | 3.0 | | 1.3 | |
| Diethylene triamine pentaacetic acid | 0.0 | | 0.3 | |
| Diethylene triamine penta (methylene phosphonic acid) | 0.1 | | 0.0 | |
| glutamic-N,N- diacetic acid | 0.4 | | --- | |
| Tinopal CBS-X | 0.2 | | 0.04 | |
| Amphiphilic alkoxylated grease cleaning polymer³ | 2.0 | | 2.0 | |
| Ethanol | 1.7 | | 0.0 | |
| Propylene Glycol | 2.0 | | 0.3 | |
| Diethylenetriamine | 0.1 | | 0.0 | |
| Butylated hydroxytoluene | 0.4 | | --- | |
| Tinogard TS | --- | | 0.1 | |
| ⁴Amylase Amplify^{®} (mg active) | 8.0 | | 1.7 | |
| ⁵DNase (mg active per 100g detergent) | 10 | | 3.0 | |
| Monoethanolamine | 3.0 | | 0.0 | |
| NaOH | 1.1 | | 0.0 | |
| Sodium Cumene Sulphonate | 1.6 | | 3.0 | |
| Sodium Formate | 0.2 | | 0.3 | |
| Polyethoxylated azo thiophene dye | 0.001 | | 0.001 | |
| Water, Aesthetics (Dyes, perfumes) and Minors (Enzymes including lipase, protease, additional amylase each at 0.2% active protein, solvents, structurants) | Balance | | | |

Examples 24-30. Unit Dose Laundry detergent compositions. Such unit dose formulations can comprise one or multiple compartments.

| | 24 (wt%) | 25 (wt%) | 26 (wt%) | 27 (wt%) | 28 (wt%) | 29 (wt%) | 30 (wt%) |
|---|---|---|---|---|---|---|---|
| Alkylbenzene sulfonic acid | 14.5 | 14.5 | 14.5 | 14.5 | 14.5 | 23 | 23 |
| C12-18 alkyl ethoxy 2.5 sulfate | 7.5 | 7.5 | 7.5 | 7.5 | 7.5 | 16 | 16 |
| C12-18 alkyl 7-ethoxylate | 13.0 | 13.0 | 13.0 | 13.0 | 13.0 | 3.1 | 3.8 |
| C14-15 alkyl 9-ethoxylate | 0 | 0 | 0 | 0 | 0 | 1 | 0 |
| Citric Acid | 0.6 | 0.6 | 0.6 | 0.6 | 0.6 | 0.9 | 0.7 |
| Fatty Acid | 14.8 | 14.8 | 14.8 | 14.8 | 14.8 | 6.5 | 6 |
| Amylase (mg active) | 6 | 12 | 8 | 2 | 10 | 2 | 2 |
| Ethoxylated Polyethylenimine² | 4.0 | 4.0 | 4.0 | 4.0 | 4.0 | 4.0 | 4.0 |
| Protease (Purafect Prime^{®}, 40.6 mg active/g) | 1.4 | 2.0 | 0.9 | 1.2 | 0 | 1 | 1 |
| Cellulase (Celluclean, active protein) | 0.1 | 0.2 | 0.0 | 0.0 | 0.1 | 0 | 0 |
| ⁵DNase described herein (mg active per 100g detergent) | 3.0 | 2.0 | 1.0 | 4.0 | 2.0 | 1 | 1 |
| ⁴Amylase Amplify^{®} (active protein) | 0.0 | 0.0 | 0.1 | 0.2 | 0.1 | 0.5 | 0.5 |
| ⁶Hexosaminidase as defined herein (mg active per 100g of detergent) | 2.2 | 3.1 | 2.3 | 5.2 | 5.3 | 12.2 | 5.4 |
| Hydroxyethane diphosphonic acid | 1.2 | 1.2 | 1.2 | 1.2 | 1.2 | 0 | 2.3 |
| Brightener | 0.3 | 0.3 | 0.3 | 0.3 | 0.3 | 0.2 | 0.2 |
| P-diol | 15.8 | 13.8 | 13.8 | 13.8 | 13.8 | 12.2 | 12.2 |
| Glycerol | 6.1 | 6.1 | 6.1 | 6.1 | 6.1 | 4.0 | 3.8 |
| MEA | 8.0 | 8.0 | 8.0 | 8.0 | 8.0 | 8.6 | 10.2 |
| TIPA | 0.0 | 0.0 | 2.0 | 0.0 | 0.0 | 0.0 | 0.0 |
| TEA | 0.0 | 2.0 | 0.0 | 0.0 | 0.0 | 0.0 | 0.0 |
| Cumene sulphonate | 0.0 | 0.0 | 0.0 | 0.0 | 2.0 | 0.0 | 0.0 |
| Cyclohexyl dimethanol | 0.0 | 0.0 | 0.0 | 2.0 | 0.0 | 0.0 | 0.0 |
| Water | 10 | 10 | 10 | 10 | 10 | 10 | 10 |
| Structurant | 0.14 | 0.14 | 0.14 | 0.14 | 0.14 | 0.14 | 0.14 |
| Perfume | 1.9 | 1.9 | 1.9 | 1.9 | 1.9 | 1.9 | 1.9 |
| Buffers (monoethanolamine) | To pH 8.0 | | | | | | |
| Solvents (1,2 propanediol, ethanol) & minors | To 100% | | | | | | |

### Example 31. Multiple Compartment Unit Dose Composition

Multiple compartment unit dose laundry detergent formulations of the present invention are provided below. In these examples the unit dose has three compartments, but similar compositions can be made with two, four or five compartments. The film used to encapsulate the compartments is polyvinyl alcohol.

| Base composition 1 | 31 (wt%) |
|---|---|
| Glycerol (min 99) | 5.3 |
| 1,2-propanediol | 10.0 |
| Citric Acid | 0.5 |
| Monoethanolamine | 10.0 |
| Caustic soda | - |
| Dequest 2010 | 1.1 |
| Potassium sulfite | 0.2 |
| ⁵DNase as defined herein (mg active) | 8.0 |
| Nonionic Marlipal C24EO7 | 20.1 |
| HLAS | 24.6 |
| Optical brightener FWA49 | 0.2 |
| C12-15 Fatty acid | 16.4 |
| Polymer Lutensit Z96 | 2.9 |
| Polyethyleneimine ethoxylate PEI600 E20 | 1.1 |
| MgCl2 | 0.2 |
| Solvents (1,2 propanediol, ethanol) | To 100% |

### Multi-compartment formulations

| Composition | 1 | | | 2 | | |
|---|---|---|---|---|---|---|
| Compartment | A | B | C | A | B | C |
| Volume of each compartment | 40 ml | 5 ml | 5 ml | 40 ml | 5 ml | 5 ml |
| Active material in Wt.% | | | | | | |
| Perfume | 1.6 | 1.6 | 1.6 | 1.6 | 1.6 | 1.6 |
| Dyes | < 0.01 | < 0.01 | < 0.01 | < 0.01 | < 0.01 | < 0.01 |
| TiO2 | 0.1 | - | - | - | 0.1 | - |
| Sodium Sulfite | 0.4 | 0.4 | 0.4 | 0.3 | 0.3 | 0.3 |
| Acusol 305 | 1.2 | | | 2 | - | - |
| Hydrogenated castor oil | 0.14 | 0.14 | 0.14 | 0.14 | 0.14 | 0.14 |
| Base Composition 1 | Add to 100% | Add to 100% | Add to 100% | Add to 100% | Add to 100% | Add to 100% |

### Example 32-35. Fabric softener compositions of the present invention

| | Weight % | | | |
|---|---|---|---|---|
| | Ex. 32 | Ex. 33 | Ex. 34 | Ex. 35 |
| NaHEDP | 0.007 | 0.007 | 0.007 | 0.007 |
| Formic acid | 0.044 | 0.044 | 0.044 | - |
| HCl | - | 0.009 | 0.009 | 0.009 |
| Preservative^{a} | 0.022 | 0.01 | 0.01 | 0.01 |
| FSA^{b} | 7.6 | 7.6 | 7.6 | 7.6 |
| Antifoam^{c} | 0.1 | 0.1 | 0.1 | 0.1 |
| coconut oil | 0.3 | 0.3 | 0.3 | 0.3 |
| isopropanol | 0.78 | 0.78 | 0.77 | 0.77 |
| Encapsulated perfumed | 0.15 | 0.15 | 0.15 | 0.15 |
| dye | 0.015 | 0.015 | 0.015 | 0.015 |
| Cationic polymeric thickener^{e} | 0.15 | 0.20 | 0.28 | 0.35 |
| ⁵DNase as defined herein (mg active per 100g detergent) | 6.0 | 2.0 | 1.0 | 0.5 |
| 50:50 Blend of alkyl dimethyl benzyl ammonium chloride and alkyl dimethyl ethylbenzyl ammonium chloride^{f} | - | - | 0.4 | - |
| Succinic acid | - | - | - | 5 |
| Perfume | 1.0 | 1.0 | 1.0 | 1.0 |
| deionized water | Balance | Balance | Balance | Balance |

### Raw Materials and Notes For Composition Examples 1-33

Linear alkylbenzenesulfonate having an average aliphatic carbon chain length C11-C18
C12-18 Dimethylhydroxyethyl ammonium chloride
AE3S is C12-15 alkyl ethoxy (3) sulfate
AE7 is C12-15 alcohol ethoxylate, with an average degree of ethoxylation of 7
AE9 is C12-16 alcohol ethoxylate, with an average degree of ethoxylation of 9
HSAS is a mid-branched primary alkyl sulfate with carbon chain length of about 16-17 as disclosed in US 6,020,303 and US 6,060,443
Polyacrylate MW 4500 is supplied by BASF
Carboxymethyl cellulose is Finnfix^{®} V supplied by CP Kelco, Arnhem, Netherlands
CHEC is a cationically modified hydroxyethyl cellulose polymer.

Phosphonate chelants are, for example, diethylenetetraamine pentaacetic acid (DTPA) Hydroxyethane di phosphonate (HEDP)
Savinase^{®}, Natalase^{®}, Stainzyme^{®}, Lipex^{®}, CellucleanTM, Mannaway^{®} and Whitezyme^{®} are all products of Novozymes, Bagsvaerd, Denmark.
Purafect^{®}, Purafect Prime^{®} are products of Genencor International, Palo Alto, California, USA
Fluorescent Brightener 1 is Tinopal^{®} AMS, Fluorescent Brightener 2 is Tinopal^{®} CBS-X, Direct Violet 9 is Pergasol^{®} Violet BN-Z NOBS is sodium nonanoyloxybenzenesulfonate
TAED is tetraacetylethylenediamine
S-ACMC is carboxymethylcellulose conjugated with C.I. Reactive Blue 19product name AZO-CM-CELLULOSE
Soil release agent is Repel-o-tex^{®} PF
Acrylic Acid/Maleic Acid Copolymer is molecular weight 70,000 and acrylate:maleate ratio 70:30
EDDS is a sodium salt of ethylenediamine-N,N'-disuccinic acid, (S,S) isomer Suds suppressor agglomerate is supplied by Dow Corning, Midland, Michigan, USA
HSAS is mid-branched alkyl sulfate
Liquitint^{®} Violet CT polymeric hueing dye, supplied by Milliken, Spartanburg, South Carolina, USA
Polyethoxylated azo thiophene dye is Violet DD^{™} polymeric hueing dye, supplied by Milliken, Spartanburg, South Carolina, USA.
   1 Random graft copolymer is a polyvinyl acetate grafted polyethylene oxide copolymer having a polyethylene oxide backbone and multiple polyvinyl acetate side chains. The molecular weight of the polyethylene oxide backbone is about 6000 and the weight ratio of the polyethylene oxide to polyvinyl acetate is about 40 to 60 and no more than 1 grafting point per 50 ethylene oxide units.
   2 Polyethyleneimine (MW = 600) with 20 ethoxylate groups per -NH
   3 Amphiphilic alkoxylated polymer is a polyethylenimine (MW 600), prepared from a polymer that is derivatised to contain 24 ethoxylate groups per -NH and 16 Propoxylate groups per -NH
   4Amylase is shown as mgs of active enzyme per 100g of detergent.
   5DNase in all of these examples is shown as mgs of active enzyme per 100g of detergent. DNase may comprise minor amounts of super oxide dismutase impurity.

   ^{a} Proxel GXL, 20% aqueous dipropylene glycol solution of 1,2-benzisothiazolin-3-one, supplied by Lonza.
   ^{b} N,N-bis(hydroxyethyl)-N,N-dimethyl ammonium chloride fatty acid ester. The iodine value of the parent fatty acid of this material is between 18 and 22. The material as obtained from Evonik contains impurities in the form of free fatty acid, the monoester form of N,N-bis(hydroxyethyl)-N,N-dimethyl ammonium chloride fatty acid ester, and fatty acid esters of N,N-bis(hydroxyethyl)-N-methylamine.
   ^{c} MP10^{®}, supplied by Dow Corning, 8% activity
   ^{d} as described in US 8,765,659, expressed as 100% encapsulated perfume oil
   ^{e} Rheovis^{®} CDE, cationic polymeric thickener supplied by BASF
   ^{f} N,N-dimethyl octanamide and N,N-dimethyl decanamide in about a 55:45 weight ratio, tradename Steposol^{®} M-8-10 from the Stepan Company

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A detergent composition comprising a DNase variant which compared to the polypeptide of SEQ ID NO: 1 comprises two or more substitutions selected from the group consisting of N61D, T65I, T65V, S82R, K107Q, T127S, T127V, G149N, S164D and L181S, wherein the variant has at least 60% sequence identity to SEQ ID NO: 1 and has DNase activity; and a cleaning adjunct.

2. A detergent composition according to claim 1 wherein the DNase variant comprises two, three, four, five or more of said substitutions.

3. A detergent composition according to claim 1 or claim 2 wherein the DNase variant further comprises at least one substitution selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W.

4. A detergent composition according to any preceding claim wherein the DNase variant comprises a set of substitutions selected from the group consisting of:
a) G149N together with at least one of the substitutions N61D, T65I, T65V, S82R, K107Q, T127S, T127V, S164D and L181S;
b) T65I or T65V together with at least two of the substitutions N61D, S82R, K107Q, T127S, T127V, G149N, S164D and L181S;
c) N61D together with at least two of the substitutions T65I/V, S82R, K107Q, T127S/V, G149N, S164D and L181S, preferably at least two of the substitutions T65I/V, S82R, K107Q, T127S and S164D;
d) S82R together with at least two of the substitutions N61D, T65I, T65V, K107Q, T127S, T127V, G149N, S164D and L181S;
e) K107Q together with at least two of the substitutions N61D, T65I, T65V, S82R, T127S, T127V, G149N, S164D and L181S;
f) T127S together with at least two of the substitutions N61D, T65I, T65V, S82R, K107Q, G149N, S164D and L181S; and
g) S164D together with at least one of the substitutions N61D, T65I, T65V, S82R, K107Q, T127S, T127V, G149N and L181S.

5. A detergent composition according to any preceding claim wherein the DNase variant of any of the preceding claims, comprising a set of substitutions selected from the group consisting of:
• K21L+Q48D+T65I+S82R+K107Q+T127S;
• Q14R+K21L+Q48D+T65I+T127S;
• Q48D+T65I+S82R+T127S+S164D;
• N61D+T65I+K107Q+T127S+S164D;
• Q48D+T65I+S82R+K107Q+T127S;
• Q14R+N61D+T65I+S82R+K107Q;
• N61D+S68L+G149N;
• Q14R+N61D+T65I+S82R+T127S+S164D;
• K21L+Q48D+T65I+S82R+K107Q;
• Q14R+T65I+K107Q+T127S;
• N61D+T65I+S82R+T127S+S164D;
• K21L+N61D+T65I+S82R+K107Q+T127S;
• T65V+T127V+G149N;
• T65I+K107Q+T127S+S164D;
• N61D+T65I+S82R+K107Q;
• Q14R+K21L+N61D+T65I+S82R;
• Q14R+K21L+N61D+T65I+S82R+K107Q;
• Q14R+K21L+N61D+T65I+T127S;
• N61D+T65I+S82R+K107Q+T127S+S164D;
• Q14R+K21L+T65I+K107Q+T127S;
• N61D+T65I+K107Q+T127S;
• T65I+S82R+K107Q+S164D;
• K21L+N61D+T65I+S82R;
• K21L+N61D+T65I+T127S;
• N61D+T65I+S82R+S164D;
• K21L+N61D+T65I+S82R+K107Q;
• S68L+S106A+G149N;
• N61D+T65I+T127S+S164D;
• Q14R+K21L+N61D+T65I;
• Q14R+K21L+T65I+T127S;
• T65V+G149N;
• T65V+R109T+T127V;
• Q14R+K21L+T65I+K107Q;
• K21L+T65I+S82R+K107Q;
• K21L+T65I+K107Q+T127S;
• N61D+S68L+S102Y+G149N+S164D+L181T;
• N61D+S68L+S106A+GI49N+S164D;
• T65V+R109T+G149N;
• T65V+T127V+T171W;
• T65V+T127V+L181S;
• T65I+S164D+L181W;
• N61D+S66Y+S102Y+S164D;
• N61D+S66Y+S164D;
• N61D+T65V+S164D;
• Q14W+N61D+T65I;
• Q14W+N61D+T65I+S164D;
• Q14W+N61D+T65I+S164D+L181W;
• T65I+D116W+S164D+L181W;
• T65I+D116W+S164D;
• Q14W+T65I+S164D;
• R109T+G149N;
• G149N+T171W;
• G149N;
• S164D;
• P25S+L33K+D56I+T65V+Y77T+T127V+L181S;
• P25S+L33K+T65V+Y77T+T127V+L181S;
• P25S+L33K+D56I+T65V+Y77T+R109Q+T127V+L181S;
• P25S+L33K+D56I+T65V+Y77T+D116S+T127V+L181S;
• D56L+T65V+T127V;
• D56L+T65V+T127V+T171W;
• T65V+G149N+T171W;
• Q48D+T65I+K107Q+T127S+S164D;
• T65V+Y77T+T127V;
• T65V+R109T+T127V+G149N;
• T65V+R109T+T171W;
• T65V+R109T+G149N+T171W;
• P25S+D56I+T65V+Y77T+T127V+L181S;
• Q14R+K21L+T65I+K107Q+T127S;
• N61D+S68L+S102Y;
• S66Y+T127V+L181S;
• N61D+T65I+S82R+K107Q+L181D;
• T65V+G149N+L181E;
• T65V+T127V+G149N+Y182D;
• Q48D+N61D+T65I+S82R+K107Q;
• Q48D+T65V+G149N;
• N61D+T65I+S82R+T127S+S164D+Y182D;
• N61D+T65I+S82R+T127S+S164D+L181D;
• Q48D+N61D+T65I+K107Q+T127S+S164D;
• N61D+T65I+S82R+T127S+S164D+Y182N;
• T65I+K107Q+T127S+S164D+Y182D;
• N61D+T65I+K107Q+T127S+S164D+L181E;
• N61D+T65I+K107Q+T127S+S164D+L181D;
• T65I+K107Q+T127S+S164D+L181T;
• T65I+K107Q+T127S+S164D+L181E;
• N61D+T65I+K107Q+T127S+S164D+Y182D;
• T65I+K107Q+T127S+S164D+Y182N;
• N61D+T65I+K107Q+T127S+S164D+L181Q;
• N61D+T65V+S164D+Y182N;
• N61D+T65I+K107Q+T127S+S164D+L181T;
• T65I+K107Q+T127S+S164D+L181D;
• T65I+K107Q+T127S+S164D+L181Q;
• N61D+T65V+T127S+S164D;
• Q48D+N61D+T65V+S164D;
• K21L+N61D+T65I+K107Q+T127S;
• Q14W+N61D+T65I+R109T+G149N+S164D+L181W;
• K21L+N61D+T65I+K107Q;
• Q14W+N61D+T65I+R109T+G149N;
• Q14W+T65V+R109T+G149N+W154I+L181W;
• Q14W+T65V+R109T+G149N+W154I+S164D;
• Q14W+N61D+T65V+R109T+G149N+L181W;
• Q14W+T65I+R109T+D116W+G149N+S164D+L181W;
• T65V+R109T+T127V+T171W;
• R109T+T127V+T171W;
• N61D+T65I+S82R+T127S+S164D+T171E+D175G+L181S;
• T65V+Y77R+G149N;
• T65V+Y77H+G149N;
• T65V+L76K+G149N;
• T65V+L76R+G149N;
• T19E+P25S+L33K+D56I+T65V+Y77T+T127V+L181S;
• T65I+L181S;
• S66L+G149N;
• T65V+Y77T+G149N+L181S;
• T65V+Y77T+G149N;
• D56N+T65V+Y77T+G149N;
• D56N+T65V+L76H+G149N;
• D56Q+T65V+L76H+G149N;
• S68Q+G149N+L181S;
• G149N+L181S;
• S66L+K107E+G149N+Q166D+L181S;
• T65I+G149N+Q166D;
• T65I+G149N+Q166D+Y182G;
• T65I+G149N+Q166D+L181S;
• T65I+G149N+L181S;
• T65I+Q166D+Y182G;
• T65I+Y182G;
• S68Q+G149N+Q166D+L181S;
• T65V+G149N+L181T;
• T19I+S68Q+G149N+Y182G;
• T65I+K107E+G149N+Q166D+Y182G;
• G149N+Q166D+Y182G;
• T65I+K107E+G149N;
• S68Q+G149N+Q166D;
• G149N+Y182G;
• G149N+Q166D+L181S;
• S68Q+K107E+G149N+Y182G;
• K107E+G149N+Y182G;
• T19E+T65I+K107Q+T127S+S164D; and
• N61D+T65I+K107Q+T127S+S164D+Y182N.

6. A detergent composition according to claim 5 wherein the DNase variant comprises a set of substitutions selected from the group consisting of:
• T65V+T127V+L181S;
• N61D+T65I+S82R+K107Q;
• T65V+G149N;
• N61D+T65I+K107Q+T127S+S164D;
• N61D+T65I+T127S+S164D;
• N61D+T65V+S164D;
• T65V+T127V+G149N;
• N61D+T65I+S82R+T127S+S164D; and
• T65I+K107Q+T127S+S164D.

7. A detergent composition according to claim 5 or claims 6 wherein the DNase variant comprises or consists of the polypeptide of SEQ ID NO: 1 with one of said sets of substitutions.

8. A detergent composition according to any preceding claim wherein the DNase variant has at least 70%, at least 80%, at least 85%, at least 90%, at least 91%, at least 92%, at least 93%, at least 94%, at least 95%, at least 96%, at least 97%, or at least 98% sequence identity to SEQ ID NO: 1.

9. A detergent composition according to any preceding claim wherein the variant comprises at least 5, or at least 10, or at least 15, or at least 16 or at least 17 or at least 18 of the indicated amino acid residues in the following positions: I in position 1, Y in position 13, P in position 22, P in position 25, L in position 27, P in position 39, G in position 42, W in position 57, V in position 59, L in position 76, Y in position 77, R in position 109, D in position 116, P in position 144, H in position 147, L in position 167, D in position 175 and L in position 181.

10. A detergent composition according to any preceding claim wherein the DNase variant comprises one or more substitutions selected from the group consisting of I1T, Y13S, P22T, P25S, L27S, P39S, G42S, W57S, V59S, L76V, Y77T, R109Q, D116S, P144S, H147A, L167S and D175G.

11. A detergent composition according to any preceding claim comprising additional enzyme, preferably selected from amylase, hexosaminidase, mannanase, xanthan lyase, xanthanase, and mixtures thereof.

12. A method for making a detergent composition comprising (i) obtaining a DNase variant by introducing into a parent DNase having at least 60% sequence identity to SEQ ID NO: 1 two or more substitutions selected from the group consisting of N61D, T65I, T65V, S82R, K107Q, T127S, T127V, G149N, S164D and L181S (numbering based on SEQ ID NO: 1), and recovering the variant, wherein the variant has DNase activity; and (ii) mixing the DNase variant with a cleaning adjunct.

13. Use of a detergent composition according to any of claims 1 to 11 in a cleaning process such as laundry or hard surface cleaning.

14. A method for treating a surface, preferably a fabric, the method comprising contacting the surface with an aqueous wash liquor comprising a polypeptide as defined in any of claims 1 to 11 having DNase activity, and a cleaning adjunct.

15. A method according to claim 14 wherein the anionic surfactant is present in the wash liquor in an amount from 0.2g/l to 3g/l.


SEQUENCE LISTING

<110>  The Procter & Gamble Company

<120>  CLEANING COMPOSITIONS CONTAINING POLYPEPTIDE VARIANTS

<130>  CM05307M

<160>  28

<170>  PatentIn version 3.5

<210>  1
<211>  182
<212>  PRT
<213>  Artificial Sequence

<220>
<223>  Variant

<400>  1

Ile Pro Pro Gly Thr Pro Ser Lys Ser Ala Ala Gln Tyr Gln Leu Asn
1               5                   10                  15

Ala Leu Thr Val Lys Pro Glu Gly Pro Met Leu Gly Tyr Ser Arg Asp
            20                  25                  30

Leu Phe Pro His Trp Ile Pro Gln Gly Gly Gly Cys Asp Thr Arg Gln
        35                  40                  45

Val Val Leu Lys Arg Asp Ala Asp Trp Tyr Val Gly Asn Cys Pro Val
    50                  55                  60

Thr Ser Gly Ser Trp Tyr Ser Tyr Tyr Asp Gly Leu Tyr Phe Thr Asn
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ser Lys Arg Arg Asp Phe Ala
            100                 105                 110

Asn Asp Leu Asp Gly Pro Gln Leu Ile Ala Val Ser Ala Ser Thr Asn
        115                 120                 125

Arg Ser Lys Gly Asp Gln Asp Pro Ser Thr Trp Gln Pro Pro Arg Pro
    130                 135                 140

Gly Ala His Cys Gly Tyr Ser Lys Trp Trp Ile Ser Thr Lys Tyr Lys
145                 150                 155                 160

Trp Gly Leu Ser Leu Gln Leu Ser Glu Lys Thr Ala Leu Gln Asp Met

                165                 170                 175

Leu Asn Ser Cys Leu Tyr
            180

<210> 2
<211> 182
<212> PRT
<213> Bacillus horikoshii

<400> 2

Leu Pro Pro Gly Thr Pro Thr Lys Ser Glu Ala Gln Asn Gln Leu Asn
1               5                   10                  15

Ser Leu Thr Val Lys Ser Glu Gly Ser Met Thr Gly Tyr Ser Arg Asp
            20                  25                  30

Leu Phe Pro His Trp Ser Gly Gln Gly Asn Gly Cys Asp Thr Arg Gln
        35                  40                  45

Ile Val Leu Gln Arg Asp Ala Asp Tyr Tyr Thr Gly Thr Cys Pro Thr
    50                  55                  60

Thr Ser Gly Lys Trp Tyr Ser Tyr Phe Asp Gly Val Ile Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Gln Arg Arg Ala Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Trp Trp Ile Asn Thr Lys His Arg
145                 150                 155                 160

Trp Asn Leu His Leu Gln Ser Ser Glu Lys Ser Ser Leu Gln Thr Met
                165                 170                 175

Leu Asn Gly Cys Ala Tyr
            180

<210> 3

<211>  182
<212>  PRT
<213>  Bacillus sp-62520

<400>  3

Leu Pro Pro Gly Thr Pro Ser Lys Ser Glu Ala Gln Ser Gln Leu Asn
1               5                   10                  15

Ala Leu Thr Val Lys Pro Glu Asp Pro Met Thr Gly Tyr Ser Arg Asp
            20                  25                  30

His Phe Pro His Trp Ile Ser Gln Gly Asn Gly Cys Asn Thr Arg Gln
        35                  40                  45

Ile Val Leu Gln Arg Asp Ala Asp Tyr Tyr Ser Gly Ala Cys Pro Val
    50                  55                  60

Thr Thr Gly Lys Trp Tyr Ser Tyr Phe Asp Gly Val Ile Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Arg Ser Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Trp Trp Ile Asn Thr Lys His Arg
145                 150                 155                 160

Trp Gly Leu His Leu Gln Ser Ser Glu Lys Ser Ser Leu Gln Ser Met
                165                 170                 175

Leu Asn Gly Cys Ala Tyr
            180


<210>  4
<211>  182
<212>  PRT
<213>  Bacillus horikoshii

<400>  4

Leu Pro Pro Gly Thr Pro Ser Lys Ser Glu Ala Gln Ser Gln Leu Asn
1               5                   10                  15

Ser Leu Thr Val Lys Ser Glu Asp Pro Met Thr Gly Tyr Ser Arg Asp
            20                  25                  30

His Phe Pro His Trp Ser Gly Gln Gly Asn Gly Cys Asp Thr Arg Gln
        35                  40                  45

Ile Val Leu Gln Arg Asp Ala Asp Tyr Tyr Ser Gly Asn Cys Pro Val
    50                  55                  60

Thr Ser Gly Lys Trp Tyr Ser Tyr Phe Asp Gly Val Ile Val Tyr Ser
65                  70                  75                  80

Pro Ser Glu Ile Asp Ile Asp His Val Val Pro Leu Ala Glu Ala Trp
                85                  90                  95

Arg Ser Gly Ala Ser Ser Trp Thr Thr Glu Gln Arg Arg Ser Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Trp Trp Ile Asn Thr Lys His Arg
145                 150                 155                 160

Trp Asn Leu His Leu Gln Ser Ser Glu Lys Ser Ala Leu Gln Thr Met
                165                 170                 175

Leu Asn Gly Cys Val Tyr
            180

<210>  5
<211>  182
<212>  PRT
<213>  Bacillus horikoshii

<400>  5

Leu Pro Pro Gly Thr Pro Ser Lys Ser Glu Ala Gln Ser Gln Leu Asn
1               5                   10                  15

Ser Leu Thr Val Lys Thr Glu Asp Pro Met Thr Gly Tyr Ser Arg Asp
            20                  25                  30

Leu Phe Pro His Trp Ser Gly Gln Gly Ser Gly Cys Asp Thr Arg Gln
        35                  40                  45

Ile Val Leu Gln Arg Asp Ala Asp Tyr Phe Thr Gly Thr Cys Pro Thr
    50                  55                  60

Thr Ser Gly Lys Trp Tyr Ser Tyr Phe Asp Gly Val Ile Val Tyr Ser
65                  70                  75                  80

Pro Ser Glu Ile Asp Val Asp His Ile Val Pro Leu Ala Glu Ala Trp
                85                  90                  95

Arg Ser Gly Ala Ser Ser Trp Thr Thr Glu Gln Arg Arg Ala Phe Ala
            100                 105                 110

Asn Asp Leu Thr Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Trp Trp Ile Asn Thr Lys His Arg
145                 150                 155                 160

Trp Asn Leu His Leu Gln Ser Ser Glu Lys Ser Ser Leu Gln Thr Met
                165                 170                 175

Leu Asn Gly Cys Ala Tyr
            180

<210>  6
<211>  182
<212>  PRT
<213>  Bacillus sp-16840

<400>  6

Leu Pro Pro Gly Thr Pro Ser Lys Ser Glu Ala Gln Ser Gln Leu Asn
1               5                   10                  15

Ala Leu Thr Val Lys Ala Glu Asp Pro Met Thr Gly Tyr Ser Arg Asn
            20                  25                  30

Leu Phe Pro His Trp Asn Ser Gln Gly Asn Gly Cys Asn Thr Arg Gln
        35                  40                  45

Leu Val Leu Gln Arg Asp Ala Asp Tyr Tyr Ser Gly Asn Cys Pro Val
    50                  55                  60

Thr Ser Gly Arg Trp Tyr Ser Tyr Phe Asp Gly Val Val Val Thr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Lys Glu Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Ala Ala Arg Cys Gly Tyr Ala Lys Trp Trp Ile Asn Thr Lys Tyr Arg
145                 150                 155                 160

Trp Asp Leu Ser Leu Gln Ser Ser Glu Lys Ser Ser Leu Gln Thr Met
                165                 170                 175

Leu Asn Thr Cys Ser Tyr
            180

<210> 7
<211> 182
<212> PRT
<213> Bacillus sp-16840

<400> 7

Leu Pro Pro Gly Thr Pro Ser Lys Ser Gln Ala Gln Ser Gln Leu Asn
1               5                   10                  15

Ala Leu Thr Val Lys Ala Glu Asp Pro Met Thr Gly Tyr Ser Arg Asn
            20                  25                  30

Leu Phe Pro His Trp Ser Ser Gln Gly Asn Gly Cys Asn Thr Arg Gln
        35                  40                  45

Leu Val Leu Gln Arg Asp Ala Asp Tyr Tyr Ser Gly Asn Cys Pro Val
    50                  55                  60

Thr Ser Gly Arg Trp Tyr Ser Tyr Phe Asp Gly Val Val Val Thr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Arg Glu Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Val
    130                 135                 140

Ala Ala Arg Cys Gly Tyr Ala Lys Trp Trp Ile Asn Thr Lys Tyr Arg
145                 150                 155                 160

Trp Asp Leu Ser Leu Gln Ser Ser Glu Lys Ser Ser Leu Gln Thr Met
                165                 170                 175

Leu Asn Thr Cys Ser Tyr
            180

<210>  8
<211>  182
<212>  PRT
<213>  Bacillus sp-62668

<400>  8

Leu Pro Pro Gly Thr Pro Ser Lys Ser Glu Ala Gln Ser Gln Leu Thr
1               5                   10                  15

Ser Leu Thr Val Lys Pro Glu Asp Pro Met Thr Gly Tyr Ser Arg Asp
            20                  25                  30

His Phe Pro His Trp Ile Ser Gln Gly Asn Gly Cys Asn Thr Arg Gln
        35                  40                  45

Ile Val Leu Gln Arg Asp Ala Asp Tyr Tyr Ser Gly Asn Cys Pro Val
    50                  55                  60

Thr Thr Gly Lys Trp Tyr Ser Tyr Phe Asp Gly Val Ile Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Ala Glu Gln Arg Arg Asn Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Thr
    130                 135                 140

Gly Ala Arg Cys Ala Tyr Ala Lys Trp Trp Ile Asn Thr Lys Tyr Arg
145                 150                 155                 160

Trp Gly Leu His Leu Gln Ser Ser Glu Lys Ser Ser Leu Gln Ser Met
                165                 170                 175

Leu Asn Gly Cys Ala Tyr
            180

<210>  9
<211>  185
<212>  PRT
<213>  Bacillus sp-13395

<400>  9

Ala Ser Ala Phe Pro Pro Gly Thr Pro Ser Lys Ser Thr Ala Gln Ser
1               5                   10                  15

Gln Leu Asn Ser Leu Pro Val Lys Ser Glu Gly Ser Met Asn Gly Tyr
            20                  25                  30

Ser Arg Asp Lys Phe Pro His Trp Ile Ser Gln Gly Asp Gly Cys Asp
        35                  40                  45

Thr Arg Gln Leu Val Leu Lys Arg Asp Gly Asp Tyr Tyr Ser Gly Ser
    50                  55                  60

Cys Pro Val Thr Ser Gly Lys Trp Tyr Ser Tyr Tyr Asp Gly Ile Thr
65                  70                  75                  80

Val Tyr Ser Pro Ser Glu Ile Asp Ile Asp His Ile Val Pro Leu Ala
                85                  90                  95

Glu Ala Trp Arg Ser Gly Ala Ser Gly Trp Thr Thr Glu Lys Arg Gln
            100                 105                 110

Ser Phe Ala Asn Asp Leu Asn Gly Pro Gln Leu Ile Ala Val Thr Ala
        115                 120                 125

Ser Val Asn Arg Ser Lys Gly Asp Gln Asp Pro Ser Thr Trp Gln Pro
    130                 135                 140

Pro Arg Ser Gly Ser His Cys Ala Tyr Ala Lys Met Trp Val Asn Thr
145                 150                 155                 160

Lys Tyr Arg Trp Gly Leu His Leu Gln Ser Ala Glu Lys Ser Ala Leu
                165                 170                 175

Gln Ser Met Leu Asn Ala Cys Ser Tyr
            180                 185

<210> 10
<211> 185
<212> PRT
<213> Bacillus horneckiae

<400> 10

Ala Ser Ala Phe Pro Pro Gly Thr Pro Ser Lys Ser Thr Ala Gln Ser
1               5                   10                  15

Gln Leu Asn Ser Leu Thr Val Lys Ser Glu Gly Ser Met Thr Gly Tyr
            20                  25                  30

Ser Arg Asp Lys Phe Pro His Trp Ile Ser Gln Gly Asp Gly Cys Asp
        35                  40                  45

Thr Arg Gln Leu Val Leu Lys Arg Asp Gly Asp Tyr Tyr Ser Gly Asn
    50                  55                  60

Cys Pro Val Thr Ser Gly Lys Trp Tyr Ser Tyr Tyr Asp Gly Ile Thr
65                  70                  75                  80

Val Tyr Ser Pro Ser Glu Ile Asp Ile Asp His Ile Val Pro Leu Ala
                85                  90                  95

Glu Ala Trp Arg Ser Gly Ala Ser Gly Trp Thr Thr Glu Lys Arg Gln
            100                 105                 110

Ser Phe Ala Asn Asp Leu Asn Gly Pro Gln Leu Ile Ala Val Thr Ala
        115                 120                 125

Ser Val Asn Arg Ser Lys Gly Asp Gln Asp Pro Ser Thr Trp Gln Pro
    130                 135                 140

Pro Arg Ser Gly Ser His Cys Ala Tyr Ala Lys Met Trp Val Asn Thr
145                 150                 155                 160

Lys Tyr Arg Trp Gly Leu His Val Gln Ser Ala Glu Lys Ser Ala Leu
                165                 170                 175

Gln Ser Met Leu Asn Ala Cys Ser Tyr
            180                 185

<210> 11
<211> 182

<212>  PRT
<213>  Bacillus sp-11238

<400>  11

Phe Pro Pro Glu Ile Pro Ser Lys Ser Thr Ala Gln Ser Gln Leu Asn
1               5                   10                  15

Ser Leu Thr Val Lys Ser Glu Asp Ala Met Thr Gly Tyr Ser Arg Asp
            20                  25                  30

Lys Phe Pro His Trp Ile Ser Gln Gly Asp Gly Cys Asp Thr Arg Gln
        35                  40                  45

Met Val Leu Lys Arg Asp Ala Asp Tyr Tyr Ser Gly Ser Cys Pro Val
    50                  55                  60

Thr Ser Gly Lys Trp Tyr Ser Tyr Tyr Asp Gly Ile Thr Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Arg Asn Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ser
    130                 135                 140

Gly Ala Arg Cys Ala Tyr Ala Lys Met Trp Val Asn Thr Lys Tyr Arg
145                 150                 155                 160

Trp Gly Leu His Leu Gln Ser Ala Glu Lys Ser Gly Leu Glu Ser Met
                165                 170                 175

Leu Asn Thr Cys Ser Tyr
            180


<210>  12
<211>  182
<212>  PRT
<213>  Bacillus SP-62451

<400>  12

Leu Pro Pro Asp Leu Pro Ser Lys Ser Thr Thr Gln Ala Gln Leu Asn
1               5                   10                  15

Ser Leu Asn Val Lys Asn Glu Glu Ser Met Ser Gly Tyr Ser Arg Glu
            20                  25                  30

Lys Phe Pro His Trp Ile Ser Gln Gly Asp Gly Cys Asp Thr Arg Gln
        35                  40                  45

Val Ile Leu Lys Arg Asp Ala Asp Asn Tyr Ser Gly Asn Cys Pro Val
    50                  55                  60

Thr Ser Gly Lys Trp Tyr Ser Tyr Tyr Asp Gly Ile Thr Phe Asn Asp
65                  70                  75                  80

Pro Ser Gln Leu Asp Ile Asp His Val Val Pro Leu Ala Glu Ala Trp
                85                  90                  95

Arg Ser Gly Ala Ser Ser Trp Ser Thr Ala Lys Arg Glu Asp Phe Ala
            100                 105                 110

Asn Asp Leu Asn Gly Pro Gln Leu Ile Ala Val Ser Ala Ser Ser Asn
        115                 120                 125

Arg Ser Lys Gly Asp Gln Asp Pro Ser Thr Trp Gln Pro Pro Arg Ala
    130                 135                 140

Gly Ala Asn Cys Ala Tyr Ala Lys Met Trp Ile Asn Thr Lys Tyr Asn
145                 150                 155                 160

Trp Gly Leu His Leu Gln Ser Ser Glu Lys Thr Ala Leu Gln Gly Met
                165                 170                 175

Leu Asn Ser Cys Ser Tyr
            180

<210>  13
<211>  182
<212>  PRT
<213>  Bacillus sp-18318

<400>  13

Phe Pro Pro Gly Thr Pro Ser Lys Ser Thr Ala Gln Ser Gln Leu Asn
1               5                   10                  15

Ser Leu Thr Val Lys Ser Glu Gly Ser Met Thr Gly Tyr Ser Arg Asp
            20                  25                  30

Lys Phe Pro His Trp Ile Gly Gln Gly Ser Gly Cys Asp Thr Arg Gln
        35                  40                  45

Leu Val Leu Gln Arg Asp Ala Asp Tyr Tyr Ser Gly Ser Cys Pro Val
    50                  55                  60

Thr Ser Gly Lys Trp Tyr Ser Tyr Tyr Asp Gly Val Thr Phe Tyr Asp
65                  70                  75                  80

Pro Ser Asp Leu Asp Ile Asp His Val Val Pro Leu Ala Glu Ala Trp
                85                  90                  95

Arg Ser Gly Ala Ser Ser Trp Ser Thr Gln Lys Arg Lys Asp Phe Ala
            100                 105                 110

Asn Asp Leu Ser Gly Pro Gln Leu Ile Ala Val Ser Ala Ser Ser Asn
        115                 120                 125

Arg Ser Lys Gly Asp Gln Asp Pro Ser Thr Trp Gln Pro Thr Arg Ser
    130                 135                 140

Gly Ala Ala Cys Gly Tyr Ser Lys Trp Trp Ile Ser Thr Lys His Lys
145                 150                 155                 160

Trp Gly Leu Ser Leu Gln Ser Ser Glu Lys Asn Ala Leu Gln Gly Met
                165                 170                 175

Leu Asn Ser Cys Val Tyr
            180

<210>  14
<211>  182
<212>  PRT
<213>  Bacillus idriensis

<400>  14

Leu Pro Pro Gly Thr Pro Ser Lys Ser Thr Ala Gln Ser Gln Leu Asn
1               5                   10                  15

Ala Leu Thr Val Gln Thr Glu Gly Ser Met Thr Gly Tyr Ser Arg Asp
            20                  25                  30

Lys Phe Pro His Trp Ile Ser Gln Gly Asn Gly Cys Asp Thr Arg Gln
        35                  40                  45

Val Val Leu Gln Arg Asp Ala Asp Tyr Tyr Ser Gly Thr Cys Pro Val
    50                  55                  60

Thr Ser Gly Lys Trp Tyr Ser Tyr Tyr Asp Gly Val Thr Leu Tyr Asn
65                  70                  75                  80

Pro Ser Asp Leu Asp Ile Asp His Val Val Ala Leu Ala Glu Ala Trp
                85                  90                  95

Arg Ser Gly Ala Ser Ser Trp Thr Thr Asp Lys Arg Glu Asp Phe Ala
            100                 105                 110

Asn Asp Leu Ser Gly Thr Gln Leu Ile Ala Val Ser Ala Ser Thr Asn
        115                 120                 125

Arg Ser Lys Gly Asp Gln Asp Pro Ser Thr Trp Gln Pro Pro Arg Ser
    130                 135                 140

Gly Ala Ala Cys Gly Tyr Ala Lys Trp Trp Ile Ser Thr Lys Tyr Lys
145                 150                 155                 160

Trp Asn Leu Asn Leu Gln Ser Ser Glu Lys Thr Ala Leu Gln Ser Met
                165                 170                 175

Leu Asn Ser Cys Ser Tyr
            180

<210>  15
<211>  182
<212>  PRT
<213>  Bacillus algicola

<400>  15

Phe Pro Pro Gly Thr Pro Ser Lys Ser Glu Ala Gln Ser Gln Leu Asn
1               5                   10                  15

Ser Leu Thr Val Gln Ser Glu Gly Ser Met Ser Gly Tyr Ser Arg Asp
            20                  25                  30

Lys Phe Pro His Trp Ile Gly Gln Gly Asn Gly Cys Asp Thr Arg Gln
        35                  40                  45

Leu Val Leu Gln Arg Asp Ala Asp Tyr Tyr Ser Gly Asp Cys Pro Val
    50                  55                  60

Thr Ser Gly Lys Trp Tyr Ser Tyr Phe Asp Gly Val Thr Val Tyr Asp
65                  70                  75                  80

Pro Ser Asp Leu Asp Ile Asp His Met Val Pro Met Ala Glu Ala Trp
                85                  90                  95

Arg Ser Gly Ala Ser Ser Trp Ser Thr Gln Lys Arg Glu Asp Phe Ala
            100                 105                 110

Asn Asp Leu Ser Gly Pro His Leu Ile Ala Val Thr Ala Ser Ser Asn
        115                 120                 125

Arg Ser Lys Gly Asp Gln Asp Pro Ser Thr Trp Lys Pro Thr Arg Tyr
    130                 135                 140

Gly Ala His Cys Gly Tyr Ala Lys Trp Trp Ile Asn Thr Lys Tyr Val
145                 150                 155                 160

Tyr Asp Leu Thr Leu Gln Ser Ser Glu Lys Thr Glu Leu Gln Ser Met
                165                 170                 175

Leu Asn Thr Cys Ser Tyr
            180

<210>  16
<211>  182
<212>  PRT
<213>  Enviromental sample J

<400>  16

Leu Pro Pro Asn Ile Pro Ser Lys Ala Asp Ala Leu Thr Lys Leu Asn
1               5                   10                  15

Ala Leu Thr Val Gln Thr Glu Gly Pro Met Thr Gly Tyr Ser Arg Asp
            20                  25                  30

Leu Phe Pro His Trp Ser Ser Gln Gly Asn Gly Cys Asn Thr Arg His
        35                  40                  45

Val Val Leu Lys Arg Asp Ala Asp Ser Val Val Asp Thr Cys Pro Val
    50                  55                  60

Thr Thr Gly Arg Trp Tyr Ser Tyr Tyr Asp Gly Leu Val Phe Thr Ser
65                  70                  75                  80

Ala Ser Asp Ile Asp Ile Asp His Val Val Pro Leu Ala Glu Ala Trp
                85                  90                  95

Arg Ser Gly Ala Ser Ser Trp Thr Ser Thr Lys Arg Gln Ser Phe Ala
            100                 105                 110

Asn Asp Leu Asn Gly Pro Gln Leu Ile Ala Val Ser Ala Thr Ser Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Met Trp Val Glu Thr Lys Ser Arg
145                 150                 155                 160

Trp Gly Leu Thr Leu Gln Ser Ser Glu Lys Ala Ala Leu Gln Thr Ala
                165                 170                 175

Ile Asn Ala Cys Ser Tyr
            180

<210>  17
<211>  182
<212>  PRT
<213>  Bacillus vietnamensis

<400>  17

Phe Pro Pro Gly Thr Pro Ser Lys Ser Thr Ala Gln Ser Gln Leu Asn
1               5                   10                  15

Ala Leu Thr Val Lys Ser Glu Ser Ser Met Thr Gly Tyr Ser Arg Asp
            20                  25                  30

Lys Phe Pro His Trp Ile Gly Gln Arg Asn Gly Cys Asp Thr Arg Gln
        35                  40                  45

Leu Val Leu Gln Arg Asp Ala Asp Ser Tyr Ser Gly Ser Cys Pro Val
    50                  55                  60

Thr Ser Gly Ser Trp Tyr Ser Tyr Tyr Asp Gly Val Thr Phe Thr Asp
65                  70                  75                  80

Pro Ser Asp Leu Asp Ile Asp His Val Val Pro Leu Ala Glu Ala Trp
                85                  90                  95

Arg Ser Gly Ala Ser Ser Trp Thr Thr Ala Lys Arg Glu Asp Phe Ala
            100                 105                 110

Asn Asp Leu Ser Gly Pro Gln Leu Ile Ala Val Ser Ala Ser Ser Asn
        115                 120                 125

Arg Ser Lys Gly Asp Gln Asp Pro Ser Thr Trp Gln Pro Pro Arg Ser
    130                 135                 140

Gly Ala Ala Cys Gly Tyr Ser Lys Trp Trp Ile Ser Thr Lys Tyr Lys
145                 150                 155                 160

Trp Gly Leu Ser Leu Gln Ser Ser Glu Lys Thr Ala Leu Gln Gly Met
                165                 170                 175

Leu Asn Ser Cys Ile Tyr
                180

<210>  18
<211>  182
<212>  PRT
<213>  Bacillus hwajinpoensis

<400>  18

Ile Pro Pro Gly Thr Pro Ser Lys Ser Ala Ala Gln Ser Gln Leu Asp
1               5                   10                  15

Ser Leu Ala Val Gln Ser Glu Gly Ser Met Ser Gly Tyr Ser Arg Asp
            20                  25                  30

Lys Phe Pro His Trp Ile Gly Gln Gly Asn Gly Cys Asp Thr Arg Gln
        35                  40                  45

Leu Val Leu Gln Arg Asp Ala Asp Tyr Tyr Ser Gly Asp Cys Pro Val
    50                  55                  60

Thr Ser Gly Lys Trp Tyr Ser Tyr Phe Asp Gly Val Gln Val Tyr Asp
65                  70                  75                  80

Pro Ser Tyr Leu Asp Ile Asp His Met Val Pro Leu Ala Glu Ala Trp
                85                  90                  95

Arg Ser Gly Ala Ser Ser Trp Ser Thr Gln Lys Arg Glu Asp Phe Ala
            100                 105                 110

Asn Asp Leu Asp Gly Pro His Leu Ile Ala Val Thr Ala Ser Ser Asn
        115                 120                 125

Arg Ser Lys Gly Asp Gln Asp Pro Ser Thr Trp Lys Pro Thr Arg Tyr
    130                 135                 140

Ser Ala His Cys Gly Tyr Ala Lys Trp Trp Ile Asn Thr Lys Tyr Val
145                 150                 155                 160

Tyr Asp Leu Asn Leu Gln Ser Ser Glu Lys Ser Ala Leu Gln Ser Met
                165                 170                 175

Leu Asn Thr Cys Ser Tyr
            180

<210>  19
<211>  182
<212>  PRT

<213>  Paenibacillus mucilaginosus

<400>  19

Leu Pro Pro Gly Thr Pro Ser Lys Ser Thr Ala Gln Ser Gln Leu Asn
1               5                   10                  15

Ser Leu Thr Val Lys Ser Glu Ser Thr Met Thr Gly Tyr Ser Arg Asp
            20                  25                  30

Lys Phe Pro His Trp Thr Ser Gln Gly Gly Gly Cys Asp Thr Arg Gln
        35                  40                  45

Val Val Leu Lys Arg Asp Ala Asp Tyr Tyr Ser Gly Ser Cys Pro Val
    50                  55                  60

Thr Ser Gly Lys Trp Tyr Ser Tyr Tyr Asp Gly Ile Thr Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Gln Asn Phe Ala
            100                 105                 110

Asn Asp Leu Gly Gly Pro Gln Leu Ile Ala Val Thr Ala Ser Ser Asn
        115                 120                 125

Arg Ala Lys Gly Asp Gln Asp Pro Ser Thr Trp Lys Pro Thr Arg Ser
    130                 135                 140

Gly Ala His Cys Ala Tyr Ala Lys Trp Trp Ile Asn Thr Lys Tyr Arg
145                 150                 155                 160

Trp Gly Leu His Leu Gln Ser Ser Glu Lys Thr Ala Leu Gln Ser Met
                165                 170                 175

Leu Asn Thr Cys Ser Tyr
            180


<210>  20
<211>  182
<212>  PRT
<213>  Bacillus indicus

<400>  20

Thr Pro Pro Gly Thr Pro Ser Lys Ser Thr Ala Gln Thr Gln Leu Asn
1               5                   10                  15

Ala Leu Thr Val Lys Thr Glu Gly Ser Met Thr Gly Tyr Ser Arg Asp
            20                  25                  30

Leu Phe Pro His Trp Ile Ser Gln Gly Ser Gly Cys Asp Thr Arg Gln
        35                  40                  45

Val Val Leu Lys Arg Asp Ala Asp Tyr Tyr Ser Gly Ser Cys Pro Val
    50                  55                  60

Thr Ser Gly Lys Trp Tyr Ser Tyr Tyr Asp Gly Val Thr Phe Tyr Asp
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ser Lys Arg Gln Asp Phe Ala
            100                 105                 110

Asn Asp Leu Ser Gly Pro Gln Leu Ile Ala Val Ser Ala Ser Thr Asn
        115                 120                 125

Arg Ser Lys Gly Asp Gln Asp Pro Ser Thr Trp Gln Pro Pro Arg Ala
    130                 135                 140

Gly Ala Ala Cys Gly Tyr Ser Lys Trp Trp Ile Ser Thr Lys Tyr Lys
145                 150                 155                 160

Trp Gly Leu Ser Leu Gln Ser Ser Glu Lys Thr Ala Leu Gln Gly Met
                165                 170                 175

Leu Asn Ser Cys Ser Tyr
            180

<210>  21
<211>  182
<212>  PRT
<213>  Bacillus marisflavi

<400>  21

Thr Pro Pro Val Thr Pro Ser Lys Ala Thr Ser Gln Ser Gln Leu Asn
1               5                   10                  15

Gly Leu Thr Val Lys Thr Glu Gly Ala Met Thr Gly Tyr Ser Arg Asp
            20                  25                  30

Lys Phe Pro His Trp Ser Ser Gln Gly Gly Gly Cys Asp Thr Arg Gln
        35                  40                  45

Val Val Leu Lys Arg Asp Ala Asp Ser Tyr Ser Gly Asn Cys Pro Val
    50                  55                  60

Thr Ser Gly Ser Trp Tyr Ser Tyr Tyr Asp Gly Val Lys Phe Thr Asn
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ala Gln Arg Glu Ala Phe Ala
            100                 105                 110

Asn Asp Leu Ser Gly Ser Gln Leu Ile Ala Val Ser Ala Ser Ser Asn
        115                 120                 125

Arg Ser Lys Gly Asp Gln Asp Pro Ser Thr Trp Gln Pro Pro Arg Ala
    130                 135                 140

Gly Ala Lys Cys Gly Tyr Ala Lys Trp Trp Ile Ser Thr Lys Ser Lys
145                 150                 155                 160

Trp Asn Leu Ser Leu Gln Ser Ser Glu Lys Thr Ala Leu Gln Gly Met
                165                 170                 175

Leu Asn Ser Cys Val Tyr
            180

<210>  22
<211>  184
<212>  PRT
<213>  Bacillus luciferensis

<400>  22

Ala Ser Leu Pro Pro Gly Ile Pro Ser Leu Ser Thr Ala Gln Ser Gln
1               5                   10                  15

Leu Asn Ser Leu Thr Val Lys Ser Glu Gly Ser Leu Thr Gly Tyr Ser
            20                  25                  30

Arg Asp Val Phe Pro His Trp Ile Ser Gln Gly Ser Gly Cys Asp Thr
        35                  40                  45

Arg Gln Val Val Leu Lys Arg Asp Ala Asp Tyr Tyr Ser Gly Asn Cys
    50                  55                  60

Pro Val Thr Ser Gly Lys Trp Tyr Ser Tyr Tyr Asp Gly Val Thr Val
65                  70                  75                  80

Tyr Ser Pro Ser Glu Ile Asp Ile Asp His Val Val Pro Leu Ala Glu
                85                  90                  95

Ala Trp Arg Ser Gly Ala Ser Ser Trp Thr Thr Glu Lys Arg Gln Asn
            100                 105                 110

Phe Ala Asn Asp Leu Asn Gly Pro Gln Leu Ile Ala Val Thr Ala Ser
        115                 120                 125

Ser Asn Arg Ser Lys Gly Asp Gln Asp Pro Ser Thr Trp Gln Pro Thr
    130                 135                 140

Arg Thr Gly Ala Arg Cys Ala Tyr Ala Lys Met Trp Ile Asn Thr Lys
145                 150                 155                 160

Tyr Arg Trp Gly Leu His Leu Gln Ser Ser Glu Lys Ser Ala Leu Gln
                165                 170                 175

Ser Met Leu Asn Thr Cys Ser Tyr
            180

<210>  23
<211>  182
<212>  PRT
<213>  Bacillus marisflavi

<400>  23

Thr Pro Pro Val Thr Pro Ser Lys Glu Thr Ser Gln Ser Gln Leu Asn
1               5                   10                  15

Gly Leu Thr Val Lys Thr Glu Gly Ala Met Thr Gly Tyr Ser Arg Asp
            20                  25                  30

Lys Phe Pro His Trp Ser Ser Gln Gly Gly Gly Cys Asp Thr Arg Gln
        35                  40                  45

Val Val Leu Lys Arg Asp Ala Asp Ser Tyr Ser Gly Asn Cys Pro Val
    50                  55                  60

Thr Ser Gly Ser Trp Tyr Ser Tyr Tyr Asp Gly Val Lys Phe Thr His
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ala Gln Arg Glu Ala Phe Ala
            100                 105                 110

Asn Asp Leu Ser Gly Ser Gln Leu Ile Ala Val Ser Ala Ser Ser Asn
        115                 120                 125

Arg Ser Lys Gly Asp Gln Asp Pro Ser Thr Trp Gln Pro Pro Arg Ala
    130                 135                 140

Gly Ala Lys Cys Gly Tyr Ala Lys Trp Trp Ile Ser Thr Lys Ser Lys
145                 150                 155                 160

Trp Asn Leu Ser Leu Gln Ser Ser Glu Lys Thr Ala Leu Gln Gly Met
                165                 170                 175

Leu Asn Ser Cys Val Tyr
            180

<210>  24
<211>  182
<212>  PRT
<213>  Bacillus sp. SA2-6

<400>  24

Leu Pro Ser Gly Ile Pro Ser Lys Ser Thr Ala Gln Ser Gln Leu Asn
1               5                   10                  15

Ser Leu Thr Val Lys Ser Glu Gly Ser Met Thr Gly Tyr Ser Arg Asp
            20                  25                  30

Lys Phe Pro His Trp Ile Ser Gln Gly Gly Gly Cys Asp Thr Arg Gln
        35                  40                  45

Val Val Leu Lys Arg Asp Ala Asp Tyr Tyr Ser Gly Asn Cys Pro Val
    50                  55                  60

Thr Ser Gly Lys Trp Tyr Ser Tyr Tyr Asp Gly Ile Ser Val Tyr Ser
65                  70                  75                  80

Pro Ser Glu Ile Asp Ile Asp His Val Val Pro Leu Ala Glu Ala Trp
                85                  90                  95

Arg Ser Gly Ala Ser Ser Trp Thr Thr Thr Lys Arg Gln Asn Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Tyr
    130                 135                 140

Gly Ala Arg Cys Ala Tyr Ala Lys Met Trp Ile Asn Thr Lys Tyr Arg
145                 150                 155                 160

Trp Asp Leu Asn Leu Gln Ser Ser Glu Lys Ser Ser Leu Gln Ser Met
                165                 170                 175

Leu Asp Thr Cys Ser Tyr
            180

<210>  25
<211>  7
<212>  PRT
<213>  Artificial

<220>
<223>  Motif

<220>
<221>  MISC_FEATURE
<222>  (1)..(1)
<223>  Xaa = Asp (D) or Met (M) or Leu (L)

<220>
<221>  MISC_FEATURE
<222>  (2)..(2)
<223>  Xaa = Ser (S) or Thr (T)

<220>
<221>  MISC_FEATURE
<222>  (7)..(7)
<223>  Xaa = Asp (D) or Asn (N)

<400>  25

Xaa Xaa Gly Tyr Ser Arg Xaa
1               5

<210>  26
<211>  8
<212>  PRT
<213>  Artificial

<220>
<223>  Motif

<220>
<221>  MISC_FEATURE
<222>  (3)..(3)
<223>  Xaa = any amino acid

<400>  26

Ala Ser Xaa Asn Arg Ser Lys Gly
1               5

<210>  27

<211>  182
<212>  PRT
<213>  Bacillus cibi

<400>  27


<210>  28
<211>  269
<212>  PRT
<213>  Artificial Sequence

<220>
<223>  Variant

<400>  28

Ala Gln Ser Val Pro Trp Gly Ile Glu Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Arg Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asp Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Glu Pro Glu Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Pro Gly Leu Asp Ile
            180                 185                 190

Val Ala Pro Gly Val Asp Ile Leu Ser Thr Trp Pro Gly Gly Thr Tyr
        195                 200                 205

Ala Val Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Glu
                245                 250                 255

Tyr Gly Ser Gly Leu Val Asn Ala Glu Ala Ala Thr Arg
            260                 265